(19) Europäisches Patentamt
European Patent Office
Office européen des brevets

(11) EP 3 903 759 A1

(12) EUROPEAN PATENT APPLICATION

(43) Date of publication:
**03.11.2021 Bulletin 2021/44**

(51) Int Cl.:
***A61K 6/30*** *(2020.01)* ***A61K 6/887*** *(2020.01)*

(21) Application number: **21165514.7**

(22) Date of filing: **29.03.2021**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **01.04.2020 JP 2020065587**
**01.04.2020 JP 2020065588**
**01.04.2020 JP 2020065589**

(71) Applicant: **Shofu Inc.**
**Kyoto-shi, Kyoto 605-0983 (JP)**

(72) Inventors:
- **YAMAMOTO, Kenzo**
**KYOTO, 605-0983 (JP)**
- **HARA, Daisuke**
**KYOTO, 605-0983 (JP)**
- **NISHINO, Yasuhiro**
**KYOTO, 605-0983 (JP)**
- **KITADA, Naoya**
**KYOTO, 605-0983 (JP)**

(74) Representative: **Eisenführ Speiser**
**Patentanwälte Rechtsanwälte PartGmbB**
**Postfach 31 02 60**
**80102 München (DE)**

# (54) DENTAL COMPOSITION HAVING CHARACTERISTIC IN SILANE COUPLING MATERIAL COMPOUNDING INDEX

(57) [Problem]

To provide a dental adhesive composition realizing excellent durable adhesive strength with respect to various dental restorative materials for cutting and machining such as glass ceramics containing lithium disilicate, a dental resin for cutting and machining, and a dental resin for cutting and machining made of a glass fiber reinforced resin in particular and with respect to a tooth substance, and having excellent storage stability simultaneously.

[Solution]

The dental adhesive composition of the present invention comprises a matrix containing (Al) silane coupling material represented by structural formula of [Chemical formula 1],

[Chemical formula1]

$$R^3-\underset{\substack{|\\ R^1_{(3-n)}}}{Si}-(OR^2)_n$$

(B) polymerizable monomer having an acidic group and (C) polymerizable monomer having no acidic group, and wherein the (C) polymerizable monomer having no acidic group contains (Cl) polymerizable monomer having no acidic group and having one or more hydroxyl groups, the (Al) silane coupling material represented by structural formula of [Chemical formula 1] and the (Cl) polymerizable monomer having no acidic group and having one or more hydroxyl groups coexist in at least one matrix, and the dental adhesive composition satisfies at least one of following formulas (1) and (2).



Printed by Jouve, 75001 PARIS (FR)

(Cont. next page)

[Formula (1)]

0.005 ≤ Total amount of silane coupling material compounding amount index in matrix $((S1 \times W1) / M1) \leq 0.070$

[formula (1)]

[Formula (2)]

0.001 ≤ Total amount of silane coupling material compounding amount index in composition $((S2 \times W2) / M2) \leq 0.015$

[formula (2)]

## Description

BACKGROUND OF THE INVENTION

Field of the Invention

The present invention relates to a dental adhesive composition.

Description of the Related Art

In the dental field, a dental adhesive composition has been widely used as a dental adhesive material, a dental resin cement, a dental core build-up material, a dental adhesive material, a tooth substance primer, a metal primer, a ceramics primer, a composite resin, a dental pretreatment material and the like.

Since there are a wide variety of adherends for adhesion in dental treatment, such as a tooth substance, precious metal alloy, non-precious metal alloy, oxide-based ceramics, glass-based ceramics, and a composite resin containing an inorganic filler, it has been common to use a dedicated dental adhesive composition according to each type of adherend. For example, when an adherend is a tooth substance (dentin or enamel), a dental adhesive composition containing an acidic group-containing polymerizable monomer has been used. Further, when a treated surface is a noble metal alloy containing gold, platinum, palladium, silver or the like as a main component, a dental adhesive composition containing a sulfur atom-containing polymerizable monomer has been used. Further, when an adherend is a non-precious metal alloy such as iron, nickel, chromium, cobalt, tin, aluminum, copper or titanium or is oxide-based ceramics such as zirconia or alumina, a dental adhesive composition containing an acidic group-containing polymerizable monomer has been used. In addition, when an adherend is glass-based ceramics or a composite resin containing or an inorganic filler, a dental adhesive composition containing a silane coupling material has been used. It has been considered that the silane coupling material has an alkoxysilyl group (-Si-OR group) in its molecular structure, and the alkoxysilyl group reacts and bonds with the surface of glass ceramics or an inorganic filler by mixing or heating with an acidic or basic aqueous solution to exhibit adhesive property.

Recently, since a CAD/CAM system which is controlled by a computer is making remarkable advances, and various dental restorative materials for cutting and machining prepared by cutting and machining using this system have been clinically applied in dental field. In the cutting and machining using this system, a dental restorative material for cutting and machining which has a block-shape is used for preparing an inlay, a crown or the like, and a dental restorative material for cutting and machining which has a disc-shape is used for preparing a bridge or the like. The dental adhesive compositions have been also used to adhere to these dental restorative materials for cutting and machining.

As a dental adhesive composition, in Patent Document 1, a two-paste type dental adhesive composition containing an acidic group-containing polymerizable monomer and a silane coupling material is proposed. In the invention described in Patent Document 1, both adhesive property to various adherends and storage stability are successfully achieved simultaneously by compounding an acidic group-containing polymerizable monomer and a silane coupling material having an alkoxysilyl group having a carbon chain length of 2 to 5 in different pastes and compounding silane coupling material and a basic filler in the same paste. Further, Patent Document 2 discloses a dental adhesive composition capable of exhibiting adhesive property to a tooth substance in addition to precious metal alloy, non-precious metal alloy, oxide-based ceramics, glass-based ceramics and a composite resin. However, durable adhesive strength to glass ceramics containing lithium disilicate and to tooth substance, and discoloration resistance property were insufficient.

Further, as a method for realizing durable adhesive property to lithium disilicate glass, a composition using a silane coupling material having a carbon chain length of 6 or more between a silicon atom and a polymerizable group (Patent Document 3) and a composition using both a cross-linking agent and a silanol condensation catalyst in combination in addition to a silane coupling material (Patent Document 4) have been proposed. However, the durable adhesive strength with respect to glass ceramics containing lithium disilicate and a tooth substance was insufficient. Furthermore, there have been problems in discoloration resistance property and property stability after long-term storage.

[RELEVANT REFERENCES]

[PATENT LITERATURE]

[Patent Document 1] Japanese Unexamined Patent Application Publication No. 2016-124811
[Patent Document 2] Japanese Unexamined Patent Application Publication No. 2018-177677
[Patent Document 3] International Publication WO2019/00439

[Patent Document 4] Japanese Unexamined Patent Application Publication No. 2019-94276

SUMMARY OF THE INVENTION

Technical Problem

An object of the present invention is to provide a dental adhesive composition realizing excellent durable adhesive strength with respect to various dental restorative materials for cutting and machining such as glass ceramics containing lithium disilicate, a dental resin for cutting and machining, and a dental resin for cutting and machining made of a glass fiber reinforced resin in particular and with respect to a tooth substance, and having excellent storage stability simultaneously.

Solution to Problem

The present invention provides a dental adhesive composition comprising a matrix wherein the matrix contains

(A) silane coupling material,
(B) polymerizable monomer having an acidic group,
(C) polymerizable monomer having no acidic group, and
at least one of (D) polymerization initiator and (E) polymerization accelerator,
(A) silane coupling material contains (A1) silane coupling material represented by structural formula of [Chemical formula 1],

[Chemical formula1]

$$R^3-\underset{\displaystyle R^1_{(3\text{-}n)}}{\underset{|}{Si}}-(OR^2)_n$$

(In the formula, $R^3$ represents (meth) acryloyl group having an alkyl group of $C_2$ to $C_{15}$ which may have -O-, -S-, -NH-, -C(O)-O-, -OC(O)-, -OC(O)-NH- and/or -NH-C(O)-O- group, $R_1$ and $R_2$ represent alkyl groups of C1 to C4 and may be the same or different from each other, and n is 1 to 3.)
(C) polymerizable monomer having no acidic group contains (C1) polymerizable monomer having no acidic group and having one or more hydroxyl groups,

the (A1) silane coupling material represented by structural formula of [Chemical formula 1] and the (C1) polymerizable monomer having no acidic group and having one or more hydroxyl groups coexist in at least one matrix, and
the dental adhesive composition satisfies at least one of following formulas (1) and (2).

[Formula (1)]

$$0.005 \leq \text{Total amount of silane coupling material compounding amount index in matrix } ((S1 \times W1) / M1) \leq 0.070$$

(In the formula (1), M1 is a molecular weight of each silane coupling material contained in the matrix, S1 is the number of alkoxysilyl groups in molecule of each silane coupling material contained in the matrix, and W1 is a compounding amount of each silane coupling material in 100 parts by mass of the matrix. The silane coupling material compounding amount index in matrix is calculated by the formula (1) for each type of silane coupling material. )

[Formula (2)]

$$0.001 \leq \text{Total amount of silane coupling material compounding amount index in composition } ((S2 \times W2) / M2) \leq 0.015$$

(In the formula (2), M2 is a molecular weight of each silane coupling material contained in the composition, S2 is the number of alkoxysilyl groups in the molecule of each silane coupling material contained in the composition, and W2 is a compounding amount of each silane coupling material in 100 parts by mass of the composition. The silane coupling material compounding amount index in the composition is calculated by the formula (2) for each type of silane coupling material.)

In the present invention, the total amount of silane coupling material compounding amount index in matrix may be 0.010 or more and 0.055 or less.

The dental adhesive composition in the present invention may be a dental adhesive composition wherein

the dental adhesive composition consists of a first paste and a second paste,
the first paste contains a first matrix and (F) filler,
the first matrix contains the (A) silane coupling material containing the (A1) silane coupling material represented by structural formula of [Chemical formula 1] and the (C) polymerizable monomer having no acidic group,
the second paste contains a second matrix and (F) filler,
the second matrix contains the (B) polymerizable monomer having an acidic group and the (C) polymerizable monomer having no acidic group,
the first matrix contains at least one of the (D1) chemical polymerization initiator and the (E) polymerization accelerator,
the second matrix contains at least one of the (D1) chemical polymerization initiator and the (E) polymerization accelerator, and
when the first matrix contains one or more (D1) chemical polymerization initiator, the second matrix contains the (E) polymerization accelerator, and
when the first matrix contains one or more (E) polymerization accelerator, the second matrix contains the (D1) chemical polymerization initiator.

In the present invention, the dental adhesive composition substantially may not contain (G) water.

In the present invention, a proportion of the compound having a (meth) acryloyl group and/or a (meth) acrylamide group may be 50 to 100 parts by mass in 100 parts by mass of a total amount of the (A) silane coupling material containing the (A1) silane coupling material represented by structural formula of [Chemical formula 1], the (B) polymerizable monomer having an acidic group and the (C) polymerizable monomer having no acidic group.

In the present invention, the dental adhesive composition may contain a matrix containing the (A) silane coupling material containing the (A1) silane coupling material represented by structural formula of [Chemical formula 1], the (B) polymerizable monomer having an acidic group, the (C) polymerizable monomer having no acidic group and the (D) polymerization initiator, and (F) filler.

In the present invention, the (F) filler may be surface-treated with one or more surface treatment agents selected from a silane coupling material, a surfactant, an organopolysiloxane, an inorganic oxide and a polymer compound.

The dental adhesive composition in the present invention may be a dental adhesive composition wherein wherein

the dental adhesive composition contains a matrix and (F) filler,
a compounding amount of the matrix contained in the dental adhesive composition is 25 to 75 parts by mass with respect to 100 parts by mass of the dental adhesive composition,
a compounding amount the (F) filler contained in the dental adhesive composition is 25 to 75 parts by mass with respect to 100 parts by mass of the dental adhesive composition,
a total amount of silane coupling material compounding amount index in matrix calculated in the formula (3) for each type of the (A1) silane coupling material represented by structural formula of [Chemical formula 3] satisfies following formula (3),

[Formula (3)]

$$0.005 \leq \text{Total amount of (A1) silane coupling material compounding amount index in matrix } ((S3 \times W3) / M3) \leq 0.070$$

(In the formula (3), M3 is a molecular weight of each (A1) silane coupling material represented by structural formula of [Chemical formula 1] contained in the matrix, S3 is the number of alkoxysilyl groups in molecule of each (A1) silane coupling material represented by structural formula of [Chemical formula 1] contained in the matrix, and W3 is a compounding amount of each (A1) silane coupling material represented by structural formula of [Chemical formula 1] in 100 parts by mass of the matrix. The silane coupling material compounding amount index in matrix is calculated by the formula (1) for each type of silane coupling material.)

a compounding amount of the (B) polymerizable monomer having an acidic group contained in the dental adhesive composition is 1 to 20 parts by mass with respect to 100 parts by mass of the matrix,

a compounding amount of the (C) polymerizable monomer having no acidic group contained in the dental adhesive composition is 65 to 95 parts by mass with respect to 100 parts by mass of the matrix,

a compounding amount of the (D) polymerization initiator contained in the dental adhesive composition is 0.3 to 6 parts by mass with respect to 100 parts by mass of the matrix,

a compounding amount of the (C1) polymerizable monomer having no acidic group and having one or more hydroxyl groups contained in the dental adhesive composition is 0.1 to 70 parts by mass with respect to 100 parts by mass of the matrix,

the polymerizable monomer contained in the matrix a polymerizable monomer having a (meth) acryloyl group and/or a (meth) acrylamide group,

a compounding amount of the compound having a (meth) acryloyl group and/or a (meth) acrylamide group is 50 to 99 parts by mass with respect to 100 parts by mass of the matrix.

In the present invention, the (A1) silane coupling material represented by structural formula of [Chemical formula 1] may be a silane coupling material having an acryloyl group, and satisfies at least the formula (1).

In the present invention, the dental adhesive composition may contain 15 to 80 parts by mass of the (C1) polymerizable monomer having no acidic group and having one or more hydroxyl groups with respect to 100 parts by mass of a total amount of the first matrix and the second matrix, and may be used for a dental restoration material for cutting and machining.

In the present invention, a compounding amount of the (C1) polymerizable monomer having no acidic group and having one or more hydroxyl groups contained in the first matrix may be 15 to 80 parts by mass with respect to 100 parts by mass of the first matrix,

a compounding amount of the (C1) polymerizable monomer having no acidic group and having one or more hydroxyl groups contained in the second matrix may be 15 to 80 parts by mass with respect to 100 parts by mass of the second matrix, and

the dental adhesive composition satisfies at least the formula (1) and may be used for a dental restoration material for cutting and machining.

In the present invention, the first matrix may contain (C11) polymerizable monomer having no acidic group, having one or more hydroxyl groups, and having a viscosity of 200 mPa ·s or less at 25 °C, and a compounding amount of the (C11) polymerizable monomer having no acidic group, having one or more hydroxyl groups, and having a viscosity of 200 mPa ·s or less at 25 °C may be 0.1 to 40 parts by mass with respect to 100 parts by mass of the first matrix.

In the present invention, the (F) filler compounded in the first paste may be surface-treated with one or more surface treatment agents selected from an organopolysiloxane, a silane coupling material, an inorganic oxide, a surfactant and a polymer compound.

The dental adhesive composition in the present invention may be a dental adhesive composition wherein

a volume ratio of the first paste and the second paste is 1: 0.8 to 1.2,

a compounding amount of the first matrix in the first paste is 25 to 75 parts by mass with respect to 100 parts by mass of the first paste,

a compounding amount of the (F) filler in the first paste is 25 to 75 parts by mass with respect to 100 parts by mass of the first paste,

a total amount of silane coupling material compounding amount index in matrix calculated in the formula (3) for each

type of the (A1) silane coupling material represented by structural formula of [Chemical formula 1] satisfies following formula (3),

[Formula (3)]

$$0.005 \leq \text{Total amount of (A1) silane coupling material compounding amount index in matrix } ((S3 \times W3) / M3) \leq 0.070$$

(In the formula (3), M3 is molecular weight of each (A1) silane coupling material represented by structural formula of [Chemical formula 1] contained in the matrix, S3 is the number of alkoxysilyl groups in molecule of each (A1) silane coupling material represented by structural formula of [Chemical formula 1] contained in the matrix, and W3 is a compounding amount of each (A1) silane coupling material represented by structural formula of [Chemical formula 1] in 100 parts by mass of the matrix.)
a compounding amount of the (C) polymerizable monomer having no acidic group in the first matrix is 65 to 98 parts by mass with respect to 100 parts by mass of the first matrix,
a compounding amount of the second matrix in the second paste is 25 to 75 parts by mass with respect to 100 parts by mass of the second paste,
a compounding amount of the (F) filler in the second paste is 25 to 75 parts by mass with respect to 100 parts by mass of the second paste,
a compounding amount of the (B) polymerizable monomer having an acidic group in the second matrix is 1 to 30 parts by mass with respect to 100 parts by mass of the second matrix,
a compounding amount of the (C) polymerizable monomer having no acidic group in the second matrix is 65 to 95 parts by mass with respect to 100 parts by mass of the second matrix,
a compounding amount of the (D) polymerization initiator is 0.1 to 5 parts by mass with respect to 100 parts by mass of the total amount of the first matrix and the second matrix,
a compounding amount of the (E) polymerization accelerator is 0.01 to 5 parts by mass with respect to 100 parts by mass of the total amount of the first matrix and the second matrix,
a compounding amount of the (C1) polymerizable monomer having no acidic group and having one or more hydroxyl groups is 15 to 80 parts by mass with respect to 100 parts by mass of the total amount of the first matrix and the second matrix,
a compounding amount of the (C11) polymerizable monomer having no acidic group, having one or more hydroxyl groups, and having a viscosity of 200 mPa ·s or less at 25 °C contained in 100 parts by mass of the first matrix is 0.1 to 50 parts by mass.

In the present invention, the dental adhesive composition may further contain (G) water and (H) volatile organic solvent, and satisfy at least the formula (2).

In the present invention, a compounding amount of the (C1) polymerizable monomer having no acidic group and having one or more hydroxyl groups may be 20 to 70 parts by mass with respect to 100 parts by mass of a total amount of the (A) silane coupling material containing the (A1) silane coupling material represented by structural formula of [Chemical formula 1], (B) polymerizable monomer having an acidic group and (C) polymerizable monomer having no acidic group.

In the present invention, the (A1) silane coupling material represented by structural formula of [Chemical formula 1] may be a silane coupling material having an acryloyl group.

In the present invention, the total amount of silane coupling material compounding amount index in composition may be 0.002 or more and 0.008 or less.

In the present invention, the dental adhesive composition may further contain a polymerizable monomer having one or more sulfur atoms.

The dental adhesive composition in the present invention may be a dental adhesive composition wherein
a total amount of silane coupling material compounding amount index in composition calculated in the formula (4) for each type of the (A1) silane coupling material represented by structural formula of [Chemical formula 1] satisfies following formula (4),

[Formula (4)]

$$0.001 \leq \text{Total amount of (A1) silane coupling material compounding amount index in composition } ((S4 \times W4) / M4) \leq 0.015$$

(In the formula (4), M4 is a molecular weight of each (A1) silane coupling material represented by structural formula of [Chemical formula 1] contained in the composition, S4 is the number of alkoxysilyl groups in molecule of each (A1) silane coupling material represented by structural formula of [Chemical formula 1] contained in the composition, and W4 is a compounding amount of each (A1) silane coupling material represented by structural formula of [Chemical formula 1] in 100 parts by mass of the composition.)

a compounding amount of the (B) polymerizable monomer having an acidic group contained in 100 parts by mass of the dental adhesive composition is 1 to 40 parts by mass,
a compounding amount of the (C) polymerizable monomer having no acidic group contained in 100 parts by mass of the dental adhesive composition is 5 to 60 parts by mass,
a compounding amount of the (D) polymerization initiator contained in 100 parts by mass of the dental adhesive composition is 0.01 to 5 parts by mass, and/or a compounding amount of the (E) polymerization accelerator contained in 100 parts by mass of the dental adhesive composition is 0.01 to 5 parts by mass,
a compounding amount of the (H) volatile organic solvent contained in 100 parts by mass of the dental adhesive composition is 5 to 90 parts by mass,
a compounding amount of the (G) water contained in 100 parts by mass of the dental adhesive composition is 1 to 50 parts by mass,
a compounding amount of a polymerizable monomer having no acidic group and having two or more polymerizable groups is 40 to 100 parts by mass with respect to 100 parts by mass of the (C) polymerizable monomer having no acidic group contained in the dental adhesive composition,
a compounding amount of the (C1) polymerizable monomer having no acidic group and having one or more hydroxyl groups contained in (C) polymerizable monomer having no acidic group is 20 to 70 parts by mass with respect to 100 parts by mass of a total amount of the (A) silane coupling material containing the (A1) silane coupling material represented by structural formula of [Chemical formula 1], (B) polymerizable monomer having an acidic group and (C) polymerizable monomer having no acidic group,
a compounding amount of a compound having a methacryloyl group and/or a metaacrylamide group is 60 to 100 parts by mass in 100 parts by mass of a total amount of the (A) silane coupling material containing the (A1) silane coupling material represented by structural formula of [Chemical formula 1], (B) polymerizable monomer having an acidic group and (C) polymerizable monomer having no acidic group.

The present invention provides a dental self-adhesive composite resin containing the dental adhesive composition of the present invention.

The present invention provides use of the dental adhesive composition of the present invention for adhesion to a dental resin for cutting and machining consisting of a glass fiber reinforced material containing a glass fiber and an epoxy resin.

The present invention provides use of the dental adhesive composition of the present invention for adhesion to a dental resin for cutting and machining.

The present invention provides use of the dental adhesive composition of the according present invention for a dental restoration material for cutting and machining wherein,

the dental restoration material for cutting and machining is a glass fiber reinforced material containing a glass fiber and an epoxy resin, and
the dental restoration material for cutting and machining is a material in which the orientation direction of the glass fibers is not uniform and the glass fibers are randomly compounded, or is a material which is a laminated body in which the glass fibers are woven in a cross shape, and the woven surface in a cross shape and a surface in which the woven surface in a cross shape is rotated 90° in the vertical direction are laminated surfaces of the glass fiber.

The present invention provides use of the dental adhesive composition of the present invention for a dental restoration material for cutting and machining wherein,
the dental adhesive composition is used for adhering to two or more adherend surfaces having different structures of the dental restoration material for cutting and machining.

Advantageous Effects of Invention

The present invention can provide a dental adhesive composition realizing excellent durable adhesive strength with respect to various dental restorative materials for cutting and machining such as glass ceramics containing lithium disilicate, a dental resin for cutting and machining, and a dental resin for cutting and machining made of a glass fiber reinforced resin in particular and with respect to a tooth substance, and having excellent storage stability simultaneously.

DETAILED DESCRIPTION OF THE PREFERRED EMBODIMENTS

The dental adhesive composition of the present invention is a material which can be used to restore an anatomical form of a carious part or a dental defect. Properties suitable for the desired use, for example, the mechanical strength and the adhesive strength capable of withstanding the occlusal pressure, and the color tone close to that of a tooth substance and the like can be obtained by curing a dental adhesive composition by a polymerization reaction. The polymerization reaction of the dental adhesive composition is roughly classified into a photopolymerization in which polymerization is initiated by light irradiation, and a chemical polymerization in which polymerization is initiated by a chemical polymerization initiator and a chemical polymerization accelerator. Although the photopolymerization can be performed at an arbitrary time of the operator by light irradiation, the part to which light does not reach cannot be polymerized at all by the photopolymerization. On the other hand, although the curing time in the chemical polymerization depends on the type and amount of both of the chemical polymerization initiator and the chemical polymerization accelerator and the like, because the part to which light does not reach can be polymerized, therefore, the chemical polymerization has been used for many dental materials. In addition, dental materials are required to have high storage stability in which a decrease in curability and a change in properties are not found regardless of the intended use thereof, and high color tone stability and discoloration resistance property in which color tone does not change after application to the oral cavity.

Since there are a wide variety of adherends for adhesion in dental treatment, such as a tooth substance, precious metal alloy, non-precious metal alloy, oxide-based ceramics, glass-based ceramics, and a composite resin (containing an inorganic filler), it has been common to perform filling and luting with dental adhesive composition after applying a dedicated primer or bonding material according to each type of adherend. In recent years, among ceramics, materials such as glass ceramics containing lithium disilicate which are excellent in long-term stability and mechanical strength, but are difficult to bond have become widespread. Therefore, there is a demand for a material that exhibits highly durable adhesive strength with respect to these materials.

Further, in clinical practice, because the operation of using different primers and bonding materials according to the type of adherend is complicated, there is a risk of a technical error. Furthermore, because the number of operation steps is large, the operation of filling and luting with dental adhesive composition after applying a primer or bonding material is not preferable in clinical. For these reasons, there is a demand for a dental adhesive composition can perform filling and luting regardless of the type of adherend and without applying a primer or a bonding material by compounding an acidic group-containing polymerizable monomer and a silane coupling material and therefore.

In addition, the dental adhesive composition of the present invention can be used as a dental adhesive composition for adhering various dental restorative materials for cutting and machining prepared by cutting and machining using a CAD/CAM system which is controlled by a computer. Examples of a dental restorative material for cutting and machining include a dental resin for cutting and machining made of a resin material such as acrylic resin and epoxy resin and a composite resin material containing an inorganic particle and/or an inorganic fiber for reinforcement in these resin materials. These resin materials are strongly cured by a polymerizable catalyst or a polymerization initiator, and therefore are difficult to adhere.

In particular, among these, a composite resin material in which an inorganic particle is compounded with a resin material for cutting and machining has been attracting attention in recent years. Because this material is filled with a filler such as an inorganic filler and an organic composite filler at high density and is polymerized and cured by pressurizing and heating, this material is difficult to adhere, and therefore there is a demand for a material that exhibits stable adhesive strength.

Further, since high flexural strength and toughness are required in a case where a bridge, particularly a bone anchored bridge, is installed as a superstructure of an implant, a glass fiber reinforced material made of epoxy glass is used. Since epoxy glass is cured after impregnating glass fibers with epoxy resin, it can realize higher physical properties and toughness than a composite resin material composed of a matrix containing an inorganic filler and a polymerizable monomer containing a (meth) acryloyl group or a (meth) acrylamide group, and therefore it has been spread as a frame material.

In addition to the difficulty of stable adhesion due to the strong curing of the glass fiber reinforced material, the orientation direction of the glass fibers on the adherend surface is not uniform, and therefore the condition of the adherend surface is less likely to be uniform than when the inorganic powder is compounded. On the other hand, when the glass fiber is woven, the surface where the glass fiber is woven and the surface where the glass fiber is laminated are mixed, and therefore the adherend surface is not uniform. Thus, there is a demand for a material that exhibits stable adhesive strength in both case where the epoxy resin is present on the adherend surface and case the glass fiber is present on the adherend surface.

The dental adhesive composition of the present invention may be, for example, a composition consisting of a matrix containing (A) silane coupling material containing (A1) silane coupling material represented by structural formula of [Chemical formula 1], (B) polymerizable monomer having an acidic group, (C) polymerizable monomer having no

acidic group and (D) polymerization initiator, wherein the (A1) silane coupling material represented by structural formula of [Chemical formula 1] has an acryloyl group.

The dental adhesive composition of the present invention may be, for example, a composition wherein the dental adhesive composition consists of first paste and second paste, the first paste contains at least first matrix and (F) filler, the first matrix contains at least (A) silane coupling material containing (A1) silane coupling material represented by structural formula of [Chemical formula 1] and the (C) polymerizable monomer having no acidic group, the second paste contains second matrix and (F) filler, the second matrix contains at least (B) polymerizable monomer having an acidic group and (C) polymerizable monomer having no acidic group, the first matrix contains at least one of (D1) chemical polymerization initiator and (E) polymerization accelerator, the second matrix contains at least one of (D1) chemical polymerization initiator and (E) polymerization accelerator, and when the first matrix contains one or more (D1) chemical polymerization initiator, the second matrix contains (E) polymerization accelerator, and when the first matrix contains one or more (E) polymerization accelerator, the second matrix contains (D1) chemical polymerization initiator and the dental adhesive composition contains 15 to 80 parts by mass of the (C1) polymerizable monomer having no acidic group and having one or more hydroxyl groups with respect to 100 parts by mass of a total amount of the first matrix and the second matrix.

The dental adhesive composition of the present invention may be, for example, a composition containing (A) silane coupling material containing (A1) silane coupling material represented by structural formula of [Chemical formula 1], (B) polymerizable monomer having an acidic group, (C) polymerizable monomer having no acidic group, (H) volatile organic solvent and (G) water, and one or both of (D) polymerization initiator and (E) polymerization accelerator.

Hereinafter, each component in the dental adhesive composition of the present invention is described in detail.

The silane coupling material and the polymerizable monomer contained in the composition of the present invention preferably have a polymerizable group exhibiting radical polymerizability, and specifically, from the viewpoint of easy radical polymerization, it is preferable that (meth) acrylic group and/or (meth) acrylamide group is contained as the polymerizable group. In the present specification, "(meth) acrylic" means acrylic and/or methacrylic, "(meth) acryloyl" means acryloyl and/or methacryloyl, and, "(meth) acrylate" means acrylate and/or methacrylate.

The matrix contained in the dental adhesive composition of the present invention contains (A1) silane coupling material represented by structural formula of [Chemical formula 1].

[Chemical formula 1]

$$R^3-\underset{\displaystyle R^1_{(3\text{-}n)}}{\underset{|}{Si}}-(OR^2)_n$$

In the present invention, the (A1) silane coupling material represented by structural formula of [Chemical formula 1] and the (C1) polymerizable monomer having no acidic group and having one or more hydroxyl groups coexist in at least one matrix.

In the present invention, the (A) silane coupling material containing the (A1) silane coupling material represented by structural formula of [Chemical formula 1] is compounded so as to satisfy at least one of following formulas (1) and (2). That is, the (A) silane coupling material containing the (A1) silane coupling material represented by structural formula of [Chemical formula 1] is compounded so that the total amount of silane coupling material compounding amount index in matrix calculated by the following formula (1) satisfies the following formula (1) and/or the total amount of silane coupling material compounding amount index in composition calculated by the following formula (2) satisfies the following formula (2).

[Formula (1)]

$$0.005 \leq \text{Total amount of silane coupling material compounding amount index in matrix } ((S1 \times W1) / M1) \leq 0.070$$

(In the formula (1), M1 is a molecular weight of each silane coupling material contained in the matrix, S1 is the number of alkoxysilyl groups in molecule of each silane coupling material contained in the matrix, and W1 is a compounding

amount of each silane coupling material in 100 parts by mass of the matrix. The silane coupling material compounding amount index in matrix is calculated by the formula (1) for each type of silane coupling material.)

[Formula (2)]

$$0.001 \leq \text{Total amount of silane coupling material compounding amount index in composition } ((S2 \times W2) / M2) \leq 0.015$$

(In the formula (2), M2 is a molecular weight of each silane coupling material contained in the composition, S2 is the number of alkoxysilyl groups in the molecule of each silane coupling material contained in the composition, and W2 is a compounding amount of each silane coupling material in 100 parts by mass of the composition. The silane coupling material compounding amount index in the composition is calculated by the formula (2) for each type of silane coupling material.)

The silane coupling material compounded in the matrix and/or composition is compounded for imparting a function that contributes to adhesion to the adherend. In particular, it is preferable to contain a certain amount in order to impart excellent adhesive strength to the dental restorative material for cutting and machining such as glass ceramics containing lithium disilicate and a dental resin for cutting and machining. On the other hand, when the compounding amount of the silane coupling material is large, there is a case where the storage stability is reduced, the durable adhesion strength to the tooth substance is reduced, and the discoloration resistance property is reduced. As a result of intensive studies by the present inventors, it has been found that the amount of alkoxysilyl groups in the silane coupling material compounded in the matrix and/or composition affects the adhesive strength, the storage stability, and the discoloration resistance property. Based on these, since it is preferable that the silane coupling material contained in the matrix and/or the composition is compounded in consideration of the amount of the alkoxysilyl group, the silane coupling material compounding amount index has been found. In the silane coupling material compounding amount index, the molecular weight can be calculated from the structural formula of the silane coupling material, and when the molecular weight cannot be determined from the structural formula, the average molecular weight measured by Gel Permeation Chromatography (GPC) can be used. The number of alkoxysilyl groups in the molecule of the silane coupling material can be counted from the structural formula of the silane coupling material. For example, in the structural formula of the (A1) silane coupling material represented by structural formula of [Chemical formula 1], the number of alkoxysilyl groups is counted as 3 when n = 3, and the number of alkoxysilyl groups is counted as 1 when n = 1. The alkoxysilyl group indicates (-Si-O-R). R represents a carbon chain, and at least the oxygen atom is bonded to Si and C. The number of alkoxysilyl groups in the silane coupling material is counted separately by the number of -O-R even when a plurality of -O-R are bonded to the same Si atom. When the number of alkoxysilyl groups cannot be determined from the structural formula, it is possible to identify the number of alkoxysilyl groups by quantitative analysis with gas chromatography after adding an alcohol having a carbon chain length different from that of the alkoxysilyl group of the silane coupling material excessively to the silane coupling material. From the molecular weight of the silane coupling material and the number of alkoxysilyl groups, the molecular weight of the silane coupling material with respect to the number of alkoxysilyl groups can be derived, which can be rephrased as the molar mass of the silane coupling material with respect to the alkoxysilyl group. By dividing 100 parts by mass of the matrix and/or the composition by this value, the amount of substance (mol) of the alkoxysilyl group contained in 100 parts by mass of the matrix and/or the composition can be expressed.

The dental adhesive composition of the present invention preferably satisfies both the above formulas (1) and (2).

Further, in the dental adhesive composition of the present invention, the silane coupling material compounding amount index may be determined only based on the above formula (1) when, for example, the dental adhesive composition does not contain (G) water and an organic solvent such as (H) volatile organic solvent, and may be determined only based on the above formula (2) when, for example, the dental adhesive composition contains (G) water and an organic solvent such as the (H) volatile organic solvent.

Specific examples include 2-(meth) acryloxyethyl trimethoxysilane, 3-(meth) acryloxypropyl trimethoxysilane, 3-(meth) acryloxypropyl triethoxysilane, 3-(meth) acryloxypropyl methyldimethoxysilane, 3-(meth) acryloxypropyl tripropoxysilane, 3-(meth) acryloxypropyl methyldipropoxysilane, 3-(meth) acryloxypropyl tributoxysilane, 3-(meth) acryloxypropyl methyldibutoxysilane, 4-(meth) acryloxybutyl trimethoxysilane, 5-(meth) acryloxypentyl trimethoxysilane, 6-(meth) acryloxyhexyl trimethoxysilane, 7-(meth) acryloxyheptyl trimethoxysilane, 8-(meth) acryloxyoctyl trimethoxysilane, 9-(meth) acryloxynonyl trimethoxysilane, 10-(meth) acryloxydecyl trimethoxysilane and 11-(meth) acryloxyundecyl trimethoxysilane.

Furthermore, specific examples having a urethane group or an ether group include 3,3-dimethoxy-8,37-dioxo-2,9,36-trioxa-7,38-diaza-3-silatetracontan-40-yl (meth) acrylate, 2-((3,3-dimethoxy-8-oxo-2,9,18-trioxa-7-aza-3-silanonadecane-19-oyl) amino)-2-methylpropane-1,3-diyl di (meth) acrylate, 3,3-dimethoxy-8,19-dioxo-2,9,18-trioxa-7,20-diaza-3-siladocosane-22-yl (meth) acrylate, 3,3-dimethoxy-8,22-dioxo-2,9,12,15,18,21-hexaoxa-7,23-diaza-3-silapentacosane-25-yl (meth) acrylate, 3,3-dimethoxy-8,22-dioxo-2,9,12,15,18,21,26-heptaoxa-7,23-diaza-3-silaoctacosane-28-yl (meth) acrylate, 3,3-dimethoxy-8,19-dioxo-2,9,12,15,18-pentaoxa-7,20-diaza-3-siladocosane-22-yl (meth) acrylate, 3,3-dimethoxy-8,19-dioxo-2,9,12,15,18,23-hexaoxa-7,20-diaza-3-silapentacosane-25-yl (meth) acrylate, 2-((3,3-dimethoxy-8-oxo-2,9,12,15,18-pentaoxa-7-aza-3-silanonadecane-19-oyl) amino) -2-methylpropan-1,3-diyldi (meth) acrylate, 4,4-diethoxy-17-oxo-3,16,21-trioxa-18-aza-4-silatricosane-23-yl (meth) acrylate, 4,4-diethoxy-17-oxo-3,16,21,24-tetraoxa-18-aza-4-silahexacosane-26-yl (meth) acrylate, 4,4-diethoxy-13-oxo-3,12,17-trioxa-14-aza-4-silanonadecane-19-yl (meth) acrylate, 4,4-diethoxy-17-oxo-3,16-dioxa-18-aza-4-silaicosane-20-yl (meth) acrylate and 2-methyl-2-((11-(triethoxysilyl) undecyloxy) carbonylamino) propan-1,3-diyldi (meth) acrylate. These can be used alone or in combination of two or more, and may be a compound in which alkoxysilyl groups are condensed and a degree of condensation is 2 to 6. Among these, from the viewpoint of high affinity between the dental adhesive composition and the adherend, high mechanical strength and high adhesive strength, 3-(meth) acryloxypropyl trimethoxysilane, 8-(meth) acryloxyoctyl trimethoxysilane, 11-(meth) acryloxyundecyl trimethoxysilane, 4,4-diethoxy-17-oxo-3,16-dioxa-18-aza-4-silaicosane-20-yl (meth) acrylate and 4,4-diethoxy-17-oxo-3,16,21-trioxa-18-aza-4-silatricosane-23-yl (meth) acrylate are preferable. Since 3-(meth) acryloxypropyl trimethoxysilane has a small molecular weight, it is preferable because it exhibits excellent adhesive strength in a dental restorative material for cutting and machining even when the compounding amount in the matrix is small. Because 8-(meth) acryloxyoctyl trimethoxysilane, 11-(meth) acryloxyundecyl trimethoxysilane, 4,4-diethoxy-17-oxo-3,16-dioxa-18-aza-4-silaicosane-20-yl (meth) acrylate and 4,4-diethoxy-17-oxo-3,16,21-trioxa-18-aza-4-silatricosane-23-yl (meth) acrylate have a large molecular weight and the compounding amount in the matrix is large. However, these are preferable because these compounds have high hydrophobicity and therefore has excellent durable adhesiveness to dental restorative material for cutting and machining. In addition, a silane coupling material having an alkoxysilyl group having a longer chain than a generic methoxysilyl group and an ethoxysilyl group such as 3-(meth) acryloxypropyl tripropoxysilane, 3-(meth) acryloxypropyl methyldipropoxysilane, 3-(meth) acryloxypropyl tributoxysilane and 3-(meth) acryloxypropyl methyldibutoxysilane is preferable because it has excellent storage stability. From the viewpoint of versatility and adhesive property, a compound having three alkoxysilyl groups in which n = 3 in the above structural formula is preferable.

The matrix in the present invention means a binder resin containing (A) silane coupling material containing (A1) silane coupling material represented by structural formula of [Chemical formula 1], (B) polymerizable monomer having an acidic group, (C) polymerizable monomer having no acidic group, (D) polymerization initiator and/or (E) polymerization accelerator, and is distinguished from a paste containing a matrix and a filler. The matrix in the present invention does not include a filler adsorbing a polymerization initiator component and a pigment. Unless otherwise specified in the present specification, the matrix in the two-paste type dental adhesive composition represents the sum of the matrix of the first paste and the matrix of the second paste.

When the dental adhesive composition of the present invention is two-paste type, the above formula (1) means the following formula (5).

[Formula (5)]

$$0.005 \leq \text{Total amount of silane coupling material compounding amount index in matrix } ((S5 \times W5) / M5) \leq 0.070$$

(In the formula (5), M5 is a molecular weight of each silane coupling material contained in first matrix and/or second matrix, S5 is the number of alkoxysilyl groups in molecule of each silane coupling material contained in first matrix and/or second matrix, and W5 is a compounding amount of each silane coupling material in 100 parts by mass of the total amount of first matrix and/or second matrix matrix.)

When the compounding amount of the (A) silane coupling material contained in the dental adhesive composition of the present invention exceeds 0.070 in a total amount of silane coupling material compounding amount index in matrix calculated by the formula (1) for each type of silane coupling material and exceeds 0.015 in a total amount of silane coupling material compounding amount index in composition calculated by the formula (2) for each type of silane coupling material, there is a case where the storage stability is reduced, the durable adhesion to the tooth substance is reduced, and the storage stability and the discoloration resistance property are reduced. On the other hand, when the compounding amount of the (A) silane coupling material is less than 0.005 in the total amount of silane coupling material compounding amount index in matrix and is less than 0.001 in the total amount of silane coupling material compounding amount index in composition, there is a case where adhesive property to glass ceramics containing lithium disilicate and adhesive property to a dental resin for cutting and machining are reduced. In the silane coupling material compounding amount in matrix calculated by the formula (1) and the silane coupling material compounding amount index in composition calculated by the formula (2), only the components that contribute to the adhesiveness are considered. The silane coupling material binds to the adherend via, for example, an alkoxysilyl group which is a hydrolyzable group. For example,

the silane coupling material used as a surface treatment agent for the filler is generally hydrolyzed and dehydrated and condensed by mixing with an aqueous solution having low pH of 5 or less or high pH of 9 or more, or by heating. The alkoxysilyl group of the silane coupling material used as the surface treatment agent for the filler is condensed and bonded to the surface of the filler or between the silane coupling materials, and therefore does not contribute to chemical adhesion to the adherend. Therefore, it is not included in the calculation of the silane coupling material compounding amount index in matrix and the silane coupling material compounding amount index in composition.

When a plurality of silane coupling materials are compounded in the dental adhesive composition, the total amount of silane coupling material compounding amount index in matrix is a total amount of silane coupling material compounding amount index in matrix calculated by the formula for each type of silane coupling material and the total amount of silane coupling material compounding amount index in composition is a total amount of silane coupling material compounding amount index in composition calculated by the formula for each type of silane coupling material. When one kind of silane coupling material is compounded in the dental adhesive composition, the total amount of silane coupling material compounding amount index in matrix is a silane coupling material compounding amount index in matrix calculated by the formula for the silane coupling material and the total amount of silane coupling material compounding amount index in composition is a silane coupling material compounding amount index in composition calculated by the formula for the silane coupling material.

The total amount of silane coupling material compounding amount index in matrix is preferably 0.010 or more and 0.055 or less. Further, the total amount of silane coupling material compounding amount index in composition is preferably 0.002 or more and 0.008 or less.

The (A1) silane coupling material represented by structural formula of [Chemical formula 1] may be used alone or in combination of two or more. The compounding amount of the (A1) silane coupling material represented by structural formula of [Chemical formula 1] is preferably 0.005 or more and 0.070 or less in the total amount of (A1) silane coupling material compounding amount index in matrix calculated by the formula (3) for each type of the (A1) silane coupling material represented by structural formula of [Chemical formula 1], and particularly preferably satisfies the formula (3).

[Formula (3)]

$$0.005 \leq \text{Total amount of (A1) silane coupling material compounding amount index in matrix } ((S3 \times W3) / M3) \leq 0.070$$

(In the formula (3), M3 is molecular weight of each (A1) silane coupling material represented by structural formula of [Chemical formula 1] contained in the matrix, S3 is the number of alkoxysilyl groups in molecule of each (A1) silane coupling material represented by structural formula of [Chemical formula 1] contained in the matrix, and W3 is a compounding amount of each (A1) silane coupling material represented by structural formula of [Chemical formula 1] in 100 parts by mass of the matrix.)

More preferably, the total amount of the (A1) silane coupling material compounding amount index in matrix is 0.010 or more and 0.055 or less.

The compounding amount of the (A1) silane coupling material represented by structural formula of [Chemical formula 1] is preferably 0.001 or more and 0.015 or less in the total amount of (A1) silane coupling material compounding amount index in composition calculated by the formula (4) for each type of the (A1) silane coupling material represented by structural formula of [Chemical formula 1], and particularly preferably satisfies the formula (4).

[Formula (4)]

$$0.001 \leq \text{Total amount of (A1) silane coupling material compounding amount index in composition } ((S4 \times W4) / M4) \leq 0.015$$

(In the formula (4), M4 is a molecular weight of each (A1) silane coupling material represented by structural formula of [Chemical formula 1] contained in the composition, S4 is the number of alkoxysilyl groups in molecule of each (A1) silane coupling material represented by structural formula of [Chemical formula 1] contained in the composition, and W4 is a compounding amount of each (A1) silane coupling material represented by structural formula of [Chemical formula 1] in 100 parts by mass of the composition.)

More preferably, the total amount of the (A1) silane coupling material compounding amount index in the composition is 0.002 or more and 0.008 or less.

The (A1) silane coupling material represented by structural formula of [Chemical formula 1] compounded in the dental adhesive composition preferably has an acryloyl group. In the present invention, only the (A1) silane coupling

material represented by structural formula of [Chemical formula 1] having an acryloyl group may be compounded as the silane coupling material. By compounding the (A1) silane coupling material represented by structural formula of [Chemical formula 1] having an acryloyl group, the adhesive strength to glass ceramics containing lithium disilicate is improved. When the (A1) silane coupling material represented by structural formula of [Chemical formula 1] having an acryloyl group is compounded, it is preferable that the (B) polymerizable monomer having an acidic group and the (C) polymerizable monomer having no acidic group a polymerizable monomer having an acidic group have a methacryloyl group and/or a methacrylamide group. In this case, further improvement in adhesive strength to glass ceramics containing lithium disilicate can be expected. Specifically, the compounding amount of the compound having a methacryloyl group and/or a methacrylamide group is preferably 99.9 parts by mass or less, more preferably 80 to 99.9 parts by mass, with respect to 100 parts by mass of total compounding amount of (A) silane coupling material containing (A1) silane coupling material represented by structural formula of [Chemical formula 1], (B) polymerizable monomer having an acidic group and (C) polymerizable monomer having no acidic group (including arbitrarily contained (I) polymerizable monomer having at least one sulfur atom).

In this case, the compounding amount of the (A1) silane coupling material represented by structural formula of [Chemical formula 1] having an acryloyl group with respect to the dental adhesive composition is preferably 0.005 or more and 0.070 or less, particularly preferably satisfies the formula (3), and is more preferably 0.010 or more and 0.055 or less in the total amount of (A1) silane coupling material compounding amount index in matrix calculated by the formula (3) for each type of the (A1) silane coupling material represented by structural formula of [Chemical formula 1] having an acryloyl group.

As the (A1) silane coupling material represented by structural formula of [Chemical formula 1] having an acryloyl group in the present invention, any compound can be used as long as it has an acryloyl group and an alkoxysilyl group.

Examples of the (A1) silane coupling material represented by structural formula of [Chemical formula 1] having an acryloyl group include 2-acryloxyethyl trimethoxysilane, 3-acryloxypropyl trimethoxysilane, 3-acryloxypropyl triethoxysilane, 3-acryloxypropyl methyldimethoxysilane, 4-acryloxybutyl trimethoxysilane, 6-acryloxypentyl trimethoxysilane, 6-acryloxyhexyl trimethoxysilane, 7-acryloxyheptil trimethoxysilane, 8-acryloxyoctyl trimethoxysilane, 9-acryloxynonyl trimethoxysilane, 10-acryloxydecyl trimethoxysilane and 11-acryloxy undecyltrimethoxysilane. Furthermore, specific examples having a urethane group or an ether group include 3,3-dimethoxy-8,37-dioxo-2,9,36-trioxa-7,38-diaza-3-silatetracontan-40-yl acrylate, 2-((3,3-dimethoxy-8-oxo-2,9,18-trioxa-7-aza-3-silanonadecane-19-oyl) amino)-2-methylpropane-1,3-diyl di acrylate, 3,3-dimethoxy-8,19-dioxo-2,9,18-trioxa-7,20-diaza-3-siladocosane-22-yl acrylate, 3,3-dimethoxy-8,22-dioxo-2,9,12,15,18,21-hexaoxa-7,23-diaza-3-silapentacosane-25-yl acrylate, 3,3-dimethoxy-8,22-dioxo-2,9,12,15,18,21,26-heptaoxa-7,23-diaza-3-silaoctacosane-28-yl acrylate, 3,3-dimethoxy-8,19-dioxo-2,9,12,15,18-pentaoxa-7,20-diaza-3-siladocosane-22-yl acrylate, 3,3-dimethoxy-8,19-dioxo-2,9,12,15,18,23-hexaoxa-7,20-diaza-3-silapentacosane-25-yl acrylate, 2-((3,3-dimethoxy-8-oxo-2,9,12,15,18-pentaoxa-7-aza-3-silanonadecane-19-oyl) amino)-2-methylpropan-1,3-diyldi acrylate, 4,4-diethoxy-17-oxo-3,16,21-trioxa-18-aza-4-silatricosane-12-yl acrylate, 4,4-diethoxy-17-oxo-3,16,21,24-tetraoxa-18-aza-4-silahexacosane-26-yl acrylate, 4,4-diethoxy-13-oxo-3,12,17-trioxa-14-aza-4-silanonadecane-19-yl acrylate, 4,4-diethoxy-17-oxo-3,16-dioxa-18-aza-4-silicosan-20-yl acrylate and 2-methyl-2-((11-(triethoxysilyl) undecyloxy) carbonylamino) propan-1,3-diyldi acrylate. These may be used alone or as an appropriate mixture of two or more thereof. Among these, from the viewpoint of high affinity between the dental adhesive composition and the adherend, high mechanical strength and high adhesive strength, 3-acryloxypropyl trimethoxysilane, 8-acryloxyoctyl trimethoxysilane, 11-acryloxy undecyltrimethoxysilane, 4,4-diethoxy-17-oxo-3,16-dioxa-18-aza-4-silaicosane-20-yl acrylate and 4,4-diethoxy-17-oxo-3,16,21-trioxa-18-aza-4-silatricosane-23-yl acrylate are preferable. A silane coupling material having a relatively small molecular weight, such as 3-acryloxypropyl trimethoxysilane, is more preferable for a high-viscosity composition, and have high adhesive strength to glass ceramics containing lithium disilicate is expected by a small compounding amount. A silane coupling material having a relatively large molecular weight, such as 8-acryloxyoctyl trimethoxysilane, 4,4-diethoxy-17-oxo-3,16-dioxa-18-aza-4-silaicosane-20-yl acrylate and 4,4-diethoxy-17-oxo-3,16,21-trioxa-18-aza-4-silatricosane-23-yl acrylate can be expected to have high durable adhesive property to glass ceramics containing lithium disilicate. From the viewpoint of versatility and adhesive property, a silane coupling material having three alkoxysilyl groups in which n = 3 in the above structural formula is preferable.

The (A1) silane coupling material represented by structural formula of [Chemical formula 1] having an acryloyl group may be a condensate in which an alkoxysilyl group remains. Examples of the condensate include a condensate of the compounds described in the above and a condensate with trimethoxy methylsilane, trimethoxy ethylsilane, trimethoxy propylsilane, trimethoxy butylsilane, trimethoxy allylsilane, triethoxy methylsilane, triethoxy ethylsilane, tripropoxy methylsilane, tripropoxy propylsilane, tributoxy methylsilane, tributoxy butylsilane, dimethoxy dimethylsilane or diethoxydiethylsilane. The degree of condensation is in the range of 2 to 30, preferably 2 to 6. Further, these silane coupling materials having an acryloyl group may be used alone or in combination of two or more. When the silane coupling material having an acryloyl group is contained, other silane coupling material such as a silane coupling material having a methacryloyl group may be used in combination.

The dental adhesive composition of the present invention may contain (A) silane coupling material other than

the (A1) silane coupling material represented by structural formula of [Chemical formula 1]. As the (A) silane coupling material other than the (A1) silane coupling material represented by structural formula of [Chemical formula 1], a conventionally known silane coupling material can be used. The dental adhesive composition of the present invention may not contain (A) silane coupling material other than the (A1) silane coupling material represented by structural formula of [Chemical formula 1]. The dental adhesive composition of the present invention may not contain (A) silane coupling material other than the (A1) silane coupling material represented by structural formula of [Chemical formula 1] having an acryloyl group.

The dental adhesive composition of the present invention contains (B) polymerizable monomer having an acidic group in order to impart adhesive property with respect to a tooth substance and a prosthetic device mounted in an oral cavity. For the polymerizable monomer having an acidic group, any polymerizable monomer can be used without any limitation as long as it has one or more polymerizable group and at least one acidic group such as a phosphoric acid group, a pyrophosphoric acid group, a thiophosphoric acid group, a phosphonic acid group, a sulfonic acid group and a carboxylic acid group and the like.

Specific examples of a polymerizable monomer having an acidic group having a phosphoric acid group include 2-(meth) acryloyloxyethyl dihydrogen phosphate, 3-(meth) acryloyloxypropyl dihydrogen phosphate, 4-(meth) acryloyloxybutyl dihydrogen phosphate, 5-(meth) acryloyloxypentyl dihydrogen phosphate, 6-(meth) acryloyloxyhexyl dihydrogen phosphate, 7-(meth) acryloyloxyheptyl dihydrogen phosphate, 8-(meth) acryloyloxyoctyl dihydrogen phosphate, 9-(meth) acryloyloxynonyl dihydrogen phosphate, 10-(meth) acryloyloxydecyl dihydrogen phosphate, 11-(meth) acryloyloxyundecyl dihydrogen phosphate, 12-(meth) acryloyloxydodecyl dihydrogen phosphate, 16-(meth) acryloyloxyhexadecyl dihydrogen phosphate, 20-(meth) acryloyloxyicosyl dihydrogen phosphate, bis [2-(meth) acryloyl oxyethyl] hydrogensphosphate, bis [4-(meth) acryloyl oxybutyl] hydrogen phosphate, bis [6-(meth) acryloyl oxyhexyl] hydrogen phosphate, bis [8-(meth) acryloyl oxyoctyl] hydrogen phosphate, bis [9-(meth) acryloyl oxynonyl] hydrogen phosphate, bis [10-(meth) acryloyl oxydecyl] hydrogen phosphate, 1,3-di (meth) acryloyl oxypropyl dihydrogenphosphate, 2-(meth) acryloyl oxyethylphenyl hydrogen phosphate, 2-(meth) acryloyloxyethyl-2-bromoethyl hydrogen phosphate and bis [2-(meth) acryloyloxy-(1-hyrdoxymethyl) ethyl] hydrogen phosphate; acyl chloride, alkali metal salt and ammonium salt thereof; and (meth) acrylamide compound in which the ester bond of these compounds is substituted with an amide bond, and the like.

Specific examples of a polymerizable monomer having an acidic group having a pyrophosphoric acid group include bis [2-(meth) acryloyl oxyethyl] pyrophosphate, bis [4-(meth) acryloyl oxybutyl] pyrophosphate, bis [6-(meth) acryloyl oxyhexyl] pyrophosphate, bis [8-(meth) acryloyl oxyoctyl] pyrophosphate, bis [10-(meth) acryloyl oxydecyl] pyrophosphate; acyl chloride, alkali metal salt and ammonium salt thereof; and (meth) acrylamide compound in which the ester bond of these compounds is substituted with an amide bond, and the like.

Specific examples of a polymerizable monomer having an acidic group having a thiophosphate group include 2-(meth) acryloyloxyethyl dihydrogen thiophosphate, 3-(meth) acryloyloxypropyl dihydrogen thiophosphate, 4-(meth) acryloyloxybutyl dihydrogen thiophosphate, 5-(meth) acryloyloxypentyl dihydrogen thiophosphate, 6-(meth) acryloyloxyhexyl dihydrogen thiophosphate, 7-(meth) acryloyloxyheptyl dihydrogen thiophosphate, 8-(meth) acryloyloxyoctyl dihydrogen thiophosphate, 9-(meth) acryloyloxynonyl dihydrogen thiophosphate, 10-(meth) acryloyloxydecyl dihydrogen thiophosphate, 11-(meth) acryloyloxyundecyl dihydrogen thiophosphate, 12-(meth) acryloyloxydodecyl dihydrogen thiophosphate, 16-(meth) acryloyloxyhexadecyl dihydrogen thiophosphate, 20-(meth) acryloyloxyicosyl dihydrogen thiophosphate; acyl chloride, alkali metal salt and ammonium salt thereof; and (meth) acrylamide compound in which the ester bond of these compounds is substituted with an amide bond, and the like.

Specific examples of a polymerizable monomer having an acidic group having a phosphonic acid group include 2-(meth) acryloyloxy ethylphenyl phosphonate, 5-(meth) acryloyloxy pentyl-3-phosphonopropionate, 6-(meth) acryloyloxy hexyl-3-phosphonopropionate, 10-(meth) acryloyloxy decyl-3-phosphonopropionate, 6-(meth) acryloyloxy hexyl-3-phosphonoacetate, 10-(meth) acryloyloxy decyl-3-phosphonoacetate; acyl chloride, alkali metal salt and ammonium salt thereof; and (meth)acrylamide compound in which the ester bond of these compounds is substituted with an amide bond, and the like.

Specific examples of a polymerizable monomer having an acidic group having a sulfonic acid group are not limited to, but include, 2-(meth)acrylamide-2-methyl propanesulfonic acid and 2-sulfoethyl(meth)acrylate and the like.

Specific examples of a polymerizable monomer having an acidic group having a carboxylic acid group include a (meth) acrylic-based compound having one carboxyl group in the molecule and a (meth) acrylic-based compound having a plurality of carboxyl groups in the molecule. Examples of the (meth) acrylic-based compound having one carboxyl group in the molecule include (meth) acrylic acid, N-(meth) acryloyl glycine, N-(meth) acryloyl aspartic acid, O-(meth) acryloyl tyrosine, N-(meth) acryloyl tyrosine, N-(meth) acryloyl phenylalanine, N-(meth) acryloyl-p-aminobenzoic acid, N-(meth) acryloyl-o-aminobenzoic acid, p-vinylbenzoic acid, 2-(meth) acryloyloxybenzoic acid, 3-(meth) acryloyloxybenzoic acid, 4-(meth) acryloyloxybenzoic acid, N-(meth) acryloyl-5-aminosalicylic acid, N-(meth) acryloyl-4-aminosalicylic acid, 2-(meth) acryloyloxyethyl hydrogen succinate, 2-(meth) acryloyloxyethyl hydrogen phthalate, 2-(meth) acryloyloxyethyl hydrogenmalate; acyl chloride thereof; and (meth)acrylamide compound in which the ester bond of

these compounds is substituted with an amide bond, and the like. Examples of the (meth) acrylic-based compound having a plurality of carboxyl groups in the molecule include 6-(meth) acryloyl oxyhexane-1,1-dicarboxylic acid, 9-(meth) acryloyl oxynonane-1,1-dicarboxylic acid, 10-(meth) acryloyl oxydecane-1,1-dicarboxylic acid, 11-(meth) acryloyloxy undecane-1,1-dicarboxylic acid, 12-(meth) acryloyl oxydodecane-1,1-dicarboxylic acid, 13-(meth) acryloyloxy tridecane-1,1-dicarboxylic acid, 4-(meth) acryloyloxyethyl trimeritate, 4-(meth) acryloyloxybutyl trimeritate, 4-(meth) acryloyloxyhexyl trimeritate, 4-(meth) acryloyloxydecyl trimeritate, 2-(meth) acryloyl oxyethyl-3'-(meth) acryloyloxy-2'-(3,4-dicarboxy benzoyloxy) propylsuccinate; acid anhydrides and acid halides thereof,; and (meth) acrylamide compound in which the ester bond of these compounds is substituted with an amide bond, and the like.

Among the above-described (B) polymerizable monomer having an acidic group, it is preferable to have a phosphoric acid group or a phosphonic acid group from the view point of the adhesive property of the dental adhesive composition. Especially, it is preferable to have an alkyl group or an alkylene group having 4 or more of the carbon number of a main chain in the molecule, and 10-(meth) acryloyl oxydecyl dihydrogen phosphate and (6-methacryloyloxy) hexylphosphonoacetate, 4-methacryloxyethyl trimellitic acid and 4-methacryloyloxyethoxy carbonylphthalic anhydride are more preferable. A plurality of kinds of the (B) polymerizable monomer having an acidic group can be used in combination, if necessary.

When the dental adhesive composition of the present invention is a composition consisting of a matrix containing (A) silane coupling material containing (A1) silane coupling material represented by structural formula of [Chemical formula 1], (B) polymerizable monomer having an acidic group, (C) polymerizable monomer having no acidic group and (D) polymerization initiator, the compounding amount of the (B) polymerizable monomer having an acidic group is preferably 1 to 20 parts by mass with respect to 100 parts by mass of the matrix from the viewpoint of the adhesive property and the storage stability.

When the dental adhesive composition of the present invention is a two-paste type dental adhesive composition containing (D1) chemical polymerization initiator and (E) polymerization accelerator and containing 15 to 80 parts by mass of (C1) polymerizable monomer having no acidic group and having one or more hydroxyl groups with respect to 100 parts by mass of the first matrix and the second matrix, the compounding amount of the (B) polymerizable monomer having an acidic group is preferably 1 to 30 parts by mass with respect to 100 parts by mass of the second matrix. When the compounding amount is less than 1 part by mass, the decrease in the adhesive strength to the tooth substance is particularly remarkable, and there is a possibility that the adhesive strength to the dental restorative material for cutting and machining is decreased. When the compounding amount is more than 30 parts by mass, there is a concern that the storage stability is lowered. The (B) polymerizable monomer having an acidic group may be contained in the first matrix. However, in particular, when the compounding amount in matrix containing the silane coupling material exceeds the total amount of silane coupling material compounding amount index in matrix of the formula (1), there is a case where the storage stability is lowered, and therefore it is preferable to contain only in the second matrix.

When the dental adhesive composition of the present invention is a composition containing (A) silane coupling material containing (A1) silane coupling material represented by structural formula of [Chemical formula 1], (B) polymerizable monomer having an acidic group, (C) polymerizable monomer having no acidic group, (H) volatile organic solvent and (G) water, and further containing one or both of (D) polymerization initiator and (E) polymerization accelerator, the compounding amount of the (B) polymerizable monomer having an acidic group is preferably 1 to 40 parts by mass with respect to 100 parts by mass of the dental adhesive composition. When the compounding amount is less than 1 part by mass, there is a case where the expected adhesive strength is difficult to exhibit. When the compounding amount is more than 40 parts by mass, there is a case where the storage stability is decreased.

In this case, among the (B) polymerizable monomer having an acidic group, the compounding amount of the polymerizable monomer having a highly acidic functional group such as a phosphoric acid ester or a phosphonic acid ester, for example, 10- (meth) acryloyloxydecyl dihydrogen phosphate and (6-methacryloyloxy) hexylphosphonoacetate, is preferably 0.1 to 20 parts by mass. When the compounding amount is less than 0.1 parts by mass, there is a case where the expected adhesive strength is difficult to exhibit. When the compounding amount is more than 20 parts by mass, there is a case where the storage stability is decreased.

As the (C) polymerizable monomer having no acidic group of the present invention, any known polymerizable monomer can be used without any limitation as long as it has one or more polymerizable groups and has no acidic group. The (C) polymerizable monomer having no acidic group includes one having one radical polymerizable group, one having two radical polymerizable groups, and one having three radical polymerizable groups.

Specific examples of a polymerizable monomer having one radical polymerizable group and having no acidic group include 2-hydroxyethyl (meth) acrylate, 3-hydroxypropyl (meth) acrylate, 4-hydroxybutyl (meth) acrylate, 2-hydroxypropyl (meth) acrylate, 2-hydroxybutyl (meth) acrylate, 6-hydroxyhexyl (meth) acrylate, 10-hydroxydecyl (meth) acrylate, propylene glycol mono (meth) acrylate, glycerol mono (meth) acrylate, erythritol mono (meth) acrylate, N-methylol (meth) acrylamide, N-hydroxyethyl (meth) acrylamide, N,N-(dihydroxyethyl) (meth) acrylamide, methyl (meth) acrylate, ethyl (meth) acrylate, propyl (meth) acrylate, isopropyl (meth) acrylate, butyl (meth) acrylate, isobutyl (meth) acrylate, benzyl (meth) acrylate, lauryl (meth) acrylate, 2,3-dibromopropyl (meth) acrylate, 3-(meth) acryloyloxypropyl

trimethoxysilane, 11-(meth) acryloyloxyundecyl trimethoxysilane, (meth) acrylamide and the like. Among these, from the viewpoint of high affinity of the dental adhesive composition to be obtained with the tooth substance, 2-hydroxyethyl (meth) acrylate, 3-hydroxypropyl (meth) acrylate, glycerol mono (meth) acrylate and erythritol mono (meth) acrylate are preferable.

Specific examples of the polymerizable monomer having two radical polymerizable groups and having no acidic group include 2,2-bis ((meth) acryloyloxy phenyl) propane, 2,2-bis [4-(3-(meth) acryloyloxy)-2-hydroxy propoxyphenyl] propane (generally called "Bis-GMA"), 2,2-bis (4-(meth) acryloyloxy phenyl) propane, 2,2-bis (4-(meth) acryloyloxy polyethoxyphenyl) propane, 2,2-bis (4-(meth) acryloyloxy diethoxyphenyl) propane, 2,2-bis (4-(meth) acryloyloxy tetraethoxyphenyl) propane, 2,2-bis (4-(meth)) acryloyloxy pentaethoxyphenyl) propane, 2,2-bis (4-(meth) acryloyloxy dipropoxypheny) propane, 2-(4-(meth) acryloyloxy diethoxyphenyl)-2-(4-(meth) acryloyloxy diethoxyphenyl) propane, 2-(4-(meth) acryloyloxy diethoxyphenyl)-2-(4-(meth) acryloyloxy triethoxyphenyl) propane, 2-(4-(meth) acryloyloxy dipropoxyphenyl)-2-(4-(meth) acryloyloxy triethoxyphenyl) propane, 2,2-bis (4-(meth) acryloyloxy propoxyphenyl) propane, 2,2-bis (4-(meth) acryloyloxy isopropoxyphenyl) propane, 1,4-bis (2-(meth) acryloyloxyethyl) pyromellitate, glycerol di (meth) acrylate, 1-(acryloyloxy)-3-(methacryloyloxy)-2-propanol, ethyleneglycol di (meth) acrylate, diethyleneglycol di (meth) acrylate, triethylene glycol di (meth) acrylate, propylene glycol di (meth) acrylate, butylene glycol di (meth) acrylate, neopentyl glycol di (meth) acrylate, polyethylene glycol di (meth) acrylate, 1,3-butanediol di (meth) acrylate, 1,5-pentanediol di (meth) acrylate, 1,6-hexanediol di (meth) acrylate, 1,10-decanediol di (meth) acrylate, 1,2-bis (3-methacryloyloxy-2-hydroxypropoxy) ethane, 2,2,4-trimethyl hexamethylene bis (2-carbamoyloxy ethyl) dimethacrylate (generally called "UDMA"), 1,2-bis (3-methacryloyloxy-2-hydroxy propoxy) ethane and the like. Among these, from the viewpoint of mechanical strength, 2,2-bis((meth)acryloyloxy phenyl) propane, 2,2-bis[4-(3-(meth)acryloyloxy)-2-hydroxy propoxyphenyl] propane, 2,2,4-trimethyl hexamethylene bis (2-carbamoyloxyethyl) dimethacrylate and 2,2-bis(4-(meth)acryloyloxy polyethoxyphenyl) propane are preferable, and from the viewpoint of handleability, triethyleneglycol di(meth)acrylate, neopentylglycol di(meth)acrylate and glycerol di(meth)acrylate are preferable. Among the 2,2-bis (4-(meth) acryloyloxy polyethoxyphenyl) propanes, a compound having an average addittion mole number of ethoxy group of 2.6 (generally called "D2.6E") is preferable.

Specific examples of the polymerizable monomer having three or more radical polymerizable groups and having no acidic group include trimethylolpropane tri (meth) acrylate, trimethylolethane tri (meth) acrylate, trimethylolmethane tri (meth) acrylate, pentaerythritol tri (meth) acrylate, pentaerythritol tetra (meth) acrylate, dipentaerythritol penta (meth) acrylate, N,N-(2,2,4-trimethylhexamethylene) bis [2-(aminocarboxy) propane-1,3-diol] tetra methacrylate, 1,7-diacryloyloxy-2,2,6,6-tetra acryloyloxymethyl-4-oxyheptane and the like. Among these, trimethylolpropane tri (meth) acrylate is preferable in that the mechanical strength of the resulting dental adhesive composition is large.

A plurality of kinds of the (C) polymerizable monomer having no acidic group may be used in combination, if necessary.

When the dental adhesive composition of the present invention is a composition consisting of a matrix containing (A) silane coupling material containing (A1) silane coupling material represented by structural formula of [Chemical formula 1], (B) polymerizable monomer having an acidic group, (C) polymerizable monomer having no acidic group and (D) polymerization initiator, the compounding amount of the (C) polymerizable monomer having no acidic group is preferably 65 to 95 parts by mass in the matrix from the viewpoint of improving mechanical characteristic. Further, from the viewpoint of improving mechanical characteristic, the compounding amount of the polymerizable monomer having two or more radical polymerizable groups and having no acidic group is preferably set to 40 to 100 parts by mass, more preferably 60 to 100 parts by mass in the 100 parts by mass of the (C) polymerizable monomer having no acidic group which is contained in the matrix. When the compounding amount of the polymerizable monomer having two or more radical polymerizable groups and having no acidic group is less than 40 parts by mass, there is a case where mechanical properties is lowered.

When the dental adhesive composition of the present invention is a two-paste type dental adhesive composition containing (D1) chemical polymerization initiator and (E) polymerization accelerator and containing 15 to 80 parts by mass of (C1) polymerizable monomer having no acidic group and having one or more hydroxyl groups with respect to 100 parts by mass of the first matrix and the second matrix, it is preferable that the compounding amount of the (C) polymerizable monomer having no acidic group is 65 to 98 parts by mass in 100 parts by mass of the first matrix and 65 to 95 parts by mass in 100 parts by mass of the second matrix from the viewpoint of improving mechanical characteristic. Further, from the viewpoint of improving mechanical characteristic, the compounding amount of the polymerizable monomer having two or more radical polymerizable groups and having no acidic group is preferably set to 40 to 100 parts by mass, more preferably 60 to 100 parts by mass in the 100 parts by mass of the (C) polymerizable monomer having no acidic group which is contained in the first matrix and the second matrix. When the compounding amount of the polymerizable monomer having two or more radical polymerizable groups and having no acidic group is less than 40 parts by mass, there is a case where mechanical properties is lowered.

When the dental adhesive composition of the present invention is a composition containing (A) silane coupling material containing (A1) silane coupling material represented by structural formula of [Chemical formula 1], (B) polymerizable monomer having an acidic group, (C) polymerizable monomer having no acidic group, (H) volatile organic solvent and (G) water, and further containing one or both of (D) polymerization initiator and (E) polymerization accelerator, the compounding amount of the (C) polymerizable monomer having no acidic group is preferably 5 to 60 parts by mass in 100 parts by mass of the dental adhesive composition. When the compounding amount is less than 5 part by mass, there is a case where the mechanical properties in the case of using as a dental adhesive material is low. When the compounding amount is more than 60 part by mass, there is a risk that the coating thickness in the case of applying to the adherend surface becomes thicker, and a good operability is not obtained. Further, the compounding amount of the polymerizable monomer having two or more radical polymerizable groups and having no acidic group among the (C) polymerizable monomer having no acidic group is preferably set to 40 to 100 parts by mass in the 100 parts by mass of the (C) polymerizable monomer having no acidic group. When the compounding amount of the polymerizable monomer having two or more radical polymerizable groups and having no acidic group is less than 40 parts by mass, there is a case where mechanical properties is lowered.

In the present invention, the (C) polymerizable monomer having no acidic group contains (C1) polymerizable monomer having no acidic group and having one or more hydroxyl groups. In the present invention, the (C1) polymerizable monomer having no acidic group and having one or more hydroxyl groups and the (A1) silane coupling material represented by structural formula of [Chemical formula 1] coexist in at least one matrix.

A plurality of kinds of the (C1) polymerizable monomer having no acidic group and having one or more hydroxyl groups can be used in combination, if necessary.

When the dental adhesive composition of the present invention is a composition consisting of a matrix containing (A) silane coupling material containing (A1) silane coupling material represented by structural formula of [Chemical formula 1], (B) polymerizable monomer having an acidic group, (C) polymerizable monomer having no acidic group and (D) polymerization initiator, the compounding amount of the (C1) polymerizable monomer having no acidic group and having one or more hydroxyl groups is preferably 0.1 to 70 parts by mass, more preferably 5 to 60 parts by mass, with respect to 100 parts by mass of the matrix containing (A1) silane coupling material represented by structural formula of [Chemical formula 1]. By compounding the (A1) silane coupling material represented by structural formula of [Chemical formula 1] in the matrix, high adhesive strength to glass ceramics containing lithium disilicate can be expected, and in addition, further improvement in adhesive strength to glass ceramics containing lithium disilicate and storage stability can be expected by compounding the (A1) silane coupling material represented by structural formula of [Chemical formula 1] and the (C1) polymerizable monomer having no acidic group and having one or more hydroxyl groups in the same matrix. When the compounding amount is less than 0.1 parts by mass, higher adhesive strength to glass ceramics containing lithium disilicate cannot be expected. When the compounding amount is more than 70 parts by mass, there is a concern that the storage stability is reduced.

When the dental adhesive composition of the present invention is a two-paste type dental adhesive composition containing (D1) chemical polymerization initiator and (E) polymerization accelerator and containing 15 to 80 parts by mass of (C1) polymerizable monomer having no acidic group and having one or more hydroxyl groups with respect to 100 parts by mass of the first matrix and the second matrix, the compounding amount of the (C1) polymerizable monomer having no acidic group and having one or more hydroxyl groups is 15 to 80 parts by mass with respect to 100 parts by mass of the total amount of the first matrix and the second matrix which are contained in the dental adhesive composition. As a result, adhesive strength to the dental restorative material for cutting and machining is improved. The compounding amount is preferably 20 to 70 parts by mass. When the compounding amount is less than 15 parts by mass, good adhesive strength to the dental restorative material for cutting and machining is not exhibited. On the other hand, when the compounding amount is more than 80 parts by mass, the storage stability is reduced.

When the dental adhesive composition of the present invention is a composition containing (A) silane coupling material containing (A1) silane coupling material represented by structural formula of [Chemical formula 1], (B) polymerizable monomer having an acidic group, (C) polymerizable monomer having no acidic group, (H) volatile organic solvent and (G) water, and further containing one or both of (D) polymerization initiator and (E) polymerization accelerator, the compounding amount of the (C1) polymerizable monomer having no acidic group and having one or more hydroxyl groups is 20 to 70 parts by mass with respect to 100 parts by mass of the total amount of the (A) silane coupling material containing the (A1) silane coupling material represented by structural formula of [Chemical formula 1], the (B) polymerizable monomer having an acidic group and the (C) polymerizable monomer having no acidic group. As a result, the adhesive strength to the dental resin for cutting and machining can be improved, and further, the storage stability improvement of the silane coupling material contained in the composition can be expected. More preferably, the compounding amount of the (C1) polymerizable monomer having no acidic group and having one or more hydroxyl groups is preferably 30 to 60 parts by mass. When the compounding amount exceeds 70 parts by mass, there is a case where the storage stability decrease, and when the compounding amount is less than 20 parts by mass, there is a case where the expected adhesive strength to the dental resin for cutting and machining is not exhibited.

Any known polymerizable monomer having no acidic group and having one or more hydroxyl groups can be used as the (C1) polymerizable monomer having no acidic group and having one or more hydroxyl groups without any

limitation. A polymerizable monomer in which the polymerizable group exhibits radical polymerizability is preferable. Specifically, from the viewpoint of easy radical polymerization, (meth) acrylic group and/ or (meth) acrylamide group is preferable as the polymerizable group. Specific examples include 2-hydroxyethyl (meth) acrylate, 3-hydroxypropyl (meth) acrylate, 4-hydroxybutyl (meth) acrylate, 2-hydroxypropyl (meth) acrylate, 2-hydroxybutyl (meth) acrylate, 6-hydroxyhexyl (meth) acrylate, 10-hydroxydecyl (meth) acrylate, propylene glycol mono (meth) acrylate, glycerol mono (meth) acrylate, erythritol mono (meth) acrylate, N-methylol (meth) acrylamide, N-hydroxyethyl (meth) acrylamide, N,N-(dihydroxyethyl) (meth) acrylamide, 2,2-bis [4-(3-(meth) acryloyloxy)-2-hydroxypropoxyphenyl] propane, glycerol di (meth) acrylate, 1-(acryloyloxy)-3-(methacryloyloxy)-2-propanol, pentaerythritol triacrylate, 2-hydroxy-3-phenoxypropyl acrylate, and 1,4-cyclohexane dimethanol monoacrylate. From the viewpoint of mechanical strength of the prepared dental adhesive composition and the affinity between the curable composition and the adherend, 2-hydroxyethyl (meth) acrylate, 2,2-bis [4-(3-(meth) acryloyloxy)-2-hydroxypropoxyphenyl] propane, glycerol di (meth) acrylate, 1-(acryloyloxy)-3-(methacryloyloxy)-2-propanol are preferable, and 2-bis [4-(3-(meth) acryloyloxy)-2-hydroxy propoxyphenyl] propane, glycerol di (meth) acrylate and 1-(acryloyloxy)-3-(methacryloyloxy)-2-propanol which have two polymerizable groups are more preferable.

Further, it is preferable that the first matrix containing the (A) silane coupling material containing the (A1) silane coupling material represented by structural formula of [Chemical formula 1] contains (C11) low viscosity polymerizable monomer having no acidic group and one or more hydroxyl groups and having 200 mPa ·s or less of viscosity at 25 °C, which has high fluidity at room temperature among the (C1) polymerizable monomer having no acidic group and having one or more hydroxyl groups. This low viscosity polymerizable monomer has a viscosity of 200 mPa ·s or less measured with the B-type viscometer at 25 °C. In the case of measuring the (C1) polymerizable monomer having no acidic group and having one or more hydroxyl groups having a viscosity of less than 1000 mPa ·s, B-type viscometer BM type is connected with No.1 rotor and the rotation speed is gradually increased to full scale to measure. When it cannot be measured with the No.1 rotor, it exceeds 200 mPa ·s. Further, in the case of measuring the viscosity of a polymerizable monomer having a viscosity of 15 mPa ·s or less with high accuracy, it is preferable to use a B-type viscometer BL type. As the (C11) low viscosity polymerizable monomer having no acidic group and one or more hydroxyl groups and having 200 mPa ·s or less of viscosity at 25 °C, any known low viscosity polymerizable monomer can be used without limitation and examples include 2-hydroxyethyl (meth) acrylate, 2-hydroxypropyl (meth) acrylate, 2-hydroxybutyl (meth) acrylate, propylene glycol mono (meth) acrylate, glycerol mono (meth) acrylate, glycerol di (meth) acrylate and 1-(acryloyloxy)-3-(methacryloyloxy)-2-propanol. The compounding amount of the (C11) low viscosity polymerizable monomer having no acidic group and one or more hydroxyl groups and having 200 mPa ·s or less of viscosity at 25 °C is preferably 0.1 parts by mass or more and 50 parts by mass or less, more preferably 0.1 to 40 parts by mass, and further preferably 5 to 30 parts by mass with respect to 100 parts by mass of the first matrix. When the compounding amount is less than 0.1 parts by mass, there is a case where a remarkable effect of improving adhesive property to the dental restorative material for cutting and machining is not exhibited, and when the compounding amount exceeds 50 parts by mass, there is a case where the storage stability is deteriorated.

The dental adhesive composition of the present invention may contain (I) polymerizable monomer having one or more sulfur atoms in order to impart adhesive property with respect to a noble metal. As the (I) polymerizable monomer having one or more sulfur atoms of the present invention, any known compound can be used without any limitation as long as it is a polymerizable monomer having at least one functional group having a sulfur group and one or more polymerizable groups in a molecule. Among the functional groups having a sulfur group, those that do not form a coordination bond with a noble metal such as a sulfo group are not included in this. A functional group having a sulfur group is formed from a partial structure such as >P=S, >C=S, >CSC< and the like. Examples of the sulfur atom-containing polymerizable monomer include a compound that can generate a mercapto group by tautomerism, a disulfide compound, thiophosphoric acid, a chain or cyclic thioether compound and the like. Specific examples include 10-methacryloxy decyl-6,8-dithiooctanate, 6-methacryloxy hexyl-6,8-dithiooctanate, 6-methacryloyl oxyhexyl-2-thiouracil-5-carboxylate, 2-(11-methacryloyloxy undecylthio)-5-mercapto-1,3,4-thiadiazole, 8-(meth) acryloyloxy octyl dihydrogenthiophosphate, 10-(meth) acryloyloxy decyl dihydrogenthiophosphate. The compounding amount of the (I) polymerizable monomer having one or more sulfur atoms is preferably 0.01 to 10 parts by mass with respect to 100 parts by mass of the dental adhesive composition. When the compounding amount is less than 0.01 parts by mass, there is a case where good adhesive property to the noble metal is not exhibited. When the compounding amount exceeds 10 parts by mass, there is a case where improvement in adhesive property proportional to the compounding amount is not exhibited.

In order to increase the relative polymerization rate of the (A) silane coupling material in the composition, it is preferable that the compounding amount of a compound having a methacryloyl group and/or a methacrylamide group is 50 to 99 parts by mass with respect to 100 parts by mass of the matrix. The compound having a methacryloyl group and/or a methacrylamide group referred to here may or may not have an acidic group. More preferably, the compounding amount is 70 to 100 parts by mass. When the compounding amount is less than 50 parts by mass, there is a case where the durable adhesive strength to glass ceramics containing lithium disilicate decreases. On the other hand, higher adhesive strength can be expected as compared with the case where a conventional silane coupling material having a

methacryloyl group is used. In addition, a proportion of the compound having a meth acryloyl group and/or a methacrylamide group may be 50 to 99 parts by mass in 100 parts by mass of a total amount of the (A) silane coupling material containing the (A1) silane coupling material represented by structural formula of [Chemical formula 1], the (B) polymerizable monomer having an acidic group and the (C) polymerizable monomer having no acidic group.

When the dental adhesive composition of the present invention is a composition consisting of a matrix containing (A) silane coupling material containing (A1) silane coupling material represented by structural formula of [Chemical formula 1], (B) polymerizable monomer having an acidic group, (C) polymerizable monomer having no acidic group and (D) polymerization initiator, the compounding amount of the (D) polymerization initiator is preferably 0.3 to 6 parts by mass with respect to 100 parts by mass of the matrix.

When the dental adhesive composition of the present invention is a two-paste type dental adhesive composition containing (D1) chemical polymerization initiator and (E) polymerization accelerator and containing 15 to 80 parts by mass of (C1) polymerizable monomer having no acidic group and having one or more hydroxyl groups with respect to 100 parts by mass of the first matrix and the second matrix, the compounding amount of the (D1) chemical polymerization initiator is preferably set to 0.1 to 5 parts by mass, more preferably set to 0.5 to 2 parts by mass, with respect to 100 parts by mass of the total amount of the first matrix and the second matrix from the viewpoint of improving curability. When the compounding amount of the (D1) chemical polymerization initiator is more than 5.0 parts by mass, it may be difficult to ensure sufficient operation time. On the other hand, when the compounding amount of the (D1) chemical polymerization initiator is less than 0.1 parts by mass, there is a case where mechanical strength is insufficient.

When the dental adhesive composition of the present invention is a composition containing (A) silane coupling material containing (A1) silane coupling material represented by structural formula of [Chemical formula 1], (B) polymerizable monomer having an acidic group, (C) polymerizable monomer having no acidic group, (H) volatile organic solvent and (G) water, and one or both of (D) polymerization initiator and (E) polymerization accelerator, the dental adhesive composition contains one or both of the (D) polymerization initiator and the (E) polymerization accelerator. The compounding amount of the (D) polymerization initiator is preferably 0.01 to 5 parts by mass in 100 parts by mass of the dental adhesive composition. The compounding amount of the (E) polymerization accelerator may be 0.01 to 5 parts by mass in 100 parts by mass of the dental adhesive composition.

(D2) photopolymerization initiator may be compounded as the (D) polymerization initiator in the dental adhesive composition of the present invention in order to impart photopolymerizability. Specific examples of the (D2) photopolymerization initiator include α-diketones, mono-, bis-, or tris acylphosphine oxide compound and mono- or di-acylgermanium compound.

The compounding amount of the (D2) photopolymerization initiator is not particularly limited, however, when the dental adhesive composition of the present invention is a composition consisting of a matrix containing (A) silane coupling material containing (A1) silane coupling material represented by structural formula of [Chemical formula 1], (B) polymerizable monomer having an acidic group, (C) polymerizable monomer having no acidic group and (D) polymerization initiator, wherein the (A1) silane coupling material represented by structural formula of [Chemical formula 1] has an acryloyl group, the compounding amount is preferably 0.01 to 5 parts by mass, more preferably 0.1 to 3 parts by mass, with respect to 100 parts by mass of the matrix from the viewpoint of photocurability.

When the dental adhesive composition of the present invention is a two-paste type dental adhesive composition containing (D1) chemical polymerization initiator and (E) polymerization accelerator and containing 15 to 80 parts by mass of (C1) polymerizable monomer having no acidic group and having one or more hydroxyl groups with respect to 100 parts by mass of the first matrix and the second matrix, the compounding amount is preferably 0.01 to 5 parts by mass, more preferably 0.1 to 3 parts by mass, with respect to 100 parts by mass of the total amount of the polymerizable monomer from the viewpoint of photocurability.

When the dental adhesive composition of the present invention is a composition containing (A) silane coupling material containing (A1) silane coupling material represented by structural formula of [Chemical formula 1], (B) polymerizable monomer having an acidic group, (C) polymerizable monomer having no acidic group, (H) volatile organic solvent and (G) water, and further containing one or both of (D) polymerization initiator and (E) polymerization accelerator, the compounding amount is preferably 0.01 to 5 parts by mass, more preferably 0.1 to 3 parts by mass, with respect to 100 parts by mass of the dental adhesive composition from the viewpoint of improving curability.

Specific examples of α-diketones include diacetyl, dibenzyl, camphorquinone, 2,3-pentadione, 2,3-octadione, 9,10-phenanthrenequinone, 4,4'-oxybenzyl, acenaphthenequinone and the like. Among these, camphor quinone is preferable because it is excellent in photocurability in the visible and near-ultraviolet regions and exhibits sufficient photocurability even if any light source of a halogen lamp, a light emitting diode (LED) and a xenon lamp are used.

Examples of mono-, bis-, or tris acylphosphine oxide compound include bis (2,6-dimethoxy benzoyl) phenyl phosphine oxide, bis (2,6-dimethoxy benzoyl) (2,4,4-trimethyl pentyl) phosphine oxide, bis (2,6-dimethoxy benzoyl)-n-butyl phosphine oxide, bis (2,6-dimethoxy benzoyl)-(2-methylprop-1-yl) phosphine oxide, bis (2,6-dimethoxy benzoyl)-(1-methylprop-1-yl) phosphine oxide, bis (2,6-dimethoxy benzoyl)-t-butyl phosphine oxide, bis (2,6-dimethoxy benzoyl) cyclohexyl phosphine oxide, bis (2,6-dimethoxy benzoyl) octyl phosphine oxide, bis (2-methoxy benzoyl) (2-methylprop-1-yl) phosphine oxide, bis (2-methoxy benzoyl) (1-methylprop-1-yl) phosphine oxide, bis (2,6-diethoxy benzoyl) (2 -methylprop-1-yl) phosphine oxide, bis (2,6-diethoxy benzoyl) (1-methylprop-1-yl) phosphine oxide, bis (2,6-dibutoxy benzoyl) (2-methylprop-1-yl) phosphine oxide, bis (2,4-dimethoxy benzoyl) (2-methylprop-1-yl) phosphine oxide, bis (2,4,6-trimethyl benzoyl) phenyl phosphine oxide, 2,4,6-trimethyl benzoyl diphenyl phosphine oxide, bis (2,4,6-trimethyl benzoyl) (2,4-dipentoxy phenyl) phosphine oxide, bis (2,6-dimethoxy benzoyl) benzyl phosphine oxide, bis (2,6-dimethoxy benzoyl)-2-phenylpropyl phosphine oxide, bis (2,6-dimethoxy benzoyl)-2-phenylethyl phosphine oxide, bis (2,6-dimethoxy benzoyl) benzyl phosphine oxide, bis (2,6-dimethoxy benzoyl)-2-phenylpropyl phosphine oxide, bis (2,6-dimethoxy benzoyl) -2-phenylethyl phosphine oxide, 2,6-dimethoxy benzoyl benzyl butyl phosphine oxide, 2,6-dimethoxy benzoyl benzyl octyl phosphine oxide, bis (2,4,6-trimethyl benzoyl) isobutyl phosphine oxide, 2,6-dimethoxy benzoyl-2,4,6-trimethyl benzoyl-n-butyl phosphine oxide and the like. Among these, from the viewpoint of photocurability, bis (2,6-dimethoxy benzoyl) (2,4,4-trimethyl pentyl) phosphine oxide and 2,4,6-trimethyl benzoyl diphenyl phosphine oxide are preferable.

Examples of mono- or di-acylgermanium compound include bisbenzoyl diethylgermanium, bisbenzoyl dimethylgermanium, bisbenzoyl dibutylgermanium, bis (4-methoxybenzoyl) dimethylgermanium and bis (4-methoxybenzoyl) diethylgermanium and (4-methoxybenzoyl) diethylgermanium and the like.

Specific examples of an organic peroxide as the (D1) chemical polymerization initiator include diacyl peroxides, peroxy esters, dialkyl peroxides, peroxy ketals, ketone peroxides, peroxy esters, peroxy dicarbonates, and hydro peroxides. Specific examples of diacyl peroxides include acetyl peroxide, isobutyryl peroxide, benzoyl peroxide, decanoyyl peroxide, 3,5,5-trimethylhexanoyl peroxide, 2,4-dichlorobenzoyl peroxide, and lauroyl peroxide and the like. Specific examples of peroxyesters include $\alpha$-cumylperoxy neodecanoate, t-butylperoxy neodecanoate, t-butylperoxy pivalate, 2,2,4-trimethylpentyl peroxy-2-ethyl hexanoate, t-amylperoxy-2-ethylhexanoate, t-butylperoxy-2-ethylhexanoate, di-t-butylperoxy isophthalate, di-t-butylperoxy hexahydro terephthalate, t-butylperoxy-3,3,5-trimethyl hexanoate, t-butylperoxy acetate, t-butylperoxy benzoate and t-butylperoxy maleric acid. Specific examples of dialkyl peroxides include di-t-butyl peroxide, dicumyl peroxide, t-butylcumyl peroxide, 2,5-dimethyl-2,5-di (t-butylperoxy) hexane, 1,3-bis (t-butylperoxy isopropyl) benzene, 2,5-dimethyl-2,5-di (t-butylperoxy)-3-hexyne and the like. Specific examples of ketone peroxides include methyl ethyl ketone peroxide, methyl isobutyl ketone peroxide, methyl cyclohexanone peroxide, cyclohexanone peroxide and the like. Specific examples of peroxyesters include $\alpha$-cumylperoxy neodecanoate, t-butylperoxy neodecanoate, t-butylperoxy pivalate, 2,2,4-trimethylpentyl peroxy-2-ethyl hexanoate, t-amylperoxy-2-ethylhexanoate, t-butylperoxy-2-ethylhexanoate, di-t-butylperoxy isophthalate, di-t-butylperoxy hexahydro terephthalate, t-butylperoxy-3,3,5-trimethyl hexanoate, t-butylperoxy acetate, t-butylperoxy benzoate and t-butylperoxy maleric acid. Specific examples of peroxydicarbonates include di-3-methoxyperoxy dicarbonate, di-2-ethylhexylperoxy dicarbonate, bis (4-t-butylcyclohexyl) peroxy dicarbonate, diisopropylperoxy dicarbonate, di-n-propylperoxy dicarbonate, di-2-ethoxyethylperoxy dicarbonate, diallylperoxy dicarbonate and the like. Specific examples of hydroperoxides include 2,5-dimethyl hexane-2,5-dihydroperoxide, diisopropylbenzene hydroperoxide, cumene hydroperoxide, t-butyl hydroperoxide and 1,1,3,3-tetramethyl butylhydroperoxide.

As the organic peroxide, the above-mentioned organic peroxides may be used alone, or two or more kinds of organic peroxides may be used in combination. Among these organic peroxides, benzoyl peroxide and cumene hydroperoxide are preferable from the viewpoint of curability. It is considered that the photopolymerization initiator alone is not sufficient for adhering the dental restorative material for cutting and machining having high shielding property. The dental restorative material for cutting and machining having high shielding properties is used for restoration of anterior teeth that require aesthetic property. Further, since the glass fiber reinforced resin made of epoxy resin also has a high shielding property, curing by chemical polymerization is effective.

When the dental adhesive composition of the present invention is a composition consisting of a matrix containing (A) silane coupling material containing (A1) silane coupling material represented by structural formula of [Chemical formula 1], (B) polymerizable monomer having an acidic group, (C) polymerizable monomer having no acidic group and (D) polymerization initiator, wherein the (A1) silane coupling material represented by structural formula of [Chemical formula 1] has an acryloyl group, the compounding amount of the organic peroxide as the chemical polymerization initiator is preferably 0.1 to 5 parts by mass, more preferably 0.5 to 3 parts by mass with respect to 100 parts by mass of the matrix from the viewpoint of improving curability. When the compounding amount of the organic peroxide is more than 5 parts by mass, it may be difficult to ensure sufficient operation time. On the other hand, when the compounding amount of the organic peroxide is less than 0.1 parts by mass, there is a case where mechanical strength is insufficient.

When the dental adhesive composition of the present invention is a composition containing (A) silane coupling material containing (A1) silane coupling material represented by structural formula of [Chemical formula 1], (B) polymerizable monomer having an acidic group, (C) polymerizable monomer having no acidic group, (H) volatile organic solvent and (G) water, and further containing one or both of (D) polymerization initiator and (E) polymerization accelerator, the compounding amount of the organic peroxide is preferably 0.1 to 5 parts by mass, more preferably 0.3 to 3 parts by mass with respect to 100 parts by mass of the dental adhesive composition from the viewpoint of improving curability. When the compounding amount of the organic peroxide is more than 5 parts by mass, it may be difficult to ensure sufficient operation time. On the other hand, when the compounding amount of the organic peroxide is less than 0.1

parts by mass, there is a case where mechanical strength is insufficient.

When the dental adhesive composition of the present invention is a two-paste type dental adhesive composition containing (D1) chemical polymerization initiator and (E) polymerization accelerator and containing 15 to 80 parts by mass of (C1) polymerizable monomer having no acidic group and having one or more hydroxyl groups with respect to 100 parts by mass of the first matrix and the second matrix, the compounding amount of the (D1) chemical polymerization initiator is preferably 0.1 to 5.0 parts by mass, more preferably 0.5 to 2.5 parts by mass with respect to 100 parts by mass of the total amount of the first matrix and the second matrix from the viewpoint of improving curability. When the compounding amount of the (D1) chemical polymerization initiator is more than 5.0 parts by mass, it may be difficult to ensure sufficient operation time. On the other hand, when the compounding amount of the (D1) chemical polymerization initiator is less than 0.1 parts by mass, there is a case where mechanical strength is insufficient.

In the dental adhesive composition of the present invention, in order to further improve the curability, (E) polymerization accelerator may be further compounded. Examples of the (E) polymerization accelerator include a transition metal compound of the group 4 in the periodic table, a thiourea derivative an aliphatic amine, an aromatic amine, a sulfinic acid and a salt thereof, a borate compound, a sulfur-containing reductive inorganic compound, a nitrogen-containing reductive inorganic compound, a borate compound, a barbituric acid derivative, a triazine compound, a halogen compound and the like.

When the dental adhesive composition of the present invention is a composition consisting of a matrix containing (A) silane coupling material containing (A1) silane coupling material represented by structural formula of [Chemical formula 1], (B) polymerizable monomer having an acidic group, (C) polymerizable monomer having no acidic group and (D) polymerization initiator, wherein the (A1) silane coupling material represented by structural formula of [Chemical formula 1] has an acryloyl group, the compounding amount of the (E) polymerization accelerator is preferably 0.01 to 3 parts by mass, more preferably 0.1 to 3.0 parts by mass with respect to 100 parts by mass of the matrix.

When the dental adhesive composition of the present invention is a two-paste type dental adhesive composition containing (D1) chemical polymerization initiator and (E) polymerization accelerator and containing 15 to 80 parts by mass of (C1) polymerizable monomer having no acidic group and having one or more hydroxyl groups with respect to 100 parts by mass of the first matrix and the second matrix, the compounding amount of the (E) polymerization accelerator is preferably 0.01 to 5.0 parts by mass, more preferably 0.2 to 2.5 parts by mass, with respect to 100 parts by mass of the total amount of the first matrix and the second matrix. When the compounding amount of the (E) polymerization accelerator is more than 5.0 parts by mass, it may be difficult to ensure sufficient operation time. On the other hand, when the compounding amount of the (E) polymerization accelerator is less than 0.01 parts by mass, there is a case where mechanical strength is insufficient.

In this case, the first matrix and the second matrix contain any one or more of (D1) chemical polymerization initiator and (E) polymerization accelerator. When the first matrix contains one or more (D1) chemical polymerization initiator, the second matrix contains one or more (E) polymerization accelerator. When the first matrix contains one or more (E) polymerization accelerator, the second matrix contains one or more (D1) chemical polymerization initiator.

For example, it is preferable that the first matrix contains (D1) chemical polymerization initiator and the second matrix contains (E) polymerization accelerator, or that the first matrix contains (E) polymerization accelerator and the second matrix contains (D1) chemical polymerization initiator. When the (D1) chemical polymerization initiator and the (E) polymerization accelerator are in a combination that does not affect the storage stability, these can be compounded in the same matrix. For example, even if an organic peroxide which is the (D1) chemical polymerization initiator and 4-N,N-dimethylaminobenzoic acid ethyl ester which is an aromatic amine compound and the (E) polymerization accelerator may be present in the same matrix, in the case of low concentration, the redox reaction does not occur instantly, and high storage stability is observed. Therefore, the (D1) chemical polymerization initiator and the (E) polymerization accelerator can be compounded in the same matrix. In this case, the other matrix contains the (D1) chemical polymerization initiator and/or the (E) polymerization accelerator in order to induce chemical polymerization in kneading the first paste and the second paste.

When the dental adhesive composition of the present invention is a composition containing (A) silane coupling material containing (A1) silane coupling material represented by structural formula of [Chemical formula 1], (B) polymerizable monomer having an acidic group, (C) polymerizable monomer having no acidic group, (H) volatile organic solvent and (G) water, and further containing one or both of (D) polymerization initiator and (E) polymerization accelerator, the compounding amount of the (E) polymerization accelerator is preferably 0.01 to 5 parts by mass, more preferably 0.1 to 3.0 parts by mass, with respect to 100 parts by mass of the dental adhesive composition.

The transition metal compound of the period 4 in the periodic table refers to a metal compound of groups 3 to 12 of the period 4 in the periodic table, and specifically, each metal compounds of scandium (Sc), titanium (Ti), vanadium (V), manganese (Mn), iron (Fe), cobalt (Co), nickel (Ni), copper (Cu), and zinc (Zn) can be used without any limitation. Although each of the above transition metal element may have a multiple valences, they can be added to the dental adhesive composition of the present invention as long as the valence is stable. Examples include Sc (trivalent), Ti (tetravalent), V (trivalent, tetravalent or pentavalent), Cr (divalent, trivalent or hexavalent), Mn (divalent to heptavalent),

Fe (divalent or trivalent), Co (divalent or trivalent), Ni (divalent), Cu (monovalent or divalent), Zn (divalent). Specific examples of the transition metal compound include scandium iodide (trivalent) and the like as a scandium compound, titanium chloride (tetravalent), titanium tetraisopropoxide (tetravalent) and the like as titanium compounds, acetylacetone vanadium (trivalent), divanadium tetraoxide (tetravalent), vanadylacetyl acetonate (tetravalent), vanadium stearate oxide (tetravalent), vanadyl oxalate (tetravalent), vanazyl sulfate (tetravalent), oxobis (1-phenyl-1,3-butandionate) vanadium (tetravalent), bis (maltlate) oxovanadium (tetravalent), vanadium pentoxide (pentavalent), sodium metavanadate (pentavalent) and the like as a vanadium compound, manganese acetate (divalent), manganese naphthenate (divalent) and the like as manganese compounds, iron acetate (divalent), iron chloride (divalent), iron acetate (trivalent), iron chloride (trivalent) and the like as an iron compound, cobalt acetate (divalent), cobalt naphthenate (divalent) and the like as a cobalt compound, nickel chloride (divalent) and the like as a nickel compound, copper chloride (monovalent), copper bromide (monovalent), copper chloride (divalent), copper acetate (divalent) and the like as a copper compound, and zinc chloride (divalent), zinc acetate (divalent) and the like as a zinc compound.

Among these, a trivalent or tetravalent vanadium compound and a divalent copper compound are preferable. Among them, because of having higher polymerization accelerating ability, a trivalent or tetravalent vanadium compound is more preferable, and a tetravalent vanadium compound is most preferable. A plurality of kinds of these transition metal compounds in the period 4 in the periodic table may be used in combination, if necessary.

When the dental adhesive composition of the present invention is a composition consisting of a matrix containing (A) silane coupling material containing (A1) silane coupling material represented by structural formula of [Chemical formula 1], (B) polymerizable monomer having an acidic group, (C) polymerizable monomer having no acidic group and (D) polymerization initiator, wherein the (A1) silane coupling material represented by structural formula of [Chemical formula 1] has an acryloyl group, the compounding amount of the transition metal compound is preferably 0.001 to 1 parts by mass with respect to 100 parts by mass of the total amount of the matrix. When the compounding amount is less than 0.001 parts by mass, there is a case where the polymerization accelerating effect is insufficient, and when the compounding amount exceeds 1 part by mass, there is a case where it causes discoloration or gelation of the dental adhesive composition and the storage stability is lowered.

When the dental adhesive composition of the present invention is a two-paste type dental adhesive composition containing (D1) chemical polymerization initiator and (E) polymerization accelerator and containing 15 to 80 parts by mass of (C1) polymerizable monomer having no acidic group and having one or more hydroxyl groups with respect to 100 parts by mass of the first matrix and the second matrix, the compounding amount of the transition metal compound is preferably 0.001 to 1 parts by mass with respect to 100 parts by mass of the total amount of the polymerizable monomer. When the compounding amount is less than 0.001 parts by mass, there is a case where the polymerization accelerating effect is insufficient, and when the compounding amount exceeds 1 part by mass, there is a case where it causes discoloration or gelation of the dental curable adhesive composition and the storage stability is lowered.

When the dental adhesive composition of the present invention is a composition containing (A) silane coupling material containing (A1) silane coupling material represented by structural formula of [Chemical formula 1], (B) polymerizable monomer having an acidic group, (C) polymerizable monomer having no acidic group, (H) volatile organic solvent and (G) water, and further containing one or both of (D) polymerization initiator and (E) polymerization accelerator, the compounding amount of the transition metal compound is preferably 0.001 to 1 parts by mass with respect to 100 parts by mass of the dental adhesive composition. When the compounding amount is less than 0.001 parts by mass, there is a case where the polymerization accelerating effect is insufficient, and when the compounding amount exceeds 1 part by mass, there is a case where it causes discoloration or gelation of the dental adhesive composition and the storage stability is lowered.

Any known thiourea derivatives can be used as the thiourea derivative without any limitation. Specific examples of the thiourea derivatives include dimethylthiourea, diethylthiourea, tetramethylthiourea, (2-pyridyl) thiourea, N-methylthiourea, ethylenethiourea, N-allylthiourea, N-allyl-N'-(2-hydroxyethyl) thiourea, N-benzylthiourea, 1,3-dicyclohexyl thiourea, N,N'-diphenylthiourea, 1,3-di (p-tolyl) thiourea, 1-methyl-3-phenylthiourea, N-acetylthiourea, N-benzoylthiourea, diphenylthiourea, dicyclohexylthiourea and the like. Among these, (2-pyridyl) thiourea, N-acetylthiourea and N-benzoylthiourea are preferable. A plurality of kinds of these thiourea derivatives can be used in combination, if necessary.

When the dental adhesive composition of the present invention is a composition consisting of a matrix containing (A) silane coupling material containing (A1) silane coupling material represented by structural formula of [Chemical formula 1], (B) polymerizable monomer having an acidic group, (C) polymerizable monomer having no acidic group and (D) polymerization initiator, wherein the (A1) silane coupling material represented by structural formula of [Chemical formula 1] has an acryloyl group, the compounding amount of the thiourea derivative is preferably 0.1 to 4 parts by mass with respect to 100 parts by mass of the total amount of the matrix. When the compounding amount is less than 0.1 parts by mass, there is a case where the polymerization accelerating ability is insufficient, and when the compounding amount exceeds 4 parts by mass, there is a case where the storage stability is lowered.

When the dental adhesive composition of the present invention is a two-paste type dental adhesive composition containing (D1) chemical polymerization initiator and (E) polymerization accelerator and containing 15 to 80 parts by

mass of (C1) polymerizable monomer having no acidic group and having one or more hydroxyl groups with respect to 100 parts by mass of the first matrix and the second matrix, the compounding amount of the thiourea derivative is preferably 0.1 to 4 parts by mass with respect to 100 parts by mass of the total amount of the polymerizable monomer. When the compounding amount is less than 0.1 parts by mass, there is a case where the polymerization accelerating ability is insufficient, and when the compounding amount exceeds 4 parts by mass, there is a case where the storage stability is lowered.

When the dental adhesive composition of the present invention is a composition containing (A) silane coupling material containing (A1) silane coupling material represented by structural formula of [Chemical formula 1], (B) polymerizable monomer having an acidic group, (C) polymerizable monomer having no acidic group, (H) volatile organic solvent and (G) water, and further containing one or both of (D) polymerization initiator and (E) polymerization accelerator, the compounding amount of the thiourea derivative is preferably 0.1 to 4 parts by mass with respect to 100 parts by mass of the dental adhesive composition. When the compounding amount is less than 0.1 parts by mass, there is a case where the polymerization accelerating ability is insufficient, and when the compounding amount exceeds 4 parts by mass, there is a case where the storage stability is lowered.

Specific examples of aliphatic amine include primary aliphatic amines such as n-butylamine, n-hexylamine and n-octylamine; secondary aliphatic amines such as diisopropylamine and dibutylamine; tertiary aliphatic amines such as N-methyl diethanolamine, N-ethyl diethanolamine, N-n-butyl diethanolamine, N-lauryl diethanolamine, 2-(dimethylamino) ethyl (meth) acrylate, N-methyl diethanolamine di (meth) acrylate, N-ethyl diethanolamine di (meth) acrylate, triethanolamine mono (meth) acrylate, triethanolamine di (meth) acrylate, triethanolamine tri (meth) acrylate, triethanolamine, trimethylamine, triethylamine and tributylamine and the like. Among these, tertiary aliphatic amines are preferable from the viewpoint of the curability and storage stability of the composition, and among them, 2-(dimethyl amino) ethyl (meth) acrylate, N-methyl diethanolamine di (meth) acrylate and triethanolamine are preferable.

Specific examples of the aromatic amine compound include N,N-bis(2-hydroxyethyl)-3,5-dimethylaniline, N,N-di(2-hydroxyethyl)-p-toluidine, N,N-bis(2-hydroxyethyl)-3,4-dimethylaniline, N,N-bis(2-hydroxyethyl)-4-ethylaniline, N,N-bis(2-hydroxyethyl)-4-isopropylaniline, N,N-bis(2-hydroxyethyl)-4-t-butylaniline, N,N-bis(2-hydroxyethyl)-3,5-di-isopropylaniline, N,N-bis(2-hydroxyethyl)-3,5-di-t-butylaniline, N,N-dimethylaniline, N,N-dimethyl-p-toluidine, N,N-dimethyl-m-toluidine, N,N-diethyl-p-toluidine, N,N-dimethyl-3,5-dimethylaniline, N,N-dimethyl-3,4-dimethylaniline, N,N-dimethyl-4-ethylaniline, N,N-dimethyl-4-isopropylaniline, N,N-dimethyl-4-t-butylaniline, N,N-dimethyl-3,5-di-t-butylaniline, 4-N,N-dimethylamino benzoic acid ethyl ester, 4-N,N-dimethylamino benzoic acid methyl ester, N,N-dimethylamino benzoic acid-n-butoxyethyl ester, 4-N,N-dimethylamino benzoic acid-2-(methacryloyloxy) ethyl ester, 4-N,N-dimethylamino benzophenone, and 4-dimethylamino benzoic acid butyl. Among these, N,N-di (2-hydroxyethyl)-p-toluidine, 4-N,N-dimethylamino benzoic acid ethyl ester, and N, N-dimethylamino benzoic acid-n-butoxyethyl ester are preferable from the viewpoint of excellent solubility in polymerizable monomer, storage stability and imparting excellent curability to the composition.

Examples of sulfinic acid and its salt include p-toluene sulfinic acid, sodium p-toluene sulfinate, potassium p-toluene sulfinate, lithium p-toluene sulfinate, calcium p-toluene sulfinate, benzenesulfinic acid, sodium benzene sulfinate, potassium benzene sulfinate, lithium benzenesulfinate, calcium benzenesulfinate, 2,4,6-trimethyl benzenesulfinic acid, sodium 2,4,6-trimethyl benzenesulfinate, potassium 2,4,6-trimethyl benzenesulfinate, lithium 2,4,6-trimethyl benzenesulfinate, calcium 2,4,6-trimethyl benzenesulfinate, 2,4,6-triethyl benzenesulfinic acid, sodium 2,4,6-triethyl benzenesulfinate, potassium 2,4,6-triethyl benzenesulfinate, lithium 2,4,6-triethyl benzenesulfinate, calcium 2,4,6-triethyl benzenesulfinate, 2,4,6-triisopropyl benzenesulfinic acid, sodium 2,4,6-triisopropyl benzenesulfinate, potassium 2,4,6-triisopropyl benzenesulfinate, lithium 2,4,6-triisopropyl benzenesulfinate, calcium 2,4,6-triisopropyl benzenesulfinate and the like. Among them, sodium benzenesulfinate, sodium p-toluenesulfinate, and sodium 2,4,6-triisopropyl benzenesulfinate are particularly preferable.

As the borate compound, specific examples of the borate compound having one aryl group in one molecule include trialkylphenylboron, trialkyl (p-chlorophenyl) boron, trialkyl (p-fluorophenyl) boron, trialkyl (3,5-bistrifluoro methyl) phenyl boron, trialkyl [3,5-bis (1,1,1,3,3,3-hexafluoro-2-methoxy-2-propyl) phenyl] boron, trialkyl (p-nitrophenyl) boron, trialkyl (m-nitrophenyl) boron, trialkyl (p-butylphenyl) boron, trialkyl (m-butylphenyl) boron, trialkyl (p-butyloxyphenyl) boron, trialkyl (m-butyloxyphenyl) boron, trialkyl (p-octyloxyphenyl) boron and trialkyl (m-octyloxyphenyl) boron (the alkyl group is at least one selected from the group consisting of n-butyl group, n-octyl group and n-dodecyl group etc.) and salts thereof (sodium salt, lithium salt, potassium salt, magnesium salt, tetrabutyl ammonium salt, tetramethyl ammonium salt, tetraethyl ammonium salt, methyl pyridinium salt, ethyl pyridinium salt, butyl pyridinium salt, methyl quinolinium salt, ethyl quinolinium salt, butyl quinolinium salt and the like). Specific examples of the borate compound having two aryl groups in one molecule include dialkyl diphenylboron, dialkyl di (p-chlorophenyl) boron, dialkyl di (p-fluorophenyl) boron, dialkyl di (3,5-bistrifluoro methyl) phenyl boron, dialkyl di [3,5-bis (1,1,1,3,3,3-hexafluoro-2-methoxy-2-propyl) phenyl] boron, dialkyl di (p-nitrophenyl) boron, dialkyl di (m-nitrophenyl) boron, dialkyl di (p-butylphenyl) boron, dialkyl di (m-butylphenyl) boron, dialkyl di (p-butyl oxyphenyl) boron, dialkyl di (m-butyl oxyphenyl) boron, dialkyl di (p-octyl oxyphenyl) boron and dialkyl di (m-octyl oxyphenyl) boron (the alkyl group is at least one selected from the group consisting of n-butyl group, n-octyl group and n-dodecyl group etc.) and salts thereof (sodium salt, lithium salt, potassium salt, magnesium salt, tetrabutyl ammonium salt, tetramethyl ammonium salt, tetraethyl ammonium salt, methyl pyridinium salt, ethyl pyridinium salt, butyl pyridinium salt, methyl quinolinium salt, ethyl quinolinium salt, butyl quinolinium salt and the like). Specific examples of the borate compound having three aryl groups in one molecule include monoalkyl triphenylboron, monoalkyl tri (p-chlorophenyl) boron, monoalkyl tri (p-fluorophenyl) boron, monoalkyl tri (3,5-bistrifluoro methyl) phenyl boron, monoalkyl tri [3,5-bis (1,1,1,3,3,3-hexafluoro-2-methoxy-2-propyl) phenyl] boron, monoalkyl tri (p-nitrophenyl) boron, monoalkyl tri (m-nitrophenyl) boron, monoalkyl tri (p-butylphenyl) boron, monoalkyl tri (m-butylphenyl) boron, monoalkyl tri (p-butyl oxyphenyl) boron, monoalkyl tri (m-butyl oxyphenyl) boron, monoalkyl tri (p-octyl oxyphenyl) boron and monoalkyl tri (m-octyl oxyphenyl) boron (the alkyl group is at least one selected from the group consisting of n-butyl group, n-octyl group and n-dodecyl group etc.) and salts thereof (sodium salt, lithium salt, potassium salt, magnesium salt, tetrabutyl ammonium salt, tetramethyl ammonium salt, tetraethyl ammonium salt, methyl pyridinium salt, ethyl pyridinium salt, butyl pyridinium salt, methyl quinolinium salt, ethyl quinolinium salt, butyl quinolinium salt and the like). Specific examples of the borate compound having four aryl groups in one molecule include tetraphenylboron, tetra kis (p-chlorophenyl) boron, tetra kis (p-fluorophenyl) boron, tetra kis (3,5-bistrifluoro methyl) phenyl boron, tetra kis [3,5-bis (1,1,1,3,3,3-hexafluoro-2-methoxy-2-propyl) phenyl] boron, tetra kis (p-nitrophenyl) boron, tetra kis (m-nitrophenyl) boron, tetra kis (p-butylphenyl) boron, tetra kis (m-butylphenyl) boron, tetra kis (p-butyl oxyphenyl) boron, tetra kis (m-butyl oxyphenyl) boron, tetra kis (p-octyl oxyphenyl) boron, tetra kis (m-octyl oxyphenyl) boron, (p-fluorophenyl) triphenylboron, (3,5-bis trifluoromethyl) phenyl triphenylboron, (p-nitrophenyl) triphenylboron, (m-butyl oxyphenyl) triphenylboron, (p-butyl oxyphenyl) triphenylboron, (m-octyl oxyphenyl) triphenylboron and (p-octyl oxyphenyl) triphenylboron, and salts thereof (sodium salt, lithium salt, potassium salt, magnesium salt, tetrabutyl ammonium salt, tetramethyl ammonium salt, tetraethyl ammonium salt, methyl pyridinium salt, ethyl pyridinium salt, butyl pyridinium salt, methyl quinolinium salt, ethyl quinolinium salt, butyl quinolinium salt and the like).

Among these aryl borate compounds, it is more preferable to use a borate compound having 3 or 4 aryl groups in one molecule from the viewpoint of storage stability. Further, these aryl borate compounds can be used alone or as a mixture of two or more.

Examples of sulfur-containing reductive inorganic compound include sulfites, bisulfites, pyrosulfites, thiosulfates, thionates and dithionite. Specific examples include sodium sulfite, potassium sulfite, calcium sulfite, ammonium sulfite, sodium bisulfite, potassium bisulfite, 3-mercaptopropyl trimethoxysilane, 2-mercaptobenzoxazole, decanethiol, thiobenzoic acid and the like.

Examples of nitrogen-containing reductive inorganic compound include nitrites, and specific examples include sodium nitrite, potassium nitrite, calcium nitrite, ammonium nitrite and the like.

Specific examples of barbituric acid derivative include salts (alkali metals or alkaline earth metals are preferred) of barbituric acid, 1,3-dimethyl barbituric acid, 1,3-diphenyl barbituric acid, 1,5-dimethyl barbituric acid, 5-butyl barbituric acid, 5-ethyl barbituric acid, 5-isopropyl barbituric acid, 5-cyclohexyl barbituric acid, 1,3,5-trimethyl barbituric acid, 1,3-dimethyl-5-ethyl barbituric acid, 1,3-dimethyl-n-butyl barbituric acid, 1,3-dimethyl-5-isobutyl barbituric acid, 1,3-dimethyl barbituric acid, 1,3-dimethyl-5-cyclopentyl barbituric acid, 1,3-dimethyl-5-cyclohexyl barbituric acid, 1,3-dimethyl-5-phenyl barbituric acid, 1-cyclohexyl-1-ethyl barbituric acid, 1-benzyl-5-phenyl barbituric acid, 5-methyl barbituric acid, 5-propyl barbituric acid, 1,5-diethyl barbituric acid, 1-ethyl-5-methyl barbituric acid, 1-ethyl-5-isobutyl barbituric acid, 1,3-diethyl-5-butyl barbituric acid, 1-cyclohexyl-5-methyl barbituric acid, 1-cyclohexyl-5-ethyl barbituric acid, 1-cyclohexyl-5-octyl barbituric acid, 1-cyclohexyl-5-hexyl barbituric acid, 5-butyl-1-cyclohexyl barbituric acid, 1-benzyl-5-phenyl barbituric acid and thiobarbituric acids. Specifically, the salts of these barbituric acids include sodium 5-butyl barbiturate, sodium 1,3,5-trimethyl barbiturate, sodium 1-cyclohexyl-5-ethyl barbiturate and the like.

Specific examples of the triazine compound include 2,4,6-tris(trichloro methyl)-s-triazine, 2,4,6-tris(tribromo methyl)-s-triazine, 2-methyl-4,6-bis(trichloro methyl)-s-triazine, 2-methyl-4,6-bis(tribromo methyl)-s-triazine, 2-phenyl-4,6-bis(trichloro methyl)-s-triazine, 2-(p-methoxy phenyl)-4,6-bis(trichloro methyl)-s-triazine, 2-(p-methyl thiophenyl)-4,6-bis(trichloro methyl)-s-triazine, 2-(p-chloro phenyl)-4,6-bis(trichloro methyl)-s-triazine, 2-(2,4-dichloro phenyl)-4,6-bis(trichloro methyl)-s-triazine, 2-(p-bromo phenyl)-4,6-bis(trichloro methyl)-s-triazine, 2-(p-tolyl)-4,6-bis(trichloro methyl)-s-triazine, 2-n-propyl-4,6-bis(trichloro methyl)-s-triazine, 2-($\alpha$,$\alpha$,$\beta$-trichloro ethyl)-4,6-bis(trichloro methyl)-s-triazine, 2-styryl-4,6-bis(trichloro methyl)-s-triazine, 2-[2-(p-methoxy phenyl) ethenyl]-4,6-bis(trichloro methyl)-s-triazine, 2-[2-(o-methoxy phenyl) ethenyl]-4,6-bis(trichloro methyl)-s-triazine, 2-[2-(p-butoxy phenyl) ethenyl]-4,6-bis(trichloro methyl)-s-triazine, 2-[2-(3,4-dimethoxy phenyl) ethenyl]-4,6-bis (trichloro methyl)-s-triazine, 2-[2-(3,4,5-trimethoxy phenyl) ethenyl]-4,6-bis(trichloro methyl)-s-triazine, 2-(1-naphthyl)-4,6-bis(trichloro methyl)-s-triazine, 2-(4-biphenylyl)-4,6-bis(trichloro methyl)-s-triazine, 2-[2-{N,N-bis(2-hydroxy ethyl) amino} ethoxy]-4,6-bis(trichloro methyl)-s-triazine, 2-[2-{N-hydroxy ethyl-N-ethylamino} ethoxy]-4,6-bis(trichloro methyl)-s-triazine, 2-[2-{N-hydroxy ethyl-N-methylamino} ethoxy]-4,6-bis(trichloro methyl)-s-triazine, 2-[2-{N, N-dially amino} ethoxy]-4,6-bis(trichloro methyl)-s-triazine and the like.

Specific examples of the halogen compound include dilauryl dimethyl ammonium chloride, lauryl dimethyl benzyl ammonium chloride, benzyl trimethyl ammonium chloride, tetramethyl ammonium chloride, benzyl dimethyl acetyl ammonium chloride, dilauryl dimethyl ammonium bromide and the like.

The dental adhesive composition of the present invention may be compounded with (F) filler suitable for the application without any limitation as long as it is a known filler, it is preferable that a filler such as an inorganic filler, an organic filler and an organic-inorganic composite filler is compounded. When the dental adhesive composition consists of first paste and second paste, the (F) fillers compounded in the first paste and the second paste may be the same or different.

As the inorganic filler of the (F) filler, the chemical composition is not particularly limited, but specific examples include silicon dioxide, alumina, silica-titania, silica-titania-barium oxide, silica-zirconia, silica-alumina, lanthanum glass, borosilicate glass, soda glass, barium glass, strontium glass, glass ceramic, aluminosilicate glass, barium boroaluminosilicate glass, strontium boroaluminosilicate glass, fluoroaluminosilicate glass, calcium fluoroaluminosilicate glass, strontium fluoroaluminosilicate glass, barium fluoroaluminosilicate glass, strontium calcium fluoroaluminosilicate glass and the like. Particularly, barium fluoroaluminosilicate glass, strontium fluoroaluminosilicate glass, fluoroaluminosilicate glass and the like, which are used in dental glass ionomer cement, resin reinforced glass ionomer cement and resin cement and the like, can also be suitably used. The fluoroaluminosilicate glass as used herein has a basic structure of silicon oxide and aluminum oxide and contains an alkali metal for introducing non-crosslinked oxygen. The fluoroaluminosilicate glass further has an alkaline earth metal including strontium and fluorine as modified/coordinated ions. The fluoroaluminosilicate glass may be also a composition in which a lanthanoid series element is incorporated into the skeleton in order to impart further radiopacity. This lanthanoid series element also participates in the composition as a modified/coordinated ion.

As the organic filler of the (F) filler, specific examples include polymers such as polymethyl methacrylate, polyethyl methacrylate, methyl methacrylate-ethyl methacrylate copolymer, ethyl methacrylate-butyl methacrylate copolymer, methyl methacrylate-trimethylolpropane methacrylate copolymer, polyvinylchloride, polystyrene, chlorinated polyethylene, nylon, polysulfone, polyethersulfone and polycarbonate.

As the organic-inorganic composite filler of the (F) filler, specific examples include a pulverized composite of the above-mentioned inorganic oxide (inorganic filler) and polymer (organic filler).

These may be used alone or as a mixture of two or more thereof. The particle shape of the filler is not particularly limited, and may be a pulverized particles obtained by usual pulverization or a spherical particles.

The composition ratio of the filler (F) in the dental composition in the present invention is not particularly limited, but is preferably 25 to 75 parts by mass, more preferably 40 to 70 parts by mass, with respect to 100 parts by mass of the total amount of the dental adhesive composition when the dental adhesive composition of the present invention is a composition consisting of a matrix containing (A) silane coupling material containing (A1) silane coupling material represented by structural formula of [Chemical formula 1], (B) polymerizable monomer having an acidic group, (C) polymerizable monomer having no acidic group and (D) polymerization initiator. When the filler is not contained, there is a concern that the storage stability and the adhesive strength may be reduced. When the content of the filler is less than 25 parts by mass, there is a concern that the durable adhesive strength may be lowered, and when the content of the filler exceeds 75 parts by mass, there is a concern that the operability may be deteriorated. In this case, the content of the matrix is preferably 25 to 75 parts by mass with respect to 100 parts by mass of the total amount of the dental adhesive composition. Further, the average particle diameter of the filler (F) is preferably 0.001 to 100 μm, more preferably 0.001 to 10 μm. When the filler in the composition consists of fine particles having the average particle diameter of 10 μm or less, the paste spreads well in luting. Further, when the glass fiber reinforced resin is bonded to the dental resin for cutting and machining, the composition easily enters between the glass fibers and therefore good adhesive strength can be expected.

When the dental adhesive composition of the present invention is a two-paste type dental adhesive composition containing (D1) chemical polymerization initiator and (E) polymerization accelerator and containing 15 to 80 parts by mass of (C1) polymerizable monomer having no acidic group and having one or more hydroxyl groups with respect to 100 parts by mass of the first matrix and the second matrix, the compounding amount of the (F) filler is preferably 25 to 75 parts by mass, more preferably 40 to 70 parts by mass with respect to 100 parts by mass of the total amount of the dental composition. When the (F) filler is not contained, there is a concern that the storage stability and the adhesive strength may be reduced. When the content of the (F) filler is less than 25 parts by mass, there is a concern that the durable adhesive strength may be lowered, and when the content of the (F) filler exceeds 75 parts by mass, there is a concern that the operability may be deteriorated. Further, the average particle diameter of the (F) filler is preferably 0.001 to 100 μm, more preferably 0.001 to 10 μm. When the (F) filler in the composition consists of fine particles having the average particle diameter of 10 μm or less, the paste spreads well in luting. Further, when the glass fiber reinforced resin is bonded to the dental resin for cutting and machining, the composition easily enters between the glass fibers and therefore good adhesive strength can be expected.

It is preferable that, particularly in the case of containing silica, the filler is surface treated so as not to react with the (A) silane coupling material containing the (A1) silane coupling material represented by structural formula of [Chemical formula 1] which is compounded in for the purpose of adhering to lithium disilicate compounded in the dental composition. When the dental adhesive composition of the present invention is a two-paste type dental adhesive composition containing (D1) chemical polymerization initiator and (E) polymerization accelerator and containing 15 to 80 parts by mass of (C1) polymerizable monomer having no acidic group and having one or more hydroxyl groups with respect to 100 parts by mass of the first matrix and the second matrix, it is preferable that the (C) filler compounded in the first paste is surface-treated with a surface treatment agent. It enables long-term storage because of such as suppressing the reaction with the silane coupling material. Examples of the surface treatment method include a method of surface treatment with a surfactant, an inorganic oxide, a silane coupling material or a polymer such as a polymer compound including an organopolysiloxane, PMMA and polyacrylic acid. From the viewpoint of affinity with the polymerizable monomer, it is preferable that the surface is treated with a silane coupling material. Specific examples include 3-(meth) acryloxypropyl trimethoxysilane and 8-(meth) acryloxyoctyl trimethoxysilane. Further, the silanol group on the surface of the filler may be capped after the surface treatment with these silane coupling materials. Examples of the silane coupling material for capping include chlorotrimethylsilane and methoxytrimethylsilane. The above-described silane coupling material enhances the affinity with the polymerizable monomer, and contributes to high filling of the filler and improvement of the mechanical strength by the polymerizable monomer. The latter silane coupling material for capping reacts with silanol groups on the surface of the filler that is not completely reacted by the former silane coupling material. And the reaction of the silane coupling material compounded for the purpose of adhering to the lithium silicate compounded in the dental adhesive composition the filler in storing for a long period of time is prevented. Therefore long-term storage stability can be expected. Since the filler has few points of reaction with the (A) silane coupling material during long-term storage, it is preferable that the content of $SiO_2$ is small, specifically 90 w/w% or less, more preferably 50 w/w% or less. In the case of using a filler having a high content of $SiO_2$, a method of capping with a low molecular weight silane coupling material such as chlorotrimethylsilane and methoxytrimethylsilane after surface treatment with a silane coupling material, and coating with polyorganosiloxane are preferable for improving storage stability. When the silane coupling material and the filler are compounded without such surface treatment, not only the adhesive strength to the dental resin for cutting and machining decreases after long-term storage, but also the affinity between the filler and the polymerizable monomer is significantly changed and the fluidity of the paste is changed and therefore the operability is changed. Thus it is not preferable.

The content of the surface-treated filler is preferably 90 parts by mass or more, preferably 95 parts by mass or more with respect to 100 parts by mass of the filler.

Specific examples of the (G) water compounded in the dental adhesive composition of the present invention include deionized water and distilled water. The compounding amount of the (G) water is preferably 1 to 50 parts by mass, more preferably 10 to 50 parts by mass, further preferably 25 to 35 parts by mass, with respect to 100 parts by mass of the total amount of the dental adhesive composition.

As the (H) volatile organic solvent of the present invention, a volatile organic solvent usually having a boiling point of 150 °C or less under normal pressure and a solubility of 5% by mass or more with respect to water at 25 °C, more preferably 30% by mass or more, and, most preferably, an organic solvent which can dissolved in water at an arbitrary ratio is used. Among these, a water-soluble volatile organic solvent having a boiling point of 100 °C or less under normal pressure is preferable, and specific examples include ethanol, methanol, 1-propanol, isopropyl alcohol, acetone, methylethyl ketone, 1,2-dimethoxyethane, 1,2-diethoxyethane and tetrahydrofuran. Further, among the above-mentioned volatile organic solvents, ethanol, isopropyl alcohol, acetone and methylethyl ketone are more preferable.

The (H) volatile organic solvent may be used alone or in combination of two or more. The compounding amount of the (H) volatile organic solvent is preferably 5 to 90 parts by mass, more preferably 10 to 90 parts by mass, most preferably 30 to 60 parts by mass, with respect to 100 parts by mass of total amount of the dental adhesive composition.

Moreover, the dental adhesive composition of the present invention may be compounded with a well-known additives in the range in which as long as the properties does not decrease. Specific examples of such additives include polymerization inhibitors, antioxidants, pigments, dyes, ultraviolet absorbers, organic solvents, thickeners and the like.

Examples of suitable embodiment in the case that the dental adhesive composition of the present invention is a composition consisting of a matrix containing (A) silane coupling material containing (A1) silane coupling material represented by structural formula of [Chemical formula 1], (B) polymerizable monomer having an acidic group, (C) polymerizable monomer having no acidic group and (D) polymerization initiator, wherein the (A1) silane coupling material represented by structural formula of [Chemical formula 1] has an acryloyl group include following one-paste type dental adhesive composition and two-paste type dental adhesive composition.

< One-paste type dental adhesive composition >

When the dental adhesive composition of the present invention is one-paste type dental adhesive composition, it can be used as a dental composite resin for filling and a dental cement. The one-paste type dental adhesive composition of the present invention is preferable because it has few technical errors and the risk of mixing of air bubbles is low, and it is particularly preferable to use as a dental self-adhesive composite resin among the dental composite resin. In the case of one-paste type dental adhesive composition, it contains (A) silane coupling material containing (A1) silane

coupling material represented by structural formula of [Chemical formula 1], (B) polymerizable monomer having an acidic group, (C) polymerizable monomer having no acidic group and (D) polymerization initiator. More preferably, it contains (F) filler, (D2) photopolymerization initiator and (E) polymerization accelerator. Particularly, when the (A1) silane coupling material represented by structural formula of [Chemical formula 1] having an acryloyl group is used, good adhesive strength to the glass ceramics containing lithium disilicate is expected by compounding a smaller amount as compared with the case where a conventional silane coupling material having a methacryloyl group is used, and both adhesive property and storage stability can be expected by compounding based on the silane coupling material compounding amount index.

< Two-paste type dental adhesive composition >

When the dental adhesive composition the present invention is two-paste type dental adhesive composition, it can be used as a dental composite resin for filling and a dental cement. The two packs including first paste and second paste are kneaded immediately before use. The mixing ratio of the first paste and the second paste is, in terms of volume ratio, preferably 1:0.8 to 1.2, and more preferably 1:1. The mass ratio is preferably 1: 0.8 to 1.2, and more preferably 1:1. The two-paste type adhesive composition of the present invention is preferably used as a dental self-adhesive resin cement among the dental resin cement. In the case of two-paste type dental adhesive composition, it contains (A) silane coupling material containing (A1) silane coupling material represented by structural formula of [Chemical formula 1], (B) polymerizable monomer having an acidic group, (C) polymerizable monomer having no acidic group and (D) polymerization initiator. More preferably, it contains (F) filler, (D1) chemical polymerization initiator and (E) polymerization accelerator. Particularly, when the (A1) silane coupling material represented by structural formula of [Chemical formula 1] having an acryloyl group is used, good adhesive strength to the glass ceramics containing lithium disilicate is expected by compounding a smaller amount as compared with the case where a conventional silane coupling material having a methacryloyl group is used, and both adhesive property and storage stability can be expected by compounding based on the silane coupling material compounding amount index.

In this case, the dental adhesive composition of the present invention may consist of first paste and second paste. In this case, for example, the first paste may contain first matrix and (F) filler, and the first matrix may contain (A) silane coupling material containing (A1) silane coupling material represented by structural formula of [Chemical formula 1], and (C) polymerizable monomer having no acidic group. The second paste may contain second matrix and (F) filler, and the second matrix may contain (B) polymerizable monomer having an acidic group and (C) polymerizable monomer having no acidic group. The first matrix and the second matrix contain at least one of (D) polymerization initiator and (E) polymerization accelerator. When the first matrix contains one or more (D1) chemical polymerization initiator, the second matrix contains one or more (E) polymerization accelerator. When the first matrix contains one or more (E) polymerization accelerator, the second matrix contains one or more (D1) chemical polymerization initiator. In this case, the dental adhesive composition substantially may not contain (G) water.

The dental adhesive composition of the present invention may contain a matrix containing (A) silane coupling material containing (A1) silane coupling material represented by structural formula of [Chemical formula 1], (B) polymerizable monomer having an acidic group, (C) polymerizable monomer having no acidic group and (D) polymerization initiator, and (F) filler.

Specifically, a compounding amount of the matrix contained in the dental adhesive composition may be 25 to 75 parts by mass with respect to 100 parts by mass of the dental adhesive composition, and a compounding amount of the (F) filler contained in the dental adhesive composition may be 25 to 75 parts by mass with respect to 100 parts by mass of the dental adhesive composition.

When the dental adhesive composition of the present invention is a two-paste type dental adhesive composition containing (D1) chemical polymerization initiator and (E) polymerization accelerator and containing 15 to 80 parts by mass of (C1) polymerizable monomer having no acidic group and having one or more hydroxyl groups with respect to 100 parts by mass of the first matrix and the second matrix, the mixing ratio of the first paste and the second paste is, in terms of volume ratio, preferably 1:0.8 to 1.2, and more preferably 1:1. The mass ratio is preferably 1: 0.8 to 1.2, and more preferably 1: 1.

In this case, the compounding amount of the first matrix of the first paste may be 25 to 75 parts by mass with respect to 100 parts by mass of the first paste, and the compounding amount of the (F) filler of the first paste may be 25 to 75 parts by mass with respect to 100 parts by mass of the first paste, and the compounding amount of the (C) polymerizable monomer having no acidic group of the first matrix may be 65 to 98 parts by mass with respect to 100 parts by mass of the first matrix.

In this case, the compounding amount of the second matrix of the second paste may be 25 to 75 parts by mass with respect to 100 parts by mass of the second paste, and the compounding amount of the (F) filler of the second paste may be 25 to 75 parts by mass with respect to 100 parts by mass of the second paste.

In the dental adhesive composition of the present invention, regardless of the one paste type or two pastes

type, the compounding amount of the matrix is preferably 25 to 75 parts by mass with respect to 100 parts by mass of the dental adhesive composition and the compounding amount of the (F) filler contained in the dental adhesive composition is preferably 25 to 75 parts by mass with respect to 100 parts by mass of the dental adhesive composition. With such a compounding amount, it can be expected that the dental adhesive composition has appropriate fluidity and good durability derived from and sufficient mechanical strength. When the compounding amount of the matrix contained in the dental adhesive composition is less than 25 parts by mass with respect to 100 parts by mass of the dental adhesive composition, the proportion of the filler is large, therefore there is a case where it does not have an appropriate fluidity for using as a dental adhesive composition. When the compounding amount of the matrix contained in the dental adhesive composition exceeds 75 parts by mass, there is a case where a sufficient improvement in strength is not exhibited. Depending on the type of the filler, high fluidity is exhibited even when the filling amount of the filler in the composition is large, or high strength is exhibited even when the filling amount is small.

The two paste type dental adhesive composition of the present invention can be used for adhesion to a dental restorative material for cutting and machining. The dental restorative material for cutting and machining may be, for example, a glass fiber reinforced material containing a glass fiber and an epoxy resin. In this case, the dental restorative material for cutting and machining may be a material in which the orientation direction of the glass fibers is not uniform and the glass fibers are randomly compounded. Alternatively, the dental restorative material for cutting and machining may be a material which is a laminated body in which the glass fibers are woven in a cross shape, and the woven surface in a cross shape and a surface in which the woven surface in a cross shape is rotated 90° in the vertical direction are laminated surfaces of the glass fiber.

The dental restorative material for cutting and machining may have two or more adherend surfaces having different structures.

Examples of suitable embodiment in the case that the dental adhesive composition of the present invention is a composition containing (A) silane coupling material containing (A1) silane coupling material represented by structural formula of [Chemical formula 1], (B) polymerizable monomer having an acidic group, (C) polymerizable monomer having no acidic group, (H) volatile organic solvent and (G) water, and one or both of (D) polymerization initiator and (E) polymerization accelerator include a dental adhesive material, a tooth substance primer, a metal primer, a ceramics primer and the like. Further, in each application, the dental adhesive composition of the present invention may be used as two-pack type in which the components of the dental adhesive composition of the present invention are divided into two packs. Specific embodiments in the case that the dental adhesive composition is applied are shown below.

< Dental adhesive material >

When the present invention is used as a dental adhesive, examples of specific embodiment include two-pack mixing one-step type in which two packs including first pack and second pack are mixing immediately before use, two-pack two-step type in which second pack is applied after first pack is applied, and one-pack one-step type in which one pack can be used as it is. Among these, the one-pack one-step type is more preferable because the work at the time of use is simple and technical errors are unlikely to occur. In the case of the one-pack one-step type, the composition contains (A) silane coupling material containing (A1) silane coupling material represented by structural formula of [Chemical formula 1], (B) polymerizable monomer having an acidic group, (C) polymerizable monomer having no acidic group, (D) polymerization initiator and/or (E) polymerization accelerator, (H) volatile organic solvent, and (G) water. When the one-pack one-step type dental adhesive composition is prepared according to the silane coupling material compounding amount index in composition of the present invention, good storage stability can be expected. Further, by using a silane coupling material having an acryloyl group as the (A1) silane coupling material represented by structural formula of [Chemical formula 1], good adhesive strength to glass ceramics such as glass ceramics containing lithium disilicate and composite resin containing inorganic component can be expected as compared with the case where a conventional silane coupling material having a methacryloyl group is used.

< Tooth substance primer >

The tooth substance primer is used for increasing the adhesive strength in luting cement and a prosthetic device by modifying the surface of the tooth substance in the case of adhering to the tooth substance. When the present invention is used as a tooth substance primer, examples of specific embodiment include two-pack mixing one-step type in which two packs including first pack and second pack are mixing immediately before use, two-pack two-step type in which second pack is applied after first pack is applied, and one-pack one-step type in which one pack can be used as it is. Among these, the one-pack one-step type is more preferable because the work at the time of use is simple and technical errors are unlikely to occur. In the case of the one-pack one-step type, the composition contains (A) silane coupling material, (B) polymerizable monomer having an acidic group, (C) polymerizable monomer having no acidic group, (D) polymerization initiator and/or (E) polymerization accelerator, (H) volatile organic solvent, and (G) water. It is

more preferable to contain (D) polymerization initiator and/or (E) polymerization accelerator which accelerates interfacial polymerization of the material applied on applied tooth substance primer (for example, resin cement).

< Metal primer>

The metal primer is used for increasing the adhesive strength in luting with cement between an abutment tooth and a prosthesis device consisting of metal and non-precious metal by modifying the surface of metal and non-precious metal in the case of adhering to the metal and non-precious metal.

When the present invention is used as a metal primer, examples of specific embodiment include two-pack mixing one-step type in which two packs including first pack and second pack are mixing immediately before use, two-pack two-step type in which second pack is applied after first pack is applied, and one-pack one-step type in which one pack can be used as it is. Among these, the one-pack one-step type is more preferable because the work at the time of use is simple and technical errors are unlikely to occur. In the case of the one-pack one-step type, the composition contains (A) silane coupling material containing (A1) silane coupling material represented by structural formula of [Chemical formula 1], (B) polymerizable monomer having an acidic group, (C) polymerizable monomer having no acidic group, (D) polymerization initiator and/or (E) polymerization accelerator, (H) volatile organic solvent, and (G) water.

< Ceramics primer >

The ceramics primer is used for increasing the adhesive strength in luting with cement between an abutment tooth and a prosthesis device consisting of ceramics and glass ceramics by modifying the surface of ceramics and glass ceramics in the case of adhering to the ceramics such as zirconia and alumina, and glass ceramics made of feldspar and lithium disilicate. When the present invention is used as a ceramics primer, examples of specific embodiment include two-pack mixing one-step type in which two packs including first pack and second pack are mixing immediately before use, and one-pack one- step type in which one pack can be used as it is. Among these, the one-pack one-step type is more preferable because the work at the time of use is simple and technical errors are unlikely to occur. In the case of the one-pack one-step type, the composition contains (A) silane coupling material containing (A1) silane coupling material represented by structural formula of [Chemical formula 1], (B) polymerizable monomer having an acidic group, (C) polymerizable monomer having no acidic group, (D) polymerization initiator and/or (E) polymerization accelerator, (H) volatile organic solvent, and (G) water. When the one-pack one-step type dental adhesive composition is prepared according to the silane coupling material compounding amount index in composition of the present invention, good storage stability can be expected. Further, by using a silane coupling material having an acryloyl group as the (A1) silane coupling material represented by structural formula of [Chemical formula 1], good adhesive strength to glass ceramics such as glass ceramics containing lithium disilicate and composite resin containing inorganic component can be expected as compared with the case where a conventional silane coupling material having a methacryloyl group is used.

The method for preparing the dental adhesive composition of the present invention is not particularly limited, and it can be prepared by compounding each component. Preferably, a composition is prepared by preparing uniform solution by mixing (B) polymerizable monomer having an acidic group, (C) polymerizable monomer having no acidic group and (H) volatile organic solvent, and thereafter adding (A) silane coupling material containing (A1) silane coupling material represented by structural formula of [Chemical formula 1], (D) polymerization initiator and/or (E) polymerization accelerator, and (G) water. When (B) polymerizable monomer having an acidic group contacts with (A) silane coupling material containing (A1) silane coupling material represented by structural formula of [Chemical formula 1] and (D) polymerization initiator during weighing, there is a possibility of inducing decomposition of the (A) silane coupling material containing (A1) silane coupling material represented by structural formula of [Chemical formula 1] and the (D) polymerization initiator, and when (G) water is mixed later, it is possible to reduce preparing time.

[Examples]

Hereinafter, the present invention will be described in detail with reference to Examples and Comparative Examples. However, the present invention is not limited to these Examples. The abbreviations used below are as follows. The ratio of the component in each Example and Comparative Example is shown in parts by mass in the table.

[(A) silane coupling material]

OTES: n-octyl triethoxysilane
MTTSP: 3-[tris (trimethylsilyloxy) silyl] propyl methacrylate

AAPTMS: 3-acrylamidepropyl trimethoxysilane
MAPTMS: 3-methacrylamidepropyl triethoxysilane

< (A1) silane coupling material represented by structural formula of [Chemical formula 1]>

(Silane coupling material having acryloyl group)

APTMS: acryloylpropyl trimethoxysilane
APMES: acryloylpropylmethyl diethoxysilane
APMMS: acryloylpropylmethyl dimethoxysilane
APDMS: acryloylpropyl dimethylmethoxysilane

C11A: 4,4-diethoxy-17-oxo-3,16-dioxa-18-aza-4-silaicosane-20-yl (meth) acrylate

[Chemical formula 2]

C11DA: 2-methyl-2-((((11-(triethoxysilyl) undecyl) oxy) carbonyl) amino) propan-1,3-diyl diacrylate

[Chemical formula 3]

(Silane coupling material having a methacrylic group (not having acryloyl group))

MPTMS: methacryloylpropyl trimethoxysilane
MOTMS: methacryloyloctyl trimethoxysilane
MPTBS: methacryloylpropyl tributosixilane

C11EG: 4,4-diethoxy-17-oxo-3,16,21-trioxa-18-aza-4-silatricosane-23-yl (meth) acrylate

[Chemical formula 4]

[(B) polymerizable monomer having an acidic group]

MDP: 10-methacryloyloxydecyl dihydrogen phosphate
MHPA: (6-methacryloyloxy) hexylphosphonoacetate
META: 4-[(2-methacryloyl oxyethoxy) carbonyl] phthalic anhydride
MET: 4-methacryloxyethyl trimellitic acid

[(C) polymerizable monomer having no acidic group]

< (C11) low viscosity polymerizable monomer having no acidic group and one or more hydroxyl groups >

GDMA glycerin dimethacrylate (35 mPa ·s) (having methacrylic group and/or methacrylamide group) (the number of polymerizable groups: 2)
HEMA: 2-hydroxyethyl methacrylate (8 mPa ·s) (having methacrylic group and/or methacrylamide group) (the number of polymerizable groups: 1)
HPPA: 2-hydroxy-3-phenoxypropyl acrylate (200 mPa ·s) (not having methacrylic group and/or methacrylamide group) (the number of polymerizable groups: 1)

< (C1) polymerizable monomer havingo acidic group and having one or more hydroxyl group s>

BisGMA: 2,2-bis [4-(3-(meth) acryloyloxy)-2-hydroxy propoxyphenyl] propane: Viscosity cannot be measured at 25 °C (the number of polymerizable groups: 2)
PENTA: pentaerythritol triacrylate: 800 mPa ·s (the number of polymerizable groups: 3)

< Polymerizable monomer having no acidic group and no hydroxyl group >

UDMA: 2,2,4-trimethyl hexamethylene bis (2-carbamoyloxy ethyl) dimethacrylate (the number of polymerizable groups: 2)
3G: triethylene glycol dimethacrylate (the number of polymerizable groups: 2)
NPG: neopentyl glycol dimethacrylate (the number of polymerizable groups: 2)
D2.6E: 2,2-bis (4-methacryloyloxy polyethoxyphenyl) propane in which the average addition mole number of ethoxy groups is 2.6 (the number of polymerizable groups: 2) A3.0E: EO adduct diacrylate of bisphenol A having in which the average addition mole number of ethoxy groups is 3 (not having methacryloyl group and methacrylamide group)

(the number of polymerizable groups: 2)
DMCDA: dimethylol-tricyclodecane diacrylate (not having methacryloyl group and methacrylamide group) (the number of polymerizable groups: 2)
TMPTA: trimethylolpropane triacrylate (not having methacryloyl group and methacrylamide group) (the number of polymerizable groups: 3)
EBAA: N,N'-ethylene bisacrylamide (not having methacryloyl group and methacrylamide group) (the number of polymerizable groups: 2)
MBMA: N,N'-methylene bismethacrylamide (having methacrylamide groups) (the number of polymerizable groups: 2)

(Viscosity measurement method)

Polymerizable monomer in amount of 250 g was collected in 260 mL of a wide mouthed glass bottle, and allowed to stand for 24 hours under the condition of 25 °C $\pm$ 3 °C in a state where it was shielded from light with aluminum foil. Then, the viscosity of the polymerizable monomer collected in the wide-mouthed glass bottle was measured using B-type viscometer under the conditions of 25 °C $\pm$ 3 °C.

[(D) polymerization initiator]

< (D1) chemical polymerization initiator >

CHP: cumene hydroperoxide
TMBH: 1, 1, 3, 3-tetramethyl butylhydroperoxide
BPO: benzoyl peroxide

< (D2) photopolymerization initiator >

CQ: camphorquinone

[(E) polymerization accelerator]

DMBE: 4-(dimethylamino) ethyl benzoate
DEPT: N,N-di (2-hydroxyethyl)-p-toluidine
PTSA: sodium p-toluenesulfinate
BTU: N-benzoyl thiourea
PTU: N-pyridyl thiourea
COA: acetylacetone copper
VOA: vanadyl acetylacetonate
GLC: copper gluconate

[(F) Filler]

The following raw material glasses were used as the Fillers A to C.

Filler A: silica filler
($SiO_2$: about 99.5% by mass, the rest are other inorganic components, average particle diameter: 0.8 $\mu$m)

Filler B: silica zirconia filler
($SiO_2$: about 80% by mass, $ZrO_2$: about 20% by mass, the rest are other inorganic components, average particle diameter: 1.0 $\mu$m)

Filler C: fluoroaluminosilicate
($SiO_2$: about 45% by mass, $Al_2O_3$: about 22% by mass, SrO: about 20% by mass, F: about 8% by mass, $P_2O_5$: about 5% by mass, average particle diameter: 1.2 $\mu$m)

Filler D to I were prepared as follows.

Filler D

A silane coupling treatment solution prepared by stirring 100.0 g of water, 80.0 g of ethanol, 0.003 g of phosphoric acid, and 3.0 g of 3-methacryloyloxypropyl trimethoxysilane as a silane coupling material at room temperature for 2 hours was added to 100.0 g of the Filler A and stirred for 30 minutes. Thereafter, a heat treatment was performed at 100 °C for 15 hours and sieving was performed to obtain a filler D.

Filler E

A silane coupling treatment solution prepared by stirring 100.0 g of water, 80.0 g of ethanol, 0.003 g of phosphoric acid, and 3.0 g of 3-methacryloyloxypropyl trimethoxysilane as a silane coupling material at room temperature for 2 hours was added to 100.0 g of the Filler B and stirred for 30 minutes. Thereafter, a heat treatment was performed at 100 °C for 15 hours and sieving was performed to obtain a filler E.

Filler F

A silane coupling treatment solution prepared by stirring 100.0 g of water, 80.0 g of ethanol, 0.003 g of phosphoric acid, and 3.0 g of 3-methacryloyloxypropyl trimethoxysilane as a silane coupling material at room temperature for 2 hours was added to 100.0 g of the Filler C and stirred for 30 minutes. Thereafter, a heat treatment was performed at 100 °C for 15 hours and sieving was performed to obtain a filler F.

Filler G

Polyorganosiloxane "MKC silicate MS51" in amount of 10.8 g (manufactured by Mitsubishi Chemical Corporation) was added to 100.0 g of raw material glass A, and was stirred and mixed for about 90 minutes. Then, the obtained treated slurry was left in a hot air dryer at 50 °C for 40 hours and then heated to 150 °C and held for 6 hours. The obtained heat-treated product was placed in a Henschel mixer and crushed at 1800 rpm for 5 minutes. Filler G was obtained by sieving after crushing.

Filler H

Polyorganosiloxane "MKC silicate MS51" in amount of 10.8 g (manufactured by Mitsubishi Chemical Corporation) was added to 100.0 g of raw material glass B, and was stirred and mixed for about 90 minutes. Then, the obtained treated slurry was left in a hot air dryer at 50 °C for 40 hours and then heated to 150 °C and held for 6 hours. The obtained heat-treated product was placed in a Henschel mixer and crushed at 1800 rpm for 5 minutes. Filler H was obtained by sieving after crushing.

Filler I

A silane coupling treatment solution prepared by stirring 100.0 g of water, 80.0 g of ethanol, 0.003 g of phosphoric acid, and 10.0 g of 3-methacryloyloxypropyl trimethoxysilane as a silane coupling material at room temperature for 2 hours was added to 100.0 g of the raw material glass A and stirred for 30 minutes. Thereafter, a heat treatment was performed at 100 °C for 15 hours and sieving was performed. Then, a silane coupling treatment solution prepared by stirring 100.0 g of water, 80.0 g of ethanol, 0.003 g of phosphoric acid, and 3.0 g of 3-methacryloyloxypropyl trimethoxysilane as a silane coupling material at room temperature for 2 hours was added and stirred for 5 hours. Thereafter, a heat treatment was performed at 100 °C for 15 hours and sieving was performed to obtain a filler I.

[(G) Water]

D.W : distilled water

[(H) Volatile organic solvent]

Acetone: acetone

EtOH: Ethanol

[(I) polymerizable monomer having one or more sulfur atoms]

MDDT: 10-methacryloxy decyl-6,8-dithiooctanate (polymerizable monomer having one or more sulfur atoms)

[Others]

BHT: 2,6-di-t-butyl-4-methylphenol

[Dental adhesive composition consisting of matrix containing (A) silane coupling material, (B) polymerizable monomer having an acidic group, (C) polymerizable monomer having no acidic group and (D) polymerization initiator]

(Preparing method of paste)

The polymerizable monomers shown in Examples and Comparative Examples were put into a light-shielding plastic container and mixed by using a mix rotor VMRC-5 under the condition of 100 rpm for 48 hours. Thereafter, the polymerization initiator was added and further mixed by using a mix rotor VMRC-5 under the condition of 100 rpm for 48 hours to prepare a resin liquid. Then, the resin liquid and the filler were put into a kneading container and kneaded at 1000 rpm for 20 minutes using a rotation and revolution mixer ARV-300 to obtain a dental adhesive composition or a first paste and a second paste.

The test method of each characteristic evaluated in the example and the comparative example is as follows. For the two-paste type dental adhesive composition, the first paste and the second paste were weighed with an electronic analytical scale in a dark room at 23 ± 2 °C, and mixing them under the condition of equal mass. In the case where the volume ratio was 1.0: 0.8 to 1.2, and in the preferably case where the volume ratio was 1.0: 0.9 to 1.1, the same result was obtained in the test by using a mixing chip manufactured by Mixpack Co., Ltd.

(Initial preparation product and Acceleration test product)

The prepared paste was filled in each container, and was used as initial preparation product in the case where the storage temperature was 1 to 30 °C and a storage period was less than 3 months. Further, the prepared paste was filled in each container and stored in a constant temperature incubator (manufactured by Yamato Scientific Co., Ltd.) at 40 °C for 3 months, and was used as acceleration test product. The one-paste type dental adhesive composition was filled in a polypropylene 3 mL syringe. For the two-paste type dental adhesive composition, the first paste and the second paste were filled in one syringe which is a double syringe manufactured by Mixpack Co., Ltd.

(Adhesive strength of one-paste type dental adhesive composition)

The adhesive strength of one-paste type dental adhesive composition was measured by the following method.

< Adhesive strength to tooth substance >

A test specimen of an epoxy resin embedded bovine anterior tooth was polished with water-resistant abrasive paper #600 to carve out a dentin plane. Thereafter, a double sided tape with a hole having a diameter of 4 mm was affixed to the tooth substance surface to prescribe an adhesion area. A plastic mold having an inner diameter of 4 mm and a height of 1 mm was fixed on the double-sided tape with a hole, each dental adhesive composition of Examples and Comparative Examples were filled into the inside. Thereafter, light irradiation was performed for 10 seconds with the dental polymerization LED light curing unit (PEN Bright, manufactured by SHOFU INC.). The prepared adhesive test piece after immersing in water at 37 °C for 24 hours, immersing alternately in cold water phase at 4 °C for 60 seconds and in high temperature phase at 60 °C for 60 seconds was repeated 5000 times by using a thermal shock tester (manufactured by THOMAS KAGAKU Co.,Ltd). The specimen was taken out and a universal tester (manufactured by Instron) was used to measure the shear adhesive strength at a crosshead speed of 1 mm/min. When the adhesive strength was 10 MPa or more, it was determined to have excellent adhesive strength. On the other hand, when the adhesive strength was less than 5 MPa, it was determined that the adhesive strength was low.

< Adhesive strength to glass ceramics containing lithium disilicate >

The glass ceramics containing lithium disilicate (VINTAGE PRIME PRESS, color tone E-1, manufactured by

SHOFU INC.) was fired under the conditions specified by the manufacturer to prepare an adherend (diameter: 15 mm, thickness: 3 mm). The surface of the adherend piece was polished with water-resistant abrasive paper # 600. Thereafter, the adherend surface of the adherend piece was sandblasted (0.2 MPa, 1 second) with alumina (50 μm), then was washed with water and dried. A double sided tape with a hole having a diameter of 4 mm was affixed to the adherend surface to prescribe an adhesion area. A plastic mold having an inner diameter of 4 mm and a height of 1 mm was fixed on the double-sided tape with a hole, each dental adhesive composition of Examples and Comparative Examples were filled into the inside. Thereafter, light irradiation was performed for 10 seconds with the dental polymerization LED light curing unit (PEN Bright, by SHOFU INC.). The prepared adhesive test piece after immersing in water at 37 °C for 24 hours, immersing alternately in cold water phase at 4 °C for 60 seconds and in high temperature phase at 60 °C for 60 seconds was repeated 5000 times by using a thermal shock tester (manufactured by THOMAS KAGAKU Co.,Ltd). The specimen was taken out and a universal tester (manufactured by Instron) was used to measure the shear adhesive strength at a crosshead speed of 1 mm/min. When the adhesive strength was 15 MPa or more, it was determined to have excellent adhesive strength. On the other hand, when the adhesive strength was less than 10 MPa, it was determined that the adhesive strength was low.

(Adhesive strength of two-paste type dental adhesive composition)

The adhesive strength of two-paste type dental adhesive composition was measured by the following method.

< Adhesive strength to tooth substance >

A test specimen of an epoxy resin embedded bovine anterior tooth was polished with water-resistant abrasive paper #600 to carve out a dentin plane. Thereafter, a double sided tape with a hole having a diameter of 4 mm was affixed to the tooth substance surface to prescribe an adhesion area. On the other hand, the adherend surface of the stainless rod (φ4.5 mm) was sandblasted (0.2 MPa, 1 second) with alumina (50 μm), then was washed with water and dried, and applied with a metal adhesive primer (METAL LINK, manufactured by SHOFU INC.). The first paste and the second paste which correspond the dental adhesive composition of the Example or Comparative example were sufficiently kneaded in equal mass and was applied to the adherend surface of the stainless rod in an appropriate amount, and the glass ceramics containing lithium disilicate and the stainless rod were bonded so as to fit in the frame of the double-sided tape with a hole. A load of 200 N was applied from the vertical direction of the stainless rod, and excess cement was wiped off with a cloth. Thereafter, Light irradiation was performed for 10 seconds with the dental polymerization LED light curing unit (PEN Bright, SHOFU INC.). After removing the load, the prepared adhesive test piece was immersed in water at 37 °C for 24 hours, immersing alternately in cold water phase at 4 °C for 60 seconds and in high temperature phase at 60 °C for 60 seconds was repeated 5000 times by using a thermal shock tester (manufactured by THOMAS KAGAKU Co.,Ltd). The specimen was taken out and a universal tester (manufactured by Instron) was used to measure the tensile adhesive strength at a crosshead speed of 1 mm/min. When the adhesive strength was 10 MPa or more, it was determined to have excellent adhesive strength. On the other hand, when the adhesive strength was less than 5 MPa, it was determined that the adhesive strength was low.

< Adhesive strength to glass ceramics containing lithium disilicate >

The glass ceramics containing lithium disilicate (VINTAGE PRIME PRESS, color tone E-1, manufactured by SHOFU INC.) was fired under the conditions specified by the manufacturer to prepare an adherend (diameter: 15 mm, thickness: 3 mm). The surface of the adherend piece was polished with water-resistant abrasive paper # 600. Thereafter, the adherend surface of the adherend piece was sandblasted (0.2 MPa, 1 second) with alumina (50 μm), then was washed with water and dried. A double sided tape with a hole having a diameter of 4 mm was affixed to the tooth substance surface to prescribe an adhesion area. On the other hand, the adherend surface of the stainless rod (φ4.5 mm) was sandblasted (0.2 MPa, 1 second) with alumina (50 μm), then was washed with water and dried, and applied with a metal adhesive primer (METAL LINK, SHOFU INC.). The first paste and the second paste which correspond the dental adhesive composition of the Example or Comparative example were sufficiently kneaded in equal mass and was applied to the adherend surface of the stainless rod in an appropriate amount, and the glass ceramics containing lithium disilicate and the stainless rod were bonded so as to fit in the frame of the double-sided tape with a hole. A load of 200 N was applied from the vertical direction of the stainless rod, and excess cement was wiped off with a cloth. Thereafter, light irradiation was performed for 10 seconds with the dental polymerization LED light curing unit (PEN Bright, manufactured by SHOFU INC.). After removing the load, the prepared adhesive test piece was immersed in water at 37 °C for 24 hours, immersing alternately in cold water phase at 4 °C for 60 seconds and in high temperature phase at 60 °C for 60 seconds was repeated 5000 times by using a thermal shock tester (manufactured by THOMAS KAGAKU Co.,Ltd). The specimen was taken out and a universal tester (manufactured by Instron) was used to measure the tensile adhesive

strength at a crosshead speed of 1 mm/min. When the adhesive strength was 15 MPa or more, it was determined to have excellent adhesive strength. On the other hand, when the adhesive strength was less than 10 MPa, it was determined that the adhesive strength was low.

(Discoloration resistance property)

In the case of one-paste type dental adhesive composition, a needle tip was attached, and in the case of two-paste type dental adhesive composition, the first paste and the second paste were sufficiently kneaded in equal mass, and the prepared paste was collected in a mold with a thickness of 1.0 mm and an inner diameter of 15 mm, with being careful not to allow air bubbles to enter. The both sides were pressed with cover glasses and light irradiation was performed on the both surface for 60 seconds with the dental polymerization LED light irradiator (PEN Bright, manufactured by SHOFU INC.) to prepare a disk-shaped cured product having a thickness of 1.0 mm. The cured product was polished with water-resistant abrasive paper #600, then with water-resistant abrasive paper #1200, and then with water-resistant abrasive paper #2000 to adjust the surface to be smooth. Subsequently, the cured product was immersed in a 10 mL plastic container containing 5 mL of distilled water at 37 °C for 1 day. The cured product was taken out, and after sufficiently wiping off the water, the color was measured using a spectrophotometer (CM-26d: manufactured by Konica Minolta, Inc.) under the conditions of SCE and a white background. Then, the cured product was immersed in a 10 mL plastic container containing 5 mL of 0.1% Rhodamine aqueous solution at 37 °C for 1 day, and the cured product was taken out and was washed twice with distilled water to sufficiently wipe off water from the cured product. Thereafter, the color was measured using a spectrophotometer (CM-26d: manufactured Konica Minolta, Inc.) under the conditions of SCE and a white background. The color difference $\Delta E$ was calculated from the results measured before and after immersion in 0.1% Rhodamine aqueous solution. Evaluation criteria were as follows.

A: $\Delta E$ was less than 30
B: $\Delta E$ was 30 to 45
C: $\Delta E$ was more than 45

When the evaluation was C, it was judged that the resistance property was inferior.

(Paste property)

In the case of one-paste type dental adhesive composition, a needle tip was attached, and in the case of two-paste type dental adhesive composition, the first paste and the second paste were sufficiently kneaded in equal mass, and 0.1 g of the prepared paste was allowed to stand on a slide glass and the position where the paste was left to stand was marked using a seal. Then, the slide glass was tilted at 90°, and after 20 seconds, the slide glass was returned to its original position, and the distance the paste moved was measured. Evaluation criteria for the increase/decrease in the moving distance of the paste between the paste immediately after preparation and the paste after storage at 40 °C for 3 months were as follows.

A: less than 10%
B: 10 to 40 %
C: more than 40%

When the evaluation was C, it was judged that the characteristic stability was inferior.

[Table 1]

| | (A1) Silane coupling material represented by structural formula of [Chemical formula 1] | | | | | | (B) Polymerizable monomer having an acidic group | | | (C) Polymerizable monomer having no acidic group | | | | | | | | | Photo polymerization initiator | Chemical polymerization initiator | Polymerization accelerator | | | Polymerization inhibitor |
|---|---|---|---|---|---|---|---|---|---|---|---|---|---|---|---|---|---|---|---|---|---|---|---|---|
| | | | | | | | | | | (C1) Having one or more hydroxyl groups | | | Having no hydroxy group | | | Having no hydroxy group and no methacryloyl group | | | | | | | | |
| | APTMS | APMES | APMMS | APDMS | C11A | C11DA | MDP | MHPA | META | BisGMA | GDMA | HEMA | UDMA | 3G | NPG | A3.0E | DMCDA | TMPTA | CQ | CHP | DMBE | PTU | VOA | BHT |
| Example A1 | 3 | | | | | | 5 | | 5 | 50 | | | 17 | | 20 | | | | 0.3 | | 0.3 | | | 0.1 |
| Example A2 | | 5 | | | | | 5 | | 5 | 50 | | | 20 | | 15 | | | | 0.3 | | 0.3 | | | 0.1 |
| Example A3 | | | 5 | | | | 5 | | 5 | 50 | | | 25 | | 10 | | | | 0.3 | 2 | 0.3 | | | 0.1 |
| Example A4 | | | | 10 | | | 10 | | 5 | 45 | | | 15 | | 15 | | | | 0.3 | | 0.3 | 0.5 | | 0.1 |
| Example A5 | | | | | 10 | | | 5 | 5 | | | 5 | 70 | 5 | | | | | 0.3 | | 0.3 | | 0.1 | 0.1 |
| Example A6 | | | | | | 10 | 5 | | 5 | 40 | | | 25 | | 15 | | | | 0.3 | | 0.3 | | | 0.1 |
| Example A7 | 4 | | | | | | 5 | | 5 | 50 | | | | | | 36 | | | 0.3 | | 0.3 | | | 0.1 |
| Example A8 | 4 | | | | | | 5 | | 5 | | | 1 | 70 | 15 | | | | | 0.3 | | 0.3 | | | 0.1 |
| Example A9 | | | | | 8 | | 10 | | 5 | 30 | | | 30 | 17 | | | | | 0.3 | | 0.3 | | | 0.1 |
| Example A10 | 5 | | | | | | | 5 | 5 | 30 | | | 35 | 20 | | | | | 0.3 | | 0.3 | | | 0.1 |
| Example A11 | 4 | | | | | | 5 | | 5 | | 16 | | 17 | | | 30 | 18 | 5 | 0.3 | | 0.3 | | | 0.1 |
| Example A12 | 4 | | | | | | 5 | | 5 | 50 | 21 | | | 15 | | | | | 0.3 | | 0.3 | | | 0.1 |
| Example A13 | 4 | | | | | | 5 | | 5 | 66 | | | | | 20 | | | | 0.3 | | 0.3 | | | 0.1 |
| Example A14 | 4 | | | | | | | 10 | | | | 1 | | 10 | 10 | 60 | | 5 | 0.3 | | 0.3 | | | |
| Example A15 | 4 | | | | | | 5 | | 5 | 62 | | | 20 | | 4 | | | | 0.3 | | 0.3 | | | 0.1 |
| Example A16 | 0.5 | | | | | | | 10 | 5 | 50.5 | | | 15 | 14 | | | | 5 | 0.3 | | 0.3 | | | 0.1 |
| Example A17 | | | | | 1 | | 10 | | 5 | | 10 | | 45 | 19 | | | | 10 | 0.3 | | 0.3 | | | 0.1 |
| Example A18 | 2 | | | | | 3 | 8 | | 5 | 50 | | | 17 | 15 | | | | | 0.3 | | 0.3 | | | 0.1 |

[Table 2]

| | Filler | | | | | | | | Total amount of silane coupling material compounding amount index in matrix | Compounding amount of polymerizable monomer having no acidic group and having one or more hydroxyl group with respect to 100 parts by mass of matrix compounded with silane coupling material | Compounding amount of compound having methacryloyl group and/or methacrylamide group contained in 100 parts by mass of matrix | Durable adhesive strength | | | | Discoloration resistance property | Paste property |
|---|---|---|---|---|---|---|---|---|---|---|---|---|---|---|---|---|---|
| | Without surface treatment | | Surace treated | | | | | | | | | Initial preparation product | Acceleration test product | Initial preparation product | Acceleration test product | | |
| | Filler A | Filler C | Filler D | Filler E | Filler F | Filler G | Filler H | Filler I | | | | Lithium disilicate | | Tooth substance | | | |
| Example A1 | | | | 20 | 180 | | | | 0.038 | 49.7 | 96.3 | 17.7 | 19.6 | 14.0 | 14.7 | A | A |
| Example A2 | | | | | 180 | | 20 | | 0.040 | 49.7 | 94.3 | 17.9 | 17.5 | 14.0 | 13.8 | A | A |
| Example A3 | | | | | | | 20 | 180 | 0.045 | 48.7 | 92.5 | 18.7 | 19.9 | 13.6 | 13.5 | A | A |
| Example A4 | | | | | | 180 | 20 | | 0.049 | 44.5 | 88.9 | 19.5 | 17.8 | 14.4 | 13.5 | A | A |
| Example A5 | | | | | | | | 200 | 0.063 | 5.0 | 89.3 | 19.6 | 12.4 | 6.7 | 8.2 | A | A |
| Example A6 | | | | 20 | 180 | | | | 0.052 | 39.7 | 89.4 | 18.5 | 19.0 | 14.2 | 15.0 | A | A |
| Example A7 | | | | 20 | 180 | | | | 0.051 | 49.7 | 59.6 | 11.3 | 11.1 | 12.1 | 12.9 | A | A |
| Example A8 | | | | | | | | 200 | 0.051 | 1.0 | 95.3 | 11.8 | 10.5 | 13.8 | 15.0 | A | A |
| Example A9 | 20 | | | 180 | | | | | 0.050 | 29.8 | 91.4 | 19.5 | 10.2 | 12.5 | 12.4 | A | B |
| Example A10 | | 20 | | | 180 | | | | 0.064 | 29.8 | 94.3 | 19.6 | 10.2 | 9.1 | 8.3 | A | B |
| Example A11 | | | | 20 | 180 | | | | 0.051 | 15.9 | 42.7 | 10.4 | 11.1 | 13.4 | 13.6 | A | A |
| Example A12 | | | | | | | | | 0.051 | 70.5 | 95.3 | 14.7 | 11.0 | 14.3 | 12.6 | A | A |
| Example A13 | | | 200 | | | | | | 0.051 | 65.5 | 95.3 | 17.5 | 12.4 | 14.8 | 12.8 | A | B |
| Example A14 | | | | 20 | 180 | | | | 0.051 | 1.0 | 30.8 | 10.2 | 11.5 | 13.2 | 14.1 | A | A |
| Example A15 | | | | 20 | 180 | | | | 0.051 | 61.6 | 95.3 | 10.4 | 12.9 | 14.9 | 13.2 | A | A |
| Example A16 | | | | 20 | 180 | | | | 0.006 | 50.1 | 93.8 | 11.7 | 10.0 | 14.2 | 14.7 | A | A |
| Example A17 | | | | | | 200 | | | 0.006 | 9.9 | 88.4 | 10.2 | 11.2 | 13.1 | 14.7 | A | A |
| Example A18 | | | | | | | 200 | | 0.041 | 49.7 | 94.3 | 18.2 | 18.6 | 13.4 | 13.4 | A | A |

[Table 3]

| | (A) Silane coupling material | | | | | | (B) polymerizable monomer having an acidic group | | | (C) Polymerizable monomer having no acidic group | | | | | | | | | Photo polymerization initiator | Chemical polymerization initiator | Polymerization accelerator | | | Polymerization inhibitor |
|---|---|---|---|---|---|---|---|---|---|---|---|---|---|---|---|---|---|---|---|---|---|---|---|---|
| | (A1) Silane coupling material represented by structural formula of [Chemical formula 1] | | | | (A) other than (A1) | | | | | (C1) Having one or more hydroxyl groups | | | Having no hydroxy group | | | Having no hydroxy group and no methacryloyl group | | | | | | | | |
| | APTMS | APMES | C11A | C11DA | MPTMS | MOTMS | MDP | MHPA | META | BisGMA | GDMA | HEMA | UDMA | 3G | NPG | D2.6E | DMCDA | TMPTA | CQ | CHP | DMBE | PTU | VOA | BHT |
| Comparative Example CA1 | 6 | | | | | | | 5 | 5 | 54 | | | 30 | | | | | | 0.3 | | 0.3 | | | 0.1 |
| Comparative Example CA2 | | | 12 | | | | | 5 | 5 | | | 8 | 70 | | | | | | 0.3 | | 0.3 | | | 0.1 |
| Comparative Example CA3 | 0.2 | | | | | | 10 | | | 20 | 10 | | 30 | 29.8 | | | | | 0.3 | | 0.3 | | | 0.1 |
| Comparative Example CA4 | | | | 0.8 | | | | 10 | 5 | 20 | 10 | | 30 | 24.2 | | | | | 0.3 | | 0.3 | | | 0.1 |
| Comparative Example CA5 | | | | | 10 | | 7 | 5 | 5 | 30 | | 5 | 30 | 8 | | | | | 0.3 | | 0.3 | | | 0.1 |
| Comparative Example CA6 | | | | | | 8 | | 5 | 5 | 30 | 5 | | 30 | 17 | | | | | 0.3 | | 0.3 | | | 0.1 |
| Comparative Example CA7 | | | | | | | 10 | 5 | 5 | 50 | | 25 | 5 | | | | | | 0.3 | | 0.3 | | | 0.1 |
| Comparative Example CA8 | | | | | 10 | | 12 | | 5 | 30 | | 5 | 30 | 8 | | | | | 0.3 | | 0.3 | | | 0.1 |
| Comparative Example CA9 | | | | | | 8 | | 10 | | 30 | 5 | | 30 | 17 | | | | | 0.3 | | 0.3 | | | 0.1 |
| Comparative Example CA10 | 5 | | | | | | | | | | | 20 | 5 | | | 70 | | | 0.3 | | 0.3 | | | 0.1 |
| Comparative Example CA11 | | | | | | 5 | | | | 20 | | | 50 | 25 | | | | | 0.3 | | 0.3 | | | 0.1 |

[Table 4]

| | Filler | | | | | | | | Total amount of silane coupling material compounding amount index in matrix | Compounding amount of polymerizable monomer having no acidic group and having one or more hydroxyl group with respect to 100 parts by mass of matrix compounded with silane coupling material | Compounding amount of compound having methacryloyl group and/or methacrylamide group contained in 100 parts by mass of matrix | Durable adhesive strength | | | | Discoloration resistance property | Paste property |
|---|---|---|---|---|---|---|---|---|---|---|---|---|---|---|---|---|---|
| | Without surface treatment | | Surace treated | | | | | | | | | Initial preparation product | Acceleration test product | Initial preparation product | Acceleration test product | | |
| | Filler A | Filler C | Filler D | Filler E | Filler F | Filler G | Filler H | Filler I | | | | Lithium disilicate | | Tooth substance | | | |
| Comparative Example CA1 | | | 200 | | | | | | 0.076 | 53.6 | 93.3 | 14.2 | 6.5 | 7.0 | 6.1 | C | C |
| Comparative Example CA2 | | | | 200 | | | | | 0.075 | 7.9 | 87.4 | 17.4 | 6.8 | 5.4 | 7.0 | C | B |
| Comparative Example CA3 | | | | | | | 200 | | 0.003 | 29.7 | 90.1 | 2.9 | 1.5 | 12.9 | 14.2 | A | A |
| Comparative Example CA4 | | | | | | 200 | | | 0.004 | 29.9 | 96.5 | 2.0 | 2.3 | 12.0 | 14.6 | A | A |
| Comparative Example CA5 | | | 200 | | | | | | 0.120 | 34.8 | 89.4 | 10.3 | 6.7 | 5.1 | 5.2 | C | C |
| Comparative Example CA6 | | | | | | | | 200 | 0.072 | 34.8 | 91.4 | 10.7 | 5.7 | 5.3 | 5.9 | C | A |
| Comparative Example CA7 | 200 | | | | | | | | 0.000 | 74.5 | 99.3 | 1.7 | 1.0 | 12.3 | 14.3 | A | A |
| Comparative Example CA8 | 200 | | | | | | | | 0.120 | 34.8 | 89.4 | 10.0 | 0.7 | 5.9 | 6.0 | C | C |
| Comparative Example CA9 | | | | | | | | 200 | 0.072 | 34.8 | 91.4 | 10.9 | 5.3 | 5.4 | 5.6 | C | A |
| Comparative Example CA10 | | | | 200 | | | | | 0.064 | 19.9 | 24.8 | 10.3 | 10.9 | 0.7 | 0.5 | A | A |
| Comparative Example CA11 | | | 200 | | | | | | 0.045 | 19.9 | 94.3 | 6.6 | 6.5 | 0.3 | 0.9 | A | A |

[Table 5]

| | Paste | (A1) Silane coupling material represented by structural formula of [Chemical formula 1] | | | | | | (B) Polymerizable monomer having an acidic group | | | (C) Polymerizable monomer having no acidic group | | | | | | | | | Photo polymerization initiator | Chemical polymerization initiator | | Polymerization accelerator | | | | | | Polymerization inhibitor |
|---|---|---|---|---|---|---|---|---|---|---|---|---|---|---|---|---|---|---|---|---|---|---|---|---|---|---|---|---|---|
| | | | | | | | | | | | (C1) Having one or more hydroxyl groups | | | Having no hydroxy group | | | Having no hydroxy group and no methacryloyl group | | | | | | | | | | | | |
| | | APTMS | APMES | APMMS | APDMS | CHA | CHDA | MDP | MHPA | META | BisGMA | GDMA | HEMA | UDMA | 3G | NPG | A3.0E | DMCDA | TMPTA | CQ | CHP | TMBH | DMBE | PTU | BTU | VOA | COA | PTSA | BHT |
| Example B1 | First | 5 | | | | | | | | | 50 | 20 | | | 25 | | | | | 0.3 | | | | 2 | | | 0.01 | | 0.1 |
| | Second | | | | | | | 5 | | 5 | 50 | 20 | | | 20 | | | | | | 3 | | 0.3 | | | | | | 0.2 |
| Example B2 | First | | | | 10 | | | | | | 50 | 20 | | | 20 | | | | | | 3 | | 0.3 | | | | | 0.5 | 0.03 |
| | Second | | | | | | | 10 | | | 50 | | | | | 40 | | | | 0.2 | | | | | 2 | | | | 0.15 |
| Example B3 | First | 8 | | | | | | | | | | 10 | 10 | 63 | | 10 | | | | | 2.6 | | 0.3 | | | | | | 0.1 |
| | Second | | | | | | | 5 | | 5 | 50 | 20 | | | 20 | | | | | 0.2 | | | | | 1 | | 0.05 | | 0.2 |
| Example B4 | First | 8 | | | | | | | | | | 12 | | 60 | | 20 | | | | | 2.5 | | 0.3 | | | | | | 0.1 |
| | Second | | | | | | | 5 | | 5 | 50 | 20 | 20 | | | | | | | 0.3 | | | | | 1 | | 0.05 | | 0.2 |
| Example B5 | First | 2 | 10 | | | | | | | | 53 | 20 | | | 15 | | | | | | 2.8 | | 0.3 | | | | | | 0.03 |
| | Second | | | | | | | 10 | | | 50 | | | | | 40 | | | | 0.2 | | | | | 1 | | | 0.05 | 0.15 |
| Example B6 | First | 3 | | | | | 10 | | | | | 27 | | 60 | | | | | | | 2.9 | | 0.3 | | | | | | 0.1 |
| | Second | | | | | | | 5 | | 5 | 50 | 20 | 20 | | | | | | | 0.2 | | | | 1 | | | 0.05 | | 0.2 |
| Example B7 | First | | 5 | | | | | | | | | 15 | | 50 | | 30 | | | | 0.5 | | | | 1 | | 0.03 | | | 0.1 |
| | Second | | | | | | | 5 | | 5 | 50 | 20 | | | 20 | | | | | | | 2.3 | 0.3 | | | | | | 0.2 |
| Example B8 | First | | | 10 | | | | | | | | 15 | | 10 | | 25 | 35 | 5 | | 0.3 | | | | | 1 | 0.03 | | | 0.1 |
| | Second | | | | | | | 5 | | 5 | | 20 | | | 25 | | 40 | | 5 | | 3.4 | | 0.3 | | | | | | 0.2 |
| Example B9 | First | 5 | | | | | | | | | | | 15 | | | 20 | 60 | | | | 3 | | 0.3 | | | | | | 0.1 |
| | Second | | | | | | | 5 | | 5 | | 10 | | 60 | 20 | | | | | 0.2 | | | | | 1 | | 0.05 | | 0.2 |
| Example B10 | First | 5 | | | | | | | | | | | 15 | 60 | | 20 | | | | | 3 | | 0.3 | | | | | | 0.1 |
| | Second | | | | | | | 5 | | 5 | | 10 | | 60 | 20 | | | | | 0.3 | | | | | 1 | | 0.05 | | 0.2 |
| Example B11 | First | 1 | | | | | | | | | | 20 | | 60 | 19 | | | | | 0.3 | | | | | 0.5 | | 0.1 | | 0.1 |
| | Second | | | | | | | 5 | | 5 | | 15 | | 50 | 25 | | | | | | 2.5 | | 0.3 | | | | | | 0.2 |
| Example B12 | First | | 1.5 | | | | | | | | | 15 | | 28.5 | | 20 | 30 | | 5 | 0.3 | | | | 1 | | 0.03 | | | 0.1 |
| | Second | | | | | | | 5 | | 5 | 20 | 20 | | | 20 | | 25 | 5 | | | | 3.5 | 0.3 | | | | | | 0.2 |
| Example B13 | First | | | | | 2 | | | | | | | 15 | 65 | | 20 | | | | 0.1 | | | | 1 | | 0.03 | | | 0.1 |
| | Second | | | | | | | | 10 | | 30 | 20 | | 20 | 20 | | | | | | 3 | | 0.2 | | | | | | 0.2 |
| Example B14 | First | | 15 | | | | | | | | | 15 | | 50 | | 20 | | | | | | | | | 1 | 0.03 | | | 0.1 |
| | Second | | | | | | | 5 | | 5 | 50 | 20 | | | 20 | | | | | | | 3.5 | | | | | | | 0.2 |
| Example B15 | First | 10.5 | | | | | | | | | 50 | 20 | | | 19.5 | | | | | | 3 | | 0.3 | | | | | | 0.1 |
| | Second | | | | | | | 5 | | 5 | 50 | 20 | | | 20 | | | | | 0.2 | | | | 1 | | | 0.01 | | 0.05 |
| Example B16 | First | 10.5 | | | | | | | | | 50 | 20 | | | 19.5 | | | | | | 3 | | 0.3 | | | | | | 0.1 |
| | Second | | | | | | | | 10 | | 50 | 20 | | | 20 | | | | | 0.2 | | | | 1.5 | | 0.03 | | | 0.05 |
| Example B17 | First | 10.5 | | | | | | | | | | 10 | | | | | 69.5 | 10 | | | | 3 | 0.3 | | | | | 0.03 | 0.1 |
| | Second | | | | | | | | 10 | | 50 | 20 | | | 10 | | | | 10 | 0.2 | | | | 1.5 | | 0.03 | | | 0 |
| Example B18 | First | 5 | | | | | | | | | | | 10 | | | | 85 | | | 0.3 | | | | 2 | | | 0.01 | | 0.1 |
| | Second | | | | | | | 15 | | 5 | | | | | | | 65 | | 15 | | 3 | | 0.3 | | | | | | 0.2 |
| Example B19 | First | | | | | | 22 | | | | 50 | | 18 | | 10 | | | | | | 2 | | 0.3 | | | | | | 0.1 |
| | Second | | | | | | | | 10 | | 50 | 15 | | | 25 | | | | | 0.2 | | | | 1 | | 0.03 | | | 0 |
| Example B20 | First | 3 | | | | 10 | | | | | | 5 | | 65 | | 19 | | | | | 3 | | 0.3 | | | | | | 0.1 |
| | Second | | | | | | | 5 | | 5 | 45 | 20 | | | 25 | | | | | 0.2 | | | | | 1 | | 0.05 | | 0.2 |
| Example B21 | First | 8 | | | | | | | | | | 20 | | 62 | | 10 | | | | | 3 | | 0.3 | | | | | | 0.1 |
| | Second | | | | | | | 20 | | 15 | | | | 50 | 15 | | | | | 0.2 | | | | 1 | | | 0.01 | | 0.05 |
| Example B22 | First | 8 | | | | | | | | | | 20 | | 62 | | 10 | | | | | 3 | | 0.3 | | | | | | 0.1 |
| | Second | | | | | | | 20 | | 15 | | | | 50 | 15 | | | | | 0.2 | | | | 1.5 | | 0.03 | | | 0.05 |

[Table 6]

| | Paste | Filler | | | | | | | | Total amount of silane coupling material compounding amount index in matrix | Compounding amount of polymerizable monomer having no acidic group and having one or more hydroxyl group with respect to 100 parts by mass of matrix compounded with silane coupling material | Compounding amount of compound having methacryloyl group and/or methacrylamide group contained in 100 parts by mass of matrix | Durable adhesive strength | | | | Discoloration resistance property | Paste property |
|---|---|---|---|---|---|---|---|---|---|---|---|---|---|---|---|---|---|---|
| | | Without surface treatment | | Surace treated | | | | | | | | | Initial preparation product | Acceleration test product | Initial preparation product | Acceleration test product | | |
| | | Filler A | Filler C | Filler D | Filler E | Filler F | Filler G | Filler H | Filler I | | | | Lithium disilicate | | Tooth substance | | | |
| Example B1 | First | | | | 200 | | | | | 0.031 | 68.4 | 94.7 | 18.1 | 12.3 | 15.0 | 13.1 | A | A |
| | Second | | | 200 | | | | | | | | | | | | | | |
| Example B2 | First | | | | 150 | 50 | | | | 0.023 | 67.4 | 92.3 | 19.3 | 14.3 | 14.5 | 13.5 | A | A |
| | Second | | | 180 | | | | | | | | | | | | | | |
| Example B3 | First | | | | 200 | | | | | 0.050 | 19.4 | 93.9 | 18.2 | 19.6 | 14.8 | 13.1 | A | A |
| | Second | | | | 200 | | | | | | | | | | | | | |
| Example B4 | First | | | | 150 | 50 | | | | 0.050 | 11.7 | 94.0 | 19.4 | 18.0 | 14.6 | 13.3 | A | A |
| | Second | | 20 | 180 | | | | | | | | | | | | | | |
| Example B5 | First | | | | 150 | 20 | | | | 0.054 | 70.8 | 91.9 | 20.0 | 10.5 | 13.1 | 14.3 | A | A |
| | Second | | | 200 | | | | | | | | | | | | | | |
| Example B6 | First | | | | 150 | 20 | | | | 0.046 | 26.1 | 91.3 | 18.5 | 18.7 | 13.7 | 13.9 | A | A |
| | Second | | | 200 | | | | | | | | | | | | | | |
| Example B7 | First | | | | 200 | | | | | 0.020 | 14.8 | 95.5 | 19.0 | 15.2 | 13.9 | 13.1 | A | A |
| | Second | | | | | | | 200 | | | | | | | | | | |
| Example B8 | First | | | 180 | | | | | | 0.046 | 14.8 | 51.1 | 12.8 | 13.9 | 13.9 | 13.7 | A | B |
| | Second | | | | | | | 200 | | | | | | | | | | |
| Example B9 | First | | | | 150 | 50 | | | | 0.031 | 14.5 | 62.9 | 13.9 | 14.3 | 14.0 | 13.1 | A | A |
| | Second | | | 200 | | | | | | | | | | | | | | |
| Example B10 | First | | | | 150 | 50 | | | | 0.031 | 14.5 | 95.3 | 17.1 | 15.1 | 13.4 | 13.0 | A | A |
| | Second | | | 200 | | | | | | | | | | | | | | |
| Example B11 | First | | | | 200 | | | | | 0.006 | 19.8 | 97.5 | 10.2 | 10.9 | 13.7 | 15.0 | A | A |
| | Second | | | 200 | | | | | | | | | | | | | | |
| Example B12 | First | | | | | | | 200 | | 0.006 | 14.8 | 65.0 | 10.1 | 11.3 | 14.0 | 13.2 | A | A |
| | Second | | | | | | | | 200 | | | | | | | | | |
| Example B13 | First | | | | | 100 | 100 | | | 0.006 | 14.8 | 96.8 | 10.3 | 10.2 | 14.2 | 13.4 | A | A |
| | Second | | | | | | | 200 | | | | | | | | | | |
| Example B14 | First | | | | 100 | 100 | | | | 0.060 | 14.8 | 90.3 | 18.7 | 13.6 | 9.8 | 9.6 | B | A |
| | Second | | | 200 | | | | | | | | | | | | | | |
| Example B15 | First | | | 200 | | | | | | 0.065 | 67.7 | 92.6 | 19.2 | 10.8 | 9.5 | 8.7 | B | B |
| | Second | | | 200 | | | | | | | | | | | | | | |
| Example B16 | First | | | | | | 200 | | | 0.065 | 67.7 | 92.4 | 18.5 | 11.5 | 8.4 | 8.3 | B | A |
| | Second | | | 200 | | | | | | | | | | | | | | |
| Example B17 | First | | | | | | | | 200 | 0.065 | 9.7 | 48.7 | 14.5 | 11.3 | 8.4 | 9.4 | B | A |
| | Second | | | 200 | | | | | | | | | | | | | | |
| Example B18 | First | | | | 200 | | | | | 0.031 | 9.8 | 14.6 | 10.5 | 11.8 | 14.7 | 11.5 | A | A |
| | Second | | | 200 | | | | | | | | | | | | | | |
| Example B19 | First | | | | | | | 200 | | 0.056 | 66.4 | 87.4 | 17.9 | 10.5 | 8.5 | 9.0 | B | A |
| | Second | | | 200 | | | | | | | | | | | | | | |
| Example B20 | First | | | | 150 | 50 | | | | 0.049 | 4.8 | 91.8 | 10.9 | 10.8 | 10.2 | 11.6 | A | A |
| | Second | | | 200 | | | | | | | | | | | | | | |
| Example B21 | First | 20 | | 180 | | | | | | 0.050 | 19.3 | 93.8 | 17.8 | 10.1 | 13.1 | 13.4 | A | B |
| | Second | 20 | | 180 | | | | | | | | | | | | | | |
| Example B22 | First | | 20 | | 180 | | | | | 0.050 | 19.3 | 93.6 | 19.9 | 10.3 | 14.8 | 13.8 | A | B |
| | Second | | 20 | | 180 | | | | | | | | | | | | | |

[Table 7]

| | | (A) Silane coupling material | | | | | | (B) Polymerizable monomer having an acidic group | | | (C) Polymerizable monomer having no acidic group | | | | | | | | |
|---|---|---|---|---|---|---|---|---|---|---|---|---|---|---|---|---|---|---|---|
| | | (A1) Silane coupling material represented by structural formula of [Chemical formula 1] | | | | (A) other than (A1) | | | | | (C1) Having one or more hydroxyl groups | | | Having no hydroxy group | | | Having no hydroxy group and no methacryloyl group | | |
| | Paste | APTMS | APMES | C11A | C11DA | MPTMS | MOTMS | MDP | MHPA | META | BisGMA | GDMA | HEMA | UDMA | 3G | NPG | A3.0E | DMCDA | TMPTA |
| Comparative Example CB1 | First | 0.6 | | | | | | | | | | 30 | | 30 | | | 39.4 | | |
| | Second | | | | | | | 10 | | 5 | 50 | 15 | | | 20 | | | | |
| Comparati Example CB2 | First | | | 1 | | | | | | | | 20 | | 61 | 18 | | | | |
| | Second | | | | | | | 5 | | 5 | 50 | 20 | | | 20 | | | | |
| Comparati Example CB3 | First | 12 | | | | | | | | | | 30 | | 20 | | | 38 | | |
| | Second | | | | | | | 15 | | 5 | 50 | 10 | | | 20 | | | | |
| Comparati Example CB4 | First | | | 23 | | | | | | | 47 | | | | 30 | | | | |
| | Second | | | | | | | | 15 | 5 | 50 | 20 | | | 10 | | | | |
| Comparati Example CB5 | First | 5 | | | | | | | | | | | 15 | 60 | | 20 | | | |
| | Second | | | | | | | | | | | 10 | 10 | 60 | 20 | | | | |
| Comparati Example CB6 | First | | | | | 13 | | | | | | | 15 | 60 | | 12 | | | |
| | Second | | | | | | | | | | | 10 | 10 | 60 | 20 | | | | |

| | | Photo polymerization initiator | Chemical polymerization initiator | | Polymerization accelerator | | | | | | Polymerization inhibitor |
|---|---|---|---|---|---|---|---|---|---|---|---|
| | Paste | CQ | CHP | TMBH | DMBE | PTU | BTU | VOA | COA | PTSA | BHT |
| Comparative Example CB1 | First | | 3 | | 0.3 | | | | | 0.2 | 0.2 |
| | Second | 0.2 | | | | 1 | | | 0.03 | 0.1 | 0.2 |
| Comparati Example CB2 | First | 0.2 | | | | | 1 | 0.03 | | 0.1 | 0.2 |
| | Second | | 3 | | 0.3 | | | | | 0.2 | 0.2 |
| Comparati Example CB3 | First | | 3 | | 0.3 | | | | | 0.2 | 0.2 |
| | Second | 0.2 | | | | | 1 | 0.03 | | 0.1 | 0.2 |
| Comparati Example CB4 | First | 0.2 | | | | 1 | | | 0.03 | 0.1 | 0.2 |
| | Second | | 3 | | 0.3 | | | | | 0.1 | 0.2 |
| Comparati Example CB5 | First | | 3 | | 0.3 | | | | | | 0.1 |
| | Second | 0.2 | | | | | 1 | | 0.05 | | 0.2 |
| Comparati Example CB6 | First | | 3 | | 0.3 | | | | | | 0.1 |
| | Second | 0.2 | | | | | 1 | | 0.05 | | 0.2 |

[Table 8]

| | Filler | | | | | | | | Total amount of silane coupling material compounding amount index in matrix | Compounding amount of polymerizable monomer having no acidic group and having one or more hydroxyl group with respect to 100 parts by mass of matrix compounded with silane coupling material | Compounding amount of compound having methacryloyl group and/or methacrylamide group contained in 100 parts by mass of matrix | Durable adhesive strength | | | | Discoloration resistance property | Paste property |
|---|---|---|---|---|---|---|---|---|---|---|---|---|---|---|---|---|---|
| | Without surface treatment | | Surace treated | | | | | | | | | Initial preparation product | Acceleration test product | Initial preparation product | Acceleration test product | | |
| | Filler A | Filler C | Filler D | Filler E | Filler F | Filler G | Filler H | Filler I | | | | Lithium disilicate | | Tooth substance | | | |
| Comparative Example CB1 | | | 200 | | | | | | 0.004 | 28.9 | 78.0 | 4.4 | 3.6 | 12.0 | 10.7 | A | A |
| | | | 200 | | | | | | | | | | | | | | |
| Comparati Example CB2 | | | | | 200 | | | | 0.004 | 19.7 | 97.0 | 4.1 | 3.2 | 13.8 | 11.3 | A | A |
| | | | 200 | | | | | | | | | | | | | | |
| Comparati Example CB3 | | | 200 | | | | | | 0.075 | 28.9 | 73.1 | 18.4 | 8.4 | 6.7 | 5.6 | C | C |
| | | | | 200 | | | | | | | | | | | | | |
| Comparati Example CB4 | | | | | 200 | | | | 0.103 | 46.8 | 86.8 | 19.8 | 8.5 | 7.0 | 5.2 | C | A |
| | | | | 200 | | | | | | | | | | | | | |
| Comparati Example CB5 | | | | 150 | 50 | | | | 0.031 | 14.5 | 95.2 | 12.1 | 13.6 | 1.8 | 1.0 | A | A |
| | | | 200 | | | | | | | | | | | | | | |
| Comparati Example CB6 | | | | 150 | 50 | | | | 0.040 | 14.5 | 91.3 | 8.2 | 4.6 | 1.1 | 1.2 | A | A |
| | | | 200 | | | | | | | | | | | | | | |

Examples A1 to A18 and Examples B1 to B22 contained the (A1) silane coupling material represented by structural formula of [Chemical formula 1] in the matrix and the total amount of silane coupling material compounding amount index in matrix satisfied the formula (1). Therefore, durable adhesive property to glass ceramics containing lithium disilicate was excellent, and discoloration resistance property was excellent.

Although Examples A5, A10, B14, B15, B16, B17 and B19 satisfied the formula (1), the total amount of silane coupling material compounding amount index in matrix was in the vicinity of the upper limit of the formula (1). Therefore, there was a case where the adhesive strength to glass ceramics containing lithium disilicate of the acceleration test product was lower than that of the initial preparation product, a case where the adhesive strength to dentin was reduced and a case where it was confirmed that the discoloration resistance property was reduced.

Although Examples A16, A17, B11, B12 and B13 satisfied the formula (1), the total amount of silane coupling material compounding amount index in matrix was in the vicinity of the lower limit of the formula (1). Therefore, the adhesive strength to glass ceramics containing lithium was 10 MPa or more, however it was confirmed that the adhesive strength was slightly low.

In Examples B1, B2, B5, B15, B16, B19, A12, A13 and A15, the compounding amount of the polymerizable monomer having no acidic group and having one or more hydroxyl groups in the matrix containing the silane coupling material exceeded 60%. Therefore, it was confirmed that the adhesive strength to the glass ceramics containing lithium disilicate of the acceleration test product was lower than that of the initial preparation product. In particular, it was

remarkable in Examples 12A and B5 in which the compounding amount exceeded 70%.

In Examples A8, A14 and B20, the compounding amount of the polymerizable monomer having no acidic group and having one or more hydroxyl groups in the matrix containing the silane coupling material is less than 5%. Therefore, although the adhesive strength to glass ceramics containing lithium disilicate was 10 MPa or more, it was confirmed that the adhesive strength was slightly low.

In Examples A7, A11, A14, B8, B9, B12, B17 and B18, the compounding amount of the polymerizable monomer having a methacryloyl group contained in the matrix is less than 70%. Therefore, although the adhesive strength to glass ceramics containing lithium disilicate was 10 MPa or more, it was confirmed that the adhesive strength was slightly low. In particular, it was remarkable in Examples A11, A14, B17 and B18 in which the compounding were less than 50%.

In Examples A13, B8 and B15 which contained the filler D prepared by treating a silica filler having a $SiO_2$ content of 99% or more with a silane coupling material and a silane coupling material having an acryloyl group in the matrix, it was confirmed that there was a tendency that the fluidity of the paste were increased after the acceleration test, but it was within an acceptable range.

In Examples A9, A10, B21 and B22 containing the filler A and the filler B not treated with the silane coupling material and a silane coupling material in the matrix, although the adhesive strength to the glass ceramics containing lithium disilicate after the acceleration test was 10 MPa or more, it was confirmed that the adhesive strength was slightly decreased, the fluidity of the paste was increased and the operability was deteriorated.

In Comparative Examples CA1, CA2, CB3 and CB4, the compounding amount exceeded the upper limit of the total amount of the silane coupling material compounding amount index in matrix of the formula (1). Therefore, the adhesive strength to the glass ceramics containing lithium disilicate after the acceleration test was 10 MPa or less and low, and the adhesive strength to dentin was also reduced. In addition, the discoloration resistance property was also reduced.

In Comparative Examples CA3, CA4, CB1 and CB2, since the compounding amount was less than the lower limit of the total amount of the silane coupling material compounding amount index in matrix of the formula (1). Therefore, the adhesive strength to the glass ceramics containing lithium disilicate was 10 MPa or less and low.

The compositions in Comparative Examples CA5, CA6, CA8 and CA10 were compositions in which a conventional silane coupling material having a methacryloyl group was compounded in the matrix in a compounding amount exceeding the upper limit of the total amount of the silane coupling material compounding amount index in the matrix of the formula (1). Although the adhesive strength to glass ceramics containing lithium disilicate of the initial preparation product was 10 MPa or more, the adhesive strength to glass ceramics containing lithium disilicate of the acceleration test product was10 MPa or less and low and there was a tendency that the adhesive strength to dentin was low. Furthermore, the discoloration resistance property also tended to be low.

Comparative Examples CA10, CA11, CB5 and CB6 did not contain a polymerizable monomer having an acidic group, and therefore there was a tendency that the adhesive strength to dentin was low.

The compositions of Comparative Examples CA1, CA5 and CB3 contained the filler D in which a silica filler having a $SiO_2$ content of 99% or more was treated with a silane coupling material and a silane coupling material in a compounding amount exceeding the upper limit of the total amount of the silane coupling material compounding amount index in the matrix of the formula (1). It was confirmed that the paste fluidity was remarkably improved after the acceleration test and the stability of the paste property was poor.

[Two-paste type dental adhesive composition containing (D1) chemical polymerization initiator and (E) polymerization accelerator, and containing 15 to 80 parts by mass of (C1) polymerizable monomer havingo acidic group and having one or more hydroxyl groups with respect to 100 parts by mass of the first matrix and the second matrix]

(Preparing method of paste)

The polymerizable monomers shown in Examples and Comparative Examples were put into a light-shielding plastic container and mixed by using a mix rotor VMRC-5 under the condition of 100 rpm for 48 hours. Thereafter, the polymerization initiator was added and further mixed by using a mix rotor VMRC-5 under the condition of 100 rpm for 48 hours to prepare a resin liquid. The resin liquid and filler were put into a kneading container, and were kneaded for 20 minutes at 1000 rpm using a rotation and revolution mixer ARV-300 to prepare the first paste and the second paste.

The test method of each characteristic evaluated in the example and the comparative example is as follows. The first paste and the second paste were weighed with an electronic analytical scale in a dark room at 23 ± 2 °C, and mixing them under the condition of equal mass. In the case where the volume ratio was 1.0: 0.8 to 1.2, and in the preferably case where the volume ratio was 1.0: 0.9 to 1.1, the same result was obtained in the test by using a mixing chip manufactured Mixpack Co., Ltd.

(Initial preparation product and Acceleration test product)

The compositions described in Examples and Comparative Examples were filled in 5 mL double syringe manufactured by Mixpack Co., Ltd., and stored in a constant temperature incubator (manufactured by Yamato Scientific Co., Ltd.) at 40 °C for 4 months, and was used as acceleration test product. On the other hand, the initial preparation product indicates that it was used within 3 months under the condition of 1 to 30 °C after preparation.

(Adhesive strength to tooth substance)

A test specimen of an epoxy resin embedded bovine anterior tooth was polished with water-resistant abrasive paper #600 to carve out a dentin plane. Thereafter, a tape with a hole having a diameter of 4 mm was affixed to the adherend surface to prescribe an adhesion area. On the other hand, the adherend surface of the stainless rod (φ4.5 mm) was sandblasted (0.2 MPa, 1 second) with alumina (50 μm), then was washed with water and dried, and applied with a metal adhesive primer (METAL LINK, manufactured by SHOFU INC.). The first paste and the second paste which correspond the dental adhesive composition of the Example or Comparative example were sufficiently kneaded in equal mass and was applied to the adherend surface of the stainless rod in an appropriate amount, and the glass ceramics containing lithium disilicate and the stainless rod were bonded so as to fit in the frame of the double-sided tape with a hole. Aload of 200 N was applied from the vertical direction of the stainless rod, and excess cement was wiped off with a cloth. Thereafter, Light irradiation was performed for 10 seconds with the dental polymerization LED light curing unit (PEN Bright, manufactured by SHOFU INC.). After removing the load and immersing in water at 37 °C for 24 hours, immersing alternately in cold water phase at 4 °C for 60 seconds and in high temperature phase at 60 °C for 60 seconds was repeated 5000 times by using a thermal shock tester (manufactured by THOMAS KAGAKU Co.,Ltd). The specimen was taken out and a universal tester (manufactured by Instron) was used to measure the tensile adhesive strength at a crosshead speed of 1 mm/min. When the adhesive strength was 10 MPa or more, it was determined to have excellent adhesive strength. On the other hand, when the adhesive strength was less than 5 MPa, it was determined that the adhesive strength was low.

(Adhesive strength to dental resin for cutting and machining (resin block))

A resin block (SHOFU BLOCK HC SUPER HARD manufactured by SHOFU INC.) was processed into a plate having a thickness of 3 mm using an isomet (manufactured by JEOL), and polished with water-resistant abrasive paper # 600. Thereafter, a tape (thickness 200 μm) with a hole having a diameter of 4 mm was affixed to the adherend surface to prescribe an adhesion area. The adherend surface of the stainless rod (φ4.5 mm) was sandblasted (0.2 MPa, 1 second) with alumina (50 μm), then was washed with water and dried, and applied with a metal adhesive primer (METAL LINK, manufactured by SHOFU INC.). The first paste and the second paste which correspond the dental adhesive composition of the Example or Comparative example were sufficiently kneaded in equal mass and was applied to the adherend surface of the stainless rod in an appropriate amount, and the resin block and the stainless rod were bonded so as to fit in the frame of the tape with a hole. A load of 200 N was applied from the vertical direction of the stainless rod, and excess cement was wiped off with a cloth. Thereafter, Light irradiation was performed for 10 seconds with the dental polymerization LED light curing unit (PEN Bright, manufactured by SHOFU INC.). After removing the load and immersing in water at 37 °C for 24 hours, immersing alternately in cold water phase at 4 °C for 60 seconds and in high temperature phase at 60 °C for 60 seconds was repeated 5000 times by using a thermal shock tester (manufactured by THOMAS KAGAKU Co.,Ltd). The specimen was taken out and a universal tester (manufactured by Instron) was used to measure the tensile adhesive strength at a crosshead speed of 1 mm/min. When the adhesive strength to the resin block was 15 MPa or more, it was determined to have good adhesive strength. On the other hand, when the adhesive strength to the resin block was less than 10 MPa, it was determined that the adhesive strength was insufficient.

[Adhesive strength to dental resin for cutting and machining made of glass fiber reinforced resin (hereinafter referred to as GF reinforced resin)]

The GF reinforced resin (trilina, manufactured by Bicon) was processed into a plate having a thickness of 3 mm using an isomet (manufactured by JEOL), and polished with water-resistant abrasive paper # 600. At this time, the direction in which the mesh of the glass fibers can be seen on the adherend surface (mesh adherence surface) and the direction in which the laminated surface of the glass fibers can be seen (laminated adherend surface) were distinguished in machining. Subsequently, a tape (thickness 200 μm) with a hole having a diameter of 4 mm was affixed to the adherend surface to prescribe an adhesion area. The adherend surface of the stainless rod (φ4.5 mm) was sandblasted (0.2 MPa, 1 second) with alumina (50 μm), then was washed with water and dried, and applied with a metal adhesive primer (METAL LINK, manufactured by SHOFU INC.). The first paste and the second paste which correspond the dental

adhesive composition of the Example or Comparative example were sufficiently kneaded in equal mass and was applied to the adherend surface of the stainless rod in an appropriate amount, and the GF reinforced resin and the stainless rod were bonded so as to fit in the frame of the tape with a hole. A load of 200 N was applied from the vertical direction of the stainless rod, and excess cement was wiped off with a cloth. Thereafter, Light irradiation was performed for 10 seconds with the dental polymerization LED light curing unit (PEN Bright, manufactured by SHOFU INC.). After removing the load and immersing in water at 37 °C for 24 hours, immersing alternately in cold water phase at 4 °C for 60 seconds and in high temperature phase at 60 °C for 60 seconds was repeated 5000 times by using a thermal shock tester (manufactured by THOMAS KAGAKU Co.,Ltd). The specimen was taken out and a universal tester (manufactured by Instron) was used to measure the tensile adhesive strength at a crosshead speed of 1 mm/min. When the adhesive strength to the GF reinforced resin was 15 MPa or more, it was determined to have good adhesive strength. On the other hand, when the adhesive strength to the GF reinforced resin was less than 10 MPa, it was determined that the sufficient adhesive strength was not exhibited.

(Color resistance property)

The first paste and the second paste were sufficiently kneaded in equal mass, and the prepared paste was collected in a mold with a thickness of 1.0 mm and an inner diameter of 15 mm, with being careful not to allow air bubbles to enter. The both sides were pressed with cover glasses and light irradiation was performed on the both surface for 60 seconds with the dental polymerization LED light irradiator (PEN Bright, manufactured by SHOFU INC.) to prepare a disk-shaped cured product having a thickness of 1.0 mm. The cured product was polished with water-resistant abrasive paper #600, then with water-resistant abrasive paper #1200, and then with water-resistant abrasive paper #2000 to adjust the surface to be smooth. Subsequently, the cured product was immersed in a 10 mL plastic container containing 5 mL of distilled water at 37 °C for 1 day. The cured product was taken out, and after sufficiently wiping off the water, the color was measured using a spectrophotometer (CM-26d: manufactured by Konica Minolta, Inc.) under the conditions of SCE and a white background. Then, the cured product was immersed in a 10 mL plastic container containing 5 mL of 0.1% Rhodamine aqueous solution at 37 °C for 1 day, and the cured product was taken out and was washed twice with distilled water to sufficiently wipe off water from the cured product. Thereafter, the color was measured using a spectrophotometer (CM-26d: manufactured Konica Minolta, Inc.) under the conditions of SCE and a white background. The color difference $\Delta E$ was calculated from the results measured before and after immersion in 0.1% Rhodamine aqueous solution. Evaluation criteria were as follows.

A: $\Delta E$ was less than 30
B: $\Delta E$ was 30 to 45
C: $\Delta E$ was more than 45

When the evaluation was C, it was judged that the resistance property was inferior.

(Paste property)

The first paste and the second paste were sufficiently kneaded in equal mass, and 0.1 g of the prepared paste was allowed to stand on a slide glass and the position where the paste was left to stand was marked using a seal. Then, the slide glass was tilted at 90°, and after 20 seconds, the slide glass was returned to its original position, and the distance the paste moved was measured. Evaluation criteria for the increase/decrease in the moving distance of the paste between the paste immediately after preparation and the paste after storage at 40 °C for 3 months were as follows.

A: less than 10%
B: 10 to 40 %
C: more than 40%

When the evaluation was C, it was judged that the characteristic stability was inferior.

[Table 9]

| | Paste | Matrix | | | | | | | | | | | | | | | | | | | | | | | | | | | |
|---|---|---|---|---|---|---|---|---|---|---|---|---|---|---|---|---|---|---|---|---|---|---|---|---|---|---|---|---|---|
| | | Silane coupling material | | | | | | (C) Polymerizable monomer having no acidic group | | | | | | | | | Polymerizable monomer having acidic group | | | Chemical polymerization initiator | | Photo polymerization initiator | Polymerization accelerator | | | | | | Polymerization inhibitor |
| | | (A1) Silane coupling material represented by structural formula of [Chemical formula 1] | | | | (A) other than (A1) | | Having no hydroxyl group | | | | (C1) Having hydroxyl group | | | | | | | | | | | | | | | | | |
| | | | | | | | | | | | | Other than (C11) | | (C11) low viscosity | | | | | | | | | | | | | | | |
| | | MPTMS | APTMS | C11A | C11-EG | AAPTMS | MAPTES | D2.6E | UDMA | 3G | NPG | BisGMA | PENTA | HPPA | GDMA | HEMA | MDP | MHPA | META | CHP | BPO | CQ | DMBE | DEPT | PTU | BTU | VOA | COA | BHT |
| Example 101 | First | 5 | | | | | | | 15 | | 30 | 49.9 | | | 0.1 | | | | | | | 0.3 | | | 2 | | | 0.01 | 0.1 |
| | Second | | | | | | | | 20 | | | 50 | | | 20 | | 5 | | 5 | 3 | | | 0.3 | | | | | | 0.2 |
| Example 102 | First | | 7 | | | | | | 53 | | | | | 20 | 20 | | | | | 3 | | | 0.3 | | | | | | 0.03 |
| | Second | | | | | | | | | 40 | | 50 | | | | | 10 | | | | | 0.2 | | | | 2 | | | 0.15 |
| Example 103 | First | | | 15 | | | | 35 | | 30 | | 20 | | | | | | | | 3 | | | 0.3 | | | | | | 0.1 |
| | Second | | | | | | | | 20 | | | 40 | | 10 | 20 | | 5 | | 5 | | | 0.2 | | | | 1 | | 0.05 | 0.2 |
| Example 104 | First | | | | 18 | | | 52 | | | | | | 20 | | 10 | | | | 3 | | | 0.3 | | | | | | 0.1 |
| | Second | | | | | | | | | | | 30 | 20 | | 20 | 20 | | 5 | 5 | | | 0.2 | | | | 1 | | 0.05 | 0.2 |
| Example 105 | First | 2 | 5 | | | | | | 15 | | | 28 | 20 | | 30 | | | | | 3 | | | 0.3 | | | | | | 0.03 |
| | Second | | | | | | | | | 40 | | 40 | | | | 10 | 10 | | | | | 0.2 | | | 1 | | 0.01 | | 0.15 |
| Example 106 | First | 3 | | 10 | | | | 47 | | | | 10 | | | 10 | 20 | | | | 3 | | | 0.3 | | | | | | 0.1 |
| | Second | | | | | | | | 25 | | | 45 | | | 20 | | 5 | | 5 | | | 0.2 | | | | 1 | | 0.05 | 0.2 |
| Example 107 | First | 3 | | | 8 | | | 57 | | | | | | 12 | 20 | | | | | 3 | | | 0.3 | | | | | | 0.1 |
| | Second | | | | | | | | 65 | | 25 | | | | | | 5 | | 5 | | | 0.2 | | | 1 | | | 0.05 | 0.2 |
| Example 108 | First | | | 10 | | | | 50 | | 25 | | | | | 15 | | | | | | | 0.3 | | 0.9 | 1 | | 0.03 | | 0.1 |
| | Second | | | | | | | | 25 | | | 30 | | | 20 | | 15 | | 10 | | 1.5 | | 0.3 | | | | | 0.01 | 0.2 |
| Example 109 | First | | | | 20 | | | 50 | | 25.1 | | | | | 4.9 | | | | | | | 0.3 | | | 0.5 | | | 0.1 | 0.1 |
| | Second | | | | | | | | 20 | | | 50 | | | 20 | | 5 | | 5 | 3 | | | 0.3 | | | | | | 0.2 |
| Example 110 | First | | | | 20 | | | 45 | | 15 | | | | | 20 | | | | | | | 0.3 | | | | 1 | 0.03 | | 0.1 |
| | Second | | | | | | | | 20 | | | 50 | | | 20 | | 5 | | 5 | 3 | | | 0.3 | | | | | | 0.2 |
| Example 111 | First | 1 | | | | | | 45 | 19 | | | 15 | | | 20 | | | | | | | 0.3 | | | | 0.5 | | 0.1 | 0.1 |
| | Second | | | | | | | | 25 | | | 45 | | | 20 | | 5 | | 5 | 2.5 | | | 0.3 | | | | | | 0.2 |
| Example 112 | First | | | 18 | | | | 50 | | 20 | | | | | | 12 | | | | | | 0.3 | | 1 | | 0.8 | 0.05 | | 0.1 |
| | Second | | | | | | | 20 | 20 | | | 30 | | | 20 | | | 10 | | | 1.5 | | 0.3 | | | | | | 0.2 |
| Example 113 | First | 2 | | | | | | | 13 | 10 | | 20 | | | 55 | | | | | 3 | | | 0.3 | | | | | | 0.1 |
| | Second | | | | | | | 40 | 20 | 20 | | 10 | | | | | 5 | | 5 | | | 0.2 | | | 1 | | | 0.01 | 0.05 |
| Example 114 | First | 8 | | | | | | | 10 | | | 62 | | | 20 | | | | | 3 | | | 0.3 | | | | | | 0.1 |
| | Second | | | | | | | | 15 | | | 55 | | | 20 | | | 10 | | | | 0.2 | | | 1.5 | | 0.03 | | 0.05 |
| Example 115 | First | 8 | | | | | | | 15 | | | 57 | | | | 20 | | | | 2.4 | | | 0.3 | | | | | | 0.1 |
| | Second | | | | | | | | 20 | | | 50 | | | 20 | | | 10 | | | | 0.2 | | | 1.5 | | 0.03 | | 0 |
| Example 116 | First | | | | 22 | | | | 15 | 10 | | 43 | | | | 10 | | | | 2 | | | 0.3 | | | | | | 0.1 |
| | Second | | | | | | | | 25 | 15 | | 50 | | | | | | 10 | | | | 0.2 | | | 1 | | 0.03 | | 0 |
| Example 117 | First | 5 | | | | | | 60 | | 29 | 5 | | | | 1 | | | | | 3 | | | 0.3 | | | | | | 0.1 |
| | Second | | | | | | | | 30 | | | 60 | | | | | 5 | | 5 | | | 0.2 | | | | 1 | | 0.05 | 0.2 |
| Example 118 | First | 6 | | | | | | 44 | | | | | | | 50 | | | | | 3 | | | 0.3 | | | | | | 0.1 |
| | Second | | | | | | | | | | | 40 | | | | 50 | 5 | | 5 | | | 0.2 | | | | 1 | | 0.05 | 0.2 |
| Example 119 | First | 6 | | | | | | 44 | | | | | | | 50 | | | | | 3 | | | 0.3 | | | | | | 0.1 |
| | Second | | | | | | | 40 | | | | | | 50 | | | 5 | | 5 | | | 0.2 | | | | 1 | | 0.05 | 0.2 |
| Example 120 | First | 7 | | | | | | 18 | 15 | 5 | | 40 | | | 20 | | | | | | | 0.3 | | | 2 | | | 0.01 | 0.1 |
| | Second | | | | | | | 56 | 21.5 | | | | | | 22 | | 0.5 | | | 3 | | | 0.3 | | | | | | 0.2 |
| Example 121 | First | 7 | | | | | | 48 | 15 | | | | | | 30 | | | | | | | 0.3 | | | 2 | | | 0.01 | 0.1 |
| | Second | | | | | | | | 10 | 8 | | 20 | | | 22 | | 40 | | | 3 | | | 0.3 | | | | | | 0.2 |
| Example 122 | First | 7 | | | | | | 35 | 15 | 3 | | 30 | | | 10 | | | | | | | 0.3 | | | 2 | | | 0.01 | 0.1 |
| | Second | | | | | | | | 30 | 20 | | 30 | | | 10 | | 10 | | | 3 | | | 0.3 | | | | | | 0.2 |
| Example 123 | First | 7 | | | | | | 35 | 18 | | | 30 | | | 10 | | | | | 3 | | 0.3 | | | | | | | 0.1 |
| | Second | | | | | | | | 30 | 20 | | 30 | | | 10 | | 10 | | | | | | 0.3 | | 2 | | | 0.01 | 0.2 |
| Example 124 | First | | | 10 | | | | 35 | 15 | | | 30 | | | 10 | | | | | 3 | | 0.3 | | | | | | | 0.1 |
| | Second | | | | | | | | 30 | 20 | | 30 | | | 10 | | 10 | | | | | | 0.3 | | 2 | | | 0.01 | 0.2 |
| Example 125 | First | 7 | | | | 3 | | 68 | | | | | | 20 | | 10 | | | | 3 | | | 0.3 | | | | | | 0.1 |
| | Second | | | | | | | | | | | 30 | 20 | | 20 | 20 | | 5 | 5 | | | 0.2 | | | | 1 | | 0.05 | 0.2 |
| Example 126 | First | 7 | | | | | 3 | 68 | | | | | | 20 | | 10 | | | | 3 | | | 0.3 | | | | | | 0.1 |
| | Second | | | | | | | | | | | 30 | 20 | | 20 | 20 | | 5 | 5 | | | 0.2 | | | | 1 | | 0.05 | 0.2 |

[Table 10]

| | Paste | Filler | | | | | | | Compounding amount (C11) with respect to 100 parts by mass of total amount of first matrix and second matrix | Total amount of silane coupling material compounding amount index in matrix | Compounding amount (C11) with respect to 100 parts by mass of first matrix | Durable adhesive strength | | | | | | | | Discoloration resistance property | Paste property |
|---|---|---|---|---|---|---|---|---|---|---|---|---|---|---|---|---|---|---|---|---|---|
| | | Without surface treatment | | Surace treated | | | | | | | | Initial preparation product | Acceleration test product | Initial preparation product | Acceleration test product | Initial preparation product | Acceleration test product | Initial preparation product | Acceleration test product | | |
| | | Filler A | Filler B | Filler D | Filler E | Filler F | Filler G | Filler H | | | | Resin block | | GF reinforced resin (mesh adherence surface) | | GF reinforced resin (laminated adherend) | | Tooth substance | | | |
| Example 101 | First | | | | 200 | | | | 58 | 0.029 | 0.1 | 18.8 | 17.8 | 11.7 | 11.5 | 11.2 | 10.4 | [illegible] | 12.7 | A | A |
| | Second | | | | 200 | | | | | | | | | | | | | | | | |
| Example 102 | First | | 40 | | | 160 | | | 44 | 0.044 | 38.7 | 18.9 | 14.5 | 18.0 | 13.0 | 17.6 | 15.7 | 11.4 | 11.8 | A | B |
| | Second | | | 200 | | | | | | | | | | | | | | | | | |
| Example 103 | First | | | | 150 | 50 | | | 44 | 0.046 | 0.0 | 18.2 | 18.2 | 10.6 | 11.8 | 11.7 | 10.2 | 14.8 | [illegible] | A | A |
| | Second | | | 200 | | | | | | | | | | | | | | | | | |
| Example 104 | First | | | | 150 | 50 | | | 59 | 0.049 | 28.0 | 19.2 | 18.9 | 17.9 | 17.1 | 19.0 | 19.5 | 15.5 | 10.4 | A | A |
| | Second | | | 200 | | | | | | | | | | | | | | | | | |
| Example 105 | First | | | | 150 | 50 | | | 63 | 0.043 | 25.0 | 18.3 | 17.6 | 18.7 | 17.8 | 17.9 | 19.8 | 11.5 | 12.8 | A | A |
| | Second | | | 180 | | | | | | | | | | | | | | | | | |
| Example 106 | First | | | | 120 | 50 | | | 51 | 0.048 | 25.0 | 17.9 | 19.2 | 19.6 | 17.7 | 17.0 | 17.8 | 14.2 | 11.4 | A | A |
| | Second | | | 200 | | | | | | | | | | | | | | | | | |
| Example 107 | First | | | | 150 | 50 | | | 16 | 0.040 | 30.9 | 14.9 | 13.1 | 15.7 | 13.6 | 15.5 | 15.9 | 11.0 | 11.2 | A | A |
| | Second | | | 160 | | | | | | | | | | | | | | | | | |
| Example 108 | First | | | | 200 | | | | 52 | 0.031 | 14.7 | 18.0 | 18.8 | 18.7 | 19.5 | 18.3 | 19.7 | 15.6 | 12.3 | A | A |
| | Second | | | | | | | 200 | | | | | | | | | | | | | |
| Example 109 | First | | | | 200 | | | | 57 | 0.055 | 4.9 | 19.3 | 19.9 | 18.8 | 15.8 | 15.0 | 15.8 | 11.5 | 12.8 | A | A |
| | Second | | | | | | 200 | | | | | | | | | | | | | | |
| Example 110 | First | | 10 | | | 120 | | | 44 | 0.055 | 19.7 | 18.3 | 18.5 | 17.7 | 19.7 | 17.1 | 18.0 | 15.0 | 11.6 | A | A |
| | Second | | | | | | | 130 | | | | | | | | | | | | | |
| Example 111 | First | | | | 170 | | | | 49 | 0.006 | 19.8 | 13.9 | 15.6 | 14.7 | 14.2 | 15.3 | 15.6 | 13.7 | [illegible] | A | A |
| | Second | | | 170 | | | | | | | | | | | | | | | | | |
| Example 112 | First | | | | 200 | | | | 50 | 0.056 | 11.7 | 19.2 | 13.6 | 19.3 | 14.7 | 17.9 | 14.6 | 11.9 | 10.5 | B | A |
| | Second | | | | | | 200 | | | | | | | | | | | | | | |
| Example 113 | First | | | | | | 200 | | 42 | 0.012 | 53.2 | 18.9 | 10.4 | 19.3 | 10.8 | 17.5 | [illegible] | 10.5 | 11.3 | B | A |
| | Second | | | 200 | | | | | | | | | | | | | | | | | |
| Example 114 | First | | | | | | 200 | | 77 | 0.047 | 19.3 | 19.9 | 14.8 | 18.9 | 14.7 | 17.5 | 14.0 | 11.2 | 8.2 | A | A |
| | Second | | | 200 | | | | | | | | | | | | | | | | | |
| Example 115 | First | | | | 200 | | | | 72 | 0.047 | 19.5 | 19.0 | 13.3 | 19.9 | 14.0 | 19.9 | 14.0 | 14.0 | 7.7 | A | A |
| | Second | | | 200 | | | | | | | | | | | | | | | | | |
| Example 116 | First | | | | | | | 200 | 51 | 0.061 | 9.8 | 17.1 | 13.7 | 18.9 | 13.7 | 17.2 | 14.9 | 11.3 | [illegible] | B | A |
| | Second | | | 200 | | | | | | | | | | | | | | | | | |
| Example 117 | First | | | | 150 | 50 | | | 30 | 0.029 | [illegible] | 18.5 | 19.8 | 16.0 | 14.6 | 15.3 | 14.0 | 10.5 | 11.2 | A | A |
| | Second | | | 200 | | | | | | | | | | | | | | | | | |
| Example 118 | First | | | | 150 | 50 | | | 68 | 0.035 | 48.4 | 17.8 | 14.8 | 17.0 | 13.0 | 17.5 | 14.3 | 15.5 | [illegible] | A | A |
| | Second | | | 200 | | | | | | | | | | | | | | | | | |
| Example 119 | First | | | | 150 | 50 | | | 40 | 0.035 | 48.4 | 18.6 | 13.7 | 19.7 | 13.0 | 18.6 | 15.3 | [illegible] | 15.0 | A | A |
| | Second | | | 200 | | | | | | | | | | | | | | | | | |
| Example 120 | First | | | | 200 | | | | 41 | 0.041 | 20.0 | 17.5 | 17.5 | 18.1 | 18.0 | 19.3 | 20.0 | 6.8 | [illegible] | A | A |
| | Second | | | 200 | | | | | | | | | | | | | | | | | |
| Example 121 | First | | | | 200 | | | | 56 | 0.041 | 30.0 | 17.4 | 14.2 | 20.0 | 14.0 | 18.5 | 15.4 | 14.8 | 6.2 | A | A |
| | Second | | | 200 | | | | | | | | | | | | | | | | | |
| Example 122 | First | 20 | | 180 | | | | | 59 | 0.041 | 9.8 | 17.6 | 10.9 | 18.9 | 10.6 | 17.9 | [illegible] | 11.6 | 10.4 | A | B |
| | Second | | | 200 | | | | | | | | | | | | | | | | | |
| Example 123 | First | | 20 | | 180 | | | | 59 | 0.041 | 9.7 | 19.8 | 10.6 | 19.0 | 10.1 | 17.7 | 10.5 | [illegible] | 12.4 | A | B |
| | Second | | | 200 | | | | | | | | | | | | | | | | | |
| Example 124 | First | 20 | | | | 180 | | | 59 | 0.031 | 9.7 | 18.5 | 10.1 | 17.1 | 11.0 | 19.1 | 10.3 | 15.5 | 11.7 | A | B |
| | Second | | | 200 | | | | | | | | | | | | | | | | | |
| Example 125 | First | | | | 150 | 50 | | | 56 | 0.058 | 26.9 | 18.4 | 19.0 | 19.9 | 20.0 | 18.4 | 19.0 | 14.7 | 11.4 | A | A |
| | Second | | | 200 | | | | | | | | | | | | | | | | | |
| Example 126 | First | | | | 150 | 50 | | | 56 | 0.054 | 26.9 | 18.9 | 18.6 | 19.5 | 18.3 | 18.4 | 17.2 | 11.2 | [illegible] | A | A |
| | Second | | | 200 | | | | | | | | | | | | | | | | | |

[Table 11]

| | Paste | Matrix | | | | | | | | | | | | | | | | | | | | | | | | | | | |
|---|---|---|---|---|---|---|---|---|---|---|---|---|---|---|---|---|---|---|---|---|---|---|---|---|---|---|---|---|---|
| | | Silane coupling material | | | | | | (C) Polymerizable monomer having no acidic group | | | | | | | | | Polymerizable monomer having acidic group | | | Chemical polymerization initiator | | Photo polymerization initiator | Polymerization accelerator | | | | | | Polymerization inhibitor |
| | | (A1) Silane coupling material represented by structural formula of [Chemical formula 1] | | | | (A) other than (A1) | | Having no hydroxyl group | | | | (C11) Having hydroxyl group | | | | | | | | | | | | | | | | | |
| | | | | | | | | | | | | Other than (C11) | | (C11) low viscosity | | | | | | | | | | | | | | | |
| | | MPTMS | APTMS | C11A | C11-EG | AAPTMS | MAPTES | D2.6E | UDMA | 3G | NPG | BisGMA | PENTA | HPPA | GDMA | HEMA | MDP | MHPA | META | CHP | BPO | CQ | DMBE | DEPT | PTU | BTU | VOA | COA | BHT |
| Comparative Example | First | | | | | | | | 15 | | | 65 | | | 20 | | | | | | | 0.3 | | | | 0.5 | | 0.1 | 0.1 |
| | Second | | | | | | | 25 | 15 | | | 30 | | | 20 | | 5 | | 5 | 2.5 | | | 0.3 | | | | | | 0.2 |
| Comparative Example | First | | | | | | | 50 | | | | 35 | | | | 15 | | | | | | 0.3 | | 1 | | | | | 0.1 |
| | Second | | | | | | | 20 | 20 | | | 30 | | | 20 | | | 10 | | | 1.5 | | 0.3 | | | | | | 0.2 |
| Comparative Example | First | 7 | | | | | | | 25 | 5 | | 43 | | | 20 | | | | | | | 0.3 | | | 2 | | | 0.01 | 0.1 |
| | Second | | | | | | | 50 | 30 | | | | | | 20 | | | | | 3 | | | 0.3 | | | | | | 0.2 |
| Comparative Example | First | 7 | | | | | | 50 | 13 | | | | | | | 30 | | | | | | 0.3 | | | 2 | | | 0.01 | 0.1 |
| | Second | | | | | | | | 50 | | | 30 | | | | 20 | | | | 3 | | | 0.3 | | | | | | 0.2 |
| Comparative Example | First | 20 | | | | | | 80 | | | | | | | | | | | | | | 0.3 | | | 0.8 | | 0.3 | | 0.1 |
| | Second | | | | | | | | 63 | | | | | | 25 | | 7 | | 5 | 3 | | | 0.3 | | | | | | 0.2 |
| Comparative Example | First | | | | | 10 | | 68 | | | | | | 20 | | 10 | | | | 3 | | | 0.3 | | | | | | 0.1 |
| | Second | | | | | | | | | | | 30 | 20 | | 20 | 20 | | 5 | 5 | | | 0.2 | | | | 1 | | 0.05 | 0.2 |
| Comparative Example | First | | | | | | 10 | 68 | | | | | | 20 | | 10 | | | | 3 | | | 0.3 | | | | | | 0.1 |
| | Second | | | | | | | | | | | 30 | 20 | | 20 | 20 | | 5 | 5 | | | 0.2 | | | | 1 | | 0.05 | 0.2 |

[Table 12]

| | Paste | Filler | | | | | | | Compounding amount (C11) with respect to 100 parts by mass of total amount of first matrix and second matrix | Total amount of silane coupling material compounding amount index in matrix | Compounding amount (C11) with respect to 100 parts by mass of first matrix | Durable adhesive strength | | | | | | | | Discoloration resistance property | Paste property |
|---|---|---|---|---|---|---|---|---|---|---|---|---|---|---|---|---|---|---|---|---|---|
| | | Without surface treatment | | Surace treated | | | | | | | | Initial preparation product | Acceleration test roduct | Initial preparation product | Acceleration test roduct | Initial preparation product | Acceleration test roduct | Initial preparation product | Acceleration test roduct | | |
| | | Filler A | Filler B | Filler D | Filler E | Filler F | Filler G | Filler H | | | | Resin block | | GF reinforced resin (mesh adherence surface) | | GF reinforced resin (laminated adherend surface) | | Tooth substance | | | |
| Comparative Example | First | | | | | | 200 | | 66 | 0.000 | 20 | 3.3 | 3.5 | 4.2 | 3.7 | 4.6 | 3.8 | 15.0 | 12.2 | A | B |
| | Second | | | | | 200 | | | | | | | | | | | | | | | |
| Comparative Example | First | | | | | | 200 | | 49 | 0.000 | 15 | 3.1 | 3.7 | 2.2 | 3.4 | 4.0 | 3.4 | 12.6 | 10.6 | A | B |
| | Second | | | | | 200 | | | | | | | | | | | | | | | |
| Comparative Example | First | | | | 200 | | | | 40 | 0.041 | 20 | 13.2 | 14.6 | 13.6 | 14.9 | 13.9 | 14.4 | 0.7 | 0.5 | A | B |
| | Second | | | 200 | | | | | | | | | | | | | | | | | |
| Comparative Example | First | | | | 200 | | | | 39 | 0.041 | 29 | 14.6 | 14.2 | 14.5 | 13.8 | 13.6 | 13.4 | 0.8 | 0.8 | A | B |
| | Second | | | 200 | | | | | | | | | | | | | | | | | |
| Comparative Example | First | 200 | | | | | | | 12 | 0.118 | 0 | 11.6 | 4.9 | 3.3 | 4.0 | 2.4 | 2.9 | 7.0 | 5.4 | C | C |
| | Second | | | | | | 200 | | | | | | | | | | | | | | |
| Comparative Example | First | | | | 150 | 50 | | | 56 | 0.060 | 26.9 | 4.7 | 3.7 | 3.6 | 2.8 | 2.3 | 4.0 | 16.7 | 15.8 | A | A |
| | Second | | | 200 | | | | | | | | | | | | | | | | | |
| Comparative Example | First | | | | 150 | 50 | | | 56 | 0.049 | 26.9 | 2.2 | 4.0 | 4.2 | 4.9 | 4.1 | 2.6 | 16.2 | 16.1 | A | A |
| | Second | | | 200 | | | | | | | | | | | | | | | | | |

It was confirmed that Examples 101 to 126 showed good adhesive strength to the tooth substance, the resin block, and the GF reinforced resin, showed good adhesive strength even after the acceleration test at 40 °C for 3 months, and had good storage stability.

In Example 107, since the compounding amount of the polymerizable monomer having one or more hydroxyl groups contained in the entire matrix was less than 20% by mass, there was a case the adhesive strength to the resin block and the GF reinforced resin is 15 MPa or less and there was a case where the adhesive strength was a little low.

In Examples 114 and 115, since the compounding amount of the polymerizable monomer having one or more hydroxyl groups contained in the entire matrix exceeded 70% by mass, there was a case where the adhesive strength to the resin block and the GF reinforced resin after the acceleration test was 15 MPa or less, and there was a case where the adhesive strength was slightly low.

In Example 111 in which the silane coupling material compounding amount index in matrix was in the vicinity of the lower limit of the formula (1), there was a case where the adhesive strength to the resin block and the GF reinforced resin was 15 MPa or less and the adhesive strength was a little low.

In Examples 112 and 116 in which the silane coupling material compounding amount index in matrix was in the vicinity of the upper limit of the formula (1), there was a case where the adhesive strength of the acceleration test product to the resin block and the GF reinforced resin was 15 MPa or less and the adhesive strength was a little low.

In the Examples 101, 103, 109 and 120 in which the compounding amount of the polymerizable monomer having no acidic group, having one or more hydroxyl groups, and having a viscosity of 200 mPa ·s or less at 25 °C contained in the first matrix containing a silane coupling material was less than 5% by mass, the adhesive strength to the GF reinforced resin was low. It was confirmed that in the Examples 101 and 103 in which the compounding amount of the polymerizable monomer having no acidic group, having one or more hydroxyl groups, and having a viscosity of 200 mPa ·s or less at 25 °C contained in the first matrix was 0.1% by mass or less, it was confirmed that the adhesive strength was particularly low, but the adhesive strength was 10 MPa or more.

In the Examples 102, 113, 118 and 119 in which the compounding amount of the polymerizable monomer having no acidic group, having one or more hydroxyl groups, and having a viscosity of 200 mPa ·s or less at 25 °C contained in the first matrix containing a silane coupling material exceeded 30%, there was a case where the adhesive strength of the acceleration test product to the resin block and the GF reinforced resin was 15 MPa or less, the adhesive strength was slightly low, and there was concern in the storage stability. In particular, in Example 113 in which the compounding amount of the polymerizable monomer having no acidic group, having one or more hydroxyl groups, and having a viscosity of 200 mPa ·s or less at 25 °C contained in the first matrix containing a silane coupling material exceeded 50%, the adhesive strength of the acceleration test product to the resin block and the GF reinforced resin was low.

In Examples 122, 123 and 124 which consist of a first paste containing the first matrix containing a silane coupling material and the filler A or the filler B which were not surface-treated, the adhesive strength of the acceleration test product to the resin block and the GF reinforced resin was 15 MPa or less, the adhesive strength was slightly low,

and there was a concern about storage stability.

In Example 120 in which the compounding amount of the polymerizable monomer having an acidic group was small, there was a tendency that adhesive strength to the tooth substance was low. In Example 121 in which the compounding amount of the polymerizable monomer having an acidic group was large, there was a tendency that the adhesive strength of the acceleration test product to the tooth substance decreased.

Since Comparative Examples C101 and C102 did not contain a silane coupling material, the adhesive strength to the resin block and the GF reinforced resin was remarkably low.

In Comparative Example C105, since the total amount of the silane coupling material compounding amount index in matrix exceeded the upper limit in the formula 1, the discoloration resistance is poor, and since it contained the filler A which was not surface-treated, the stability of the paste property was also poor.

In Comparative Example C103 and Comparative Example C104 not containing a polymerizable monomer having an acidic group, the adhesive strength to the tooth substance was significantly low.

Comparative Examples C106 and C107 not containing the (A1) silane coupling material represented by structural formula of [Chemical formula 1] had remarkably low adhesive strength to the resin block and the GF reinforced resin.

[Dental adhesive composition containing (A) silane coupling material, (B) polymerizable monomer having an acidic group, (C) polymerizable monomer having no acidic group, (H) volatile organic solvent and (G) water, and one or both of the (D) polymerization initiator and the (E) polymerization accelerator]

(Preparing method of paste)

The polymerizable monomers, polymerization inhibitor, and volatile organic solvent shown in Examples and Comparative Examples were put into a light-shielding plastic container and mixed by using a mix rotor VMRC-5 under the condition of 100 rpm for 48 hours. Thereafter, all components described in the table except the premixed components were added and further mixed by using a mix rotor VMRC-5 under the condition of 100 rpm for 48 hours to prepare a composition.

The test method of each characteristic evaluated in the example and the comparative example is as follows.

(Method for preparing compositions described in Examples and Comparative Examples)

The polymerizable monomers, polymerization inhibitor and volatile organic solvent shown in Examples and Comparative Examples were put into a light-shielding plastic container and mixed by using a mix rotor VMRC-5 under the condition of 100 rpm for 48 hours. Thereafter, all components described in the table except the premixed components were added and further mixed by using a mix rotor VMRC-5 under the condition of 100 rpm for 48 hours to prepare a composition.

(Storage stability of dental adhesive composition)

In a dark room where the room temperature was 23 ± 2 °C, 5 mL of each composition described in Examples and Comparative Examples was collected with a plastic dropper and filled in a polypropylene bottle. Thereafter, the nozzle and cap were attached in order, and it was confirmed that the composition was not leak even if it is turned upside down. The bottle filled with the composition was stored in an incubator at 50 °C for 3 months, and it was confirmed that no significant increase in viscosity or gelation occurred.

(Adhesive strength to glass ceramics containing lithium disilicate (hereinafter referred to as lithium disilicate))

The glass ceramics containing lithium disilicate (VINTAGE PRIME PRESS, color tone E-1, manufactured by SHOFU INC.) was fired under the conditions specified by the manufacturer to prepare an adherend (diameter: 15 mm, thickness: 3 mm). The surface of the adherend piece was polished with water-resistant abrasive paper # 600. Thereafter, the adherend surface of the adherend piece was sandblasted (0.2 MPa, 1 second) with alumina (50 μm), then was washed with water and dried. Thereafter, a double sided tape with a hole having a diameter of 4 mm was affixed to the adherend surface to prescribe an adhesion area. The one-pack type composition of each Example or Comparative Example was applied into the tape with the hole and immediately air dried. Light irradiation was performed for 5 seconds with the dental polymerization LED light curing unit (PEN Bright, manufactured by SHOFU INC.). On the other hand, the adherend surface of the stainless rod (φ4.5 mm) was sandblasted (0.2 MPa, 1 second) with alumina (50 μm), then was washed with water and dried, and applied with a metal adhesive primer (METAL LINK, manufactured by SHOFU INC.). The adherend surface of the stainless rod was applied with appropriate amount of resin cement (Resicem, manufactured by SHOFU INC.) and a resin block and the stainless rod were bonded so as to fit in the frame of the double-sided tape with a hole. The adherend surface of the stainless rod was applied with appropriate amount of resin cement (Resicem, manufactured by SHOFU INC.) and a resin block and the stainless rod were bonded so as to fit in the frame of the double-sided tape with a hole. A load of 200 N was applied from the vertical direction of the stainless rod, and excess cement was wiped off with a cloth. Thereafter, Light irradiation was performed for 10 seconds with the dental polymerization LED light curing unit (PEN Bright, manufactured by SHOFU INC.). After removing the load and immersing in water at 37 °C for 24 hours, immersing alternately in cold water phase at 4 °C for 60 seconds and in high temperature phase at 60 °C for 60 seconds was repeated 5000 times by using a thermal shock tester (manufactured by THOMAS KAGAKU Co.,Ltd). The specimen was taken out and a universal tester (manufactured by Instron) was used to measure the tensile adhesive strength at a crosshead speed of 1 mm/min. When the adhesive strength was 15 MPa or more, it was determined to have excellent adhesive strength. On the other hand, when the adhesive strength was less than 10 MPa, it was determined that the adhesive strength was low.

(Adhesive strength to dental resin for cutting and machining (hereinafter referred to as resin block))

The resin block (SHOFU BLOCK HC SUPER HARD manufactured by SHOFU INC.) was processed into a plate having a thickness of 3 mm using an isomet (manufactured by JEOL), and polished with water-resistant abrasive paper # 600. The adherend surface of the adherend piece was sandblasted (0.2 MPa, 1 second) with alumina (50 μm), then was washed with water and dried. Thereafter, a tape (thickness 200 μm) with a hole having a diameter of 4 mm was affixed to the adherend surface to prescribe an adhesion area. The one-pack type composition of each Example or Comparative Example was applied into the tape with the hole and immediately air dried. Light irradiation was performed for 5 seconds with the dental polymerization LED light curing unit (PEN Bright, manufactured by SHOFU INC.). On the other hand, the adherend surface of the stainless rod (φ4.5 mm) was sandblasted (0.2 MPa, 1 second) with alumina (50 μm), then was washed with water and dried, and applied with a metal adhesive primer (METAL LINK, manufactured by SHOFU INC.). The adherend surface of the stainless rod was applied with appropriate amount of resin cement (Resicem, manufactured by SHOFU INC.), and a resin block and the stainless rod were bonded so as to fit in the frame of the double-sided tape with a hole. A load of 200 N was applied from the vertical direction of the stainless rod, and excess cement was wiped off with a cloth. Thereafter, Light irradiation was performed for 10 seconds with the dental polymerization LED light curing unit (PEN Bright, manufactured by SHOFU INC.). After removing the load and immersing in water at 37 °C for 24 hours, immersing alternately in cold water phase at 4 °C for 60 seconds and in high temperature phase at 60 °C for 60 seconds was repeated 5000 times by using a thermal shock tester (manufactured by THOMAS KAGAKU Co.,Ltd). The specimen was taken out and a universal tester (manufactured by Instron) was used to measure the tensile adhesive strength at a crosshead speed of 1 mm/min. When the adhesive strength was 15 MPa or more, it was determined to have excellent adhesive strength. On the other hand, when the adhesive strength was less than 10 MPa, it was determined that the adhesive strength was low.

(Adhesive strength to dental resin for cutting and machining made of glass fiber reinforced resin (hereinafter referred to as GF reinforced resin))

The GF reinforced resin (Trilina, manufactured by Bicon) was processed into a plate having a thickness of 3 mm using an isomet (manufactured by JEOL), and polished with water-resistant abrasive paper # 600. At this time, the direction in which the mesh of the glass fibers can be seen on the adherend surface (mesh adherence surface) and the direction in which the laminated surface of the glass fibers can be seen (laminated adherend surface) were distinguished in machining. Subsequently, a tape (thickness 200 μm) with a hole having a diameter of 4 mm was affixed to the adherend surface to prescribe an adhesion area. The one pack type composition of each Example or Comparative Example was applied into the tape with the hole and immediately air dried. Light irradiation was performed for 5 seconds with the dental polymerization LED light curing unit (PEN Bright, manufactured by SHOFU INC.). On the other hand, the adherend surface of the stainless rod (φ4.5 mm) was sandblasted (0.2 MPa, 1 second) with alumina (50 μm), then was washed with water and dried, and applied with a metal adhesive primer (METAL LINK, manufactured by SHOFU INC.). The adherend surface of the stainless rod was applied with appropriate amount of resin cement (Resicem, manufactured by SHOFU INC.) and the GF reinforced resin and the stainless rod were bonded so as to fit in the frame of the double-sided tape with a hole. A load of 200 N was applied from the vertical direction of the stainless rod, and excess cement was wiped off with a cloth. Thereafter, Light irradiation was performed for 10 seconds with the dental polymerization LED light curing unit (PEN Bright, manufactured by SHOFU INC.). After removing the load and immersing in water at 37 °C for 24 hours, immersing alternately in cold water phase at 4 °C for 60 seconds and in high temperature phase at 60 °C for 60 seconds was repeated 5000 times by using a thermal shock tester (manufactured by THOMAS KAGAKU Co.,Ltd). The specimen was taken out and a universal tester (manufactured by Instron) was used to measure the tensile adhesive strength at a crosshead speed of 1 mm/min. When the adhesive strength was 15 MPa or more, it was determined to have excellent adhesive strength. On the other hand, when the adhesive strength was less than 10 MPa, it was

determined that the adhesive strength was low.

(Adhesive strength to tooth substance)

A test specimen of an epoxy resin embedded bovine central incisor was polished with water-resistant abrasive paper #600 to carve out a dentin plane. Thereafter, a tape (thickness 200 μm) with a hole having a diameter of 4 mm was affixed to the adherend surface to prescribe an adhesion area. The one pack type composition of each Example or Comparative Example was applied into the tape with the hole and immediately air dried. Light irradiation was performed for 5 seconds with the dental polymerization LED light curing unit (PEN Bright, manufactured by SHOFU INC.). On the other hand, the adherend surface of the stainless rod (φ4.5 mm) was sandblasted (0.2 MPa, 1 second) with alumina (50 μm), then was washed with water and dried, and applied with a metal adhesive primer (METAL LINK, manufactured by SHOFU INC.). The adherend surface of the stainless rod was applied with appropriate amount of resin cement (Resicem, manufactured by SHOFU INC.) and a resin block and the stainless rod were bonded so as to fit in the frame of the double-sided tape with a hole. A load of 200 N was applied from the vertical direction of the stainless rod, and excess cement was wiped off with a cloth. Thereafter, Light irradiation was performed for 10 seconds with the dental polymerization LED light curing unit (PEN Bright, manufactured by SHOFU INC.). After removing the load and immersing in water at 37 °C for 24 hours, immersing alternately in cold water phase at 4 °C for 60 seconds and in high temperature phase at 60 °C for 60 seconds was repeated 5000 times by using a thermal shock tester (manufactured by THOMAS KAGAKU Co.,Ltd). The specimen was taken out and a universal tester (manufactured by Instron) was used to measure the tensile adhesive strength at a crosshead speed of 1 mm/min. When the adhesive strength was 10 MPa or more, it was determined to have excellent adhesive strength. On the other hand, when the adhesive strength was less than 5 MPa, it was determined that the adhesive strength was low.

[Table 13]

| Composition | (A) Silane coupling material | | | | | | | | | | (B) Polymerizable monomer having an acidic group | | | | (C) Polymerizable monomer having no acidic group | | | | | | | | | (H) Having sulfur atom | (E) Polymerization initiator | Polymerization accelerator | | | | (F) Volatile Organic solvent | | (G) Water | Polymerization inhibitor |
|---|---|---|---|---|---|---|---|---|---|---|---|---|---|---|---|---|---|---|---|---|---|---|---|---|---|---|---|---|---|---|---|---|---|
| | (A') represented by structural | | | | | | | Other | | | | | | | Having no methacryloyl group and no methacrylamide group | | | Having methacryloyl group and/or methacrylamide group | | | | | | | Photo polymerization initiator | | | | | | | | |
| | Having methacryloyl group | | | | Having acryloyl group | | | | | | | | | | | | | Having no hydroxy group | | | Having one or more hydroxyl groups | | | | | | | | | | | | |
| | MPTMS | MOTMS | MPTES | C11EG | APTMS | C11A | C11DA | OTES | MTTSP | AAPTMS | MDP | MHPA | MET | META | A3.0E | EBAA | TMPTA | UDMA | 3G | MBAA | BisGMA | GDMA | HEMA | MDDT | CQ | DMBE | PTU | [illegible] | VOA | Acetone | EtOH | Water | BHT |
| Example 201 | 0.5 | | | | | | | | | | 5 | | | 5 | | | | | 10 | 10 | 35 | | | 0.5 | 0.3 | 0.3 | | | | 25 | | 25 | 0.3 |
| Example 202 | | 0.6 | | | | | | | | | 5 | | | 10 | | | | | 15 | | 35 | | | 0.5 | 0.3 | 0.3 | | | | 35 | | 25 | 0.2 |
| Example 203 | | | | 1 | | | | | | | 5 | | | | | | | | 15 | | 35 | | | 0.5 | 0.3 | 0.3 | | | | 25 | | 25 | 0.2 |
| Example 204 | | | | | 0.5 | | | | | | 5 | | | 5 | 5 | | | | 15 | | 35 | | | 0.5 | 0.3 | 0.3 | | | | 35 | | 25 | 0.5 |
| Example 205 | | | | | | 0.2 | | | | | | 5 | | 8 | | | | | 20 | | 35 | | | 0.5 | 0.3 | 0.3 | | | | 25 | | 25 | 0.2 |
| Example 206 | | | | | | | 1 | | | | | 5 | | 5 | | | | | 20 | | 35 | | | 0.5 | 0.3 | 0.3 | | | | | 35 | 25 | 0.2 |
| Example 207 | | | | | 0.8 | | | | | | 2 | | | 8 | 5 | | | | 10 | 5 | 35 | | | 0.5 | 0.3 | 0.3 | 0.3 | | | | 35 | 25 | 0.5 |
| Example 208 | | | | | | 1.5 | | | | | 2 | | | 8 | 5 | | | | 15 | | 35 | | | 0.5 | 0.3 | 0.3 | | 0.1 | | | 35 | 25 | 0.2 |
| Example 209 | | | | | | | 1.5 | | | | 2 | | | 8 | 5 | | | | 15 | | 35 | | | 0.5 | 0.3 | 0.3 | | | 0.3 | | 35 | 25 | 0.3 |
| Example 210 | 0.5 | | | | 0.5 | | | | | | | 3 | | 10 | | | | | | | 30 | 20 | | 0.5 | 0.3 | 0.3 | | | | 35 | | 25 | 0.5 |
| Example 211 | | | | 0.3 | | | | | | | 3 | | | | | | | | | | 30 | | 20 | 0.5 | 0.3 | 0.3 | | | | 25 | | 25 | 0.2 |
| Example 212 | 0.11 | | | | | | | | | | 3 | | | 10 | | | | | | 15 | 35 | | | 0.5 | 0.3 | 0.3 | | | | 25 | | 25 | 0.2 |
| Example 213 | | | | | 0.1 | | | | | | 3 | | | 10 | | | | | 15 | | 35 | | | 0.5 | 0.3 | 0.3 | | | | 35 | | 25 | 0.3 |
| Example 214 | 1.5 | | | | | | | | | | 3 | | | 10 | | | | 10 | 15 | | 25 | | | 0.5 | 0.3 | 0.3 | | | | | 35 | 25 | 0.3 |
| Example 215 | | | | | 1.4 | | | | | | 3 | | | 10 | | | | | 15 | | 35 | | | 0.5 | 0.3 | 0.3 | | | | 35 | | 25 | 0.3 |
| Example 216 | 1 | | | | | | | | | | 3 | | | 5 | | | | 15 | 20 | | 20 | | | 0.5 | 0.3 | 0.3 | | | | 25 | | 25 | 0.2 |
| Example 217 | | | | 1 | | | | | | | 2 | | 10 | | | | | 10 | 20 | | 20 | | | 0.5 | 0.3 | 0.3 | | | | 35 | | 25 | 0.3 |
| Example 218 | | | | | 1 | | | | | | 3 | | | 5 | 20 | 5 | 5 | | | | | 15 | 15 | 0.5 | 0.3 | 0.3 | | | | | 25 | 25 | 0.5 |
| Example 219 | | | | | | 2 | | | | | 4 | | | 5 | 25 | | 5 | | | | | 15 | 15 | 0.5 | 0.3 | 0.3 | | | | | 25 | 25 | 0.3 |
| Example 220 | | | | | | | 2 | | | | 2 | | | | 10 | | | | | | 20 | 15 | | 0.5 | 0.3 | 0.3 | | | | | 25 | 25 | 0.2 |
| Example 221 | | | | | | 1 | | | | | 3 | | | 5 | 20 | 5 | | | | | | 26 | | 0.5 | 0.3 | 0.3 | | | | 25 | | 20 | 0.3 |
| Example 222 | | | 1 | | | | | | | | 3 | | 5 | | | | | 15 | 5 | | 10 | 1 | | 0.5 | 0.3 | 0.3 | | | | 40 | | 25 | 0.3 |
| Example 223 | | | 1 | | | | | | | | 2 | | 12 | | | | | | 10 | | 20 | | | 0.5 | 0.3 | 0.3 | | | | 40 | | 25 | 0.3 |
| Example 224 | | | | 1 | | | | | | | 3 | | | 12 | | | | | 10 | | 20 | | | 0.5 | 0.3 | 0.3 | | | | 40 | | 25 | 0.5 |
| Example 225 | | | 1 | | | | | | | | 3 | | | 10 | | | 5 | 20 | | | | | 10 | 0.5 | 0.3 | 0.3 | | | | 20 | | 15 | 0.3 |
| Example 226 | 0.5 | | | | | | | | | | 3 | | | 5 | | 10 | | 20 | 10 | | | | 10 | 0.5 | 0.3 | 0.3 | | | | 35 | | 25 | 0.3 |
| Example 227 | | | | | 0.5 | | | | | | 3 | | | 5 | | | | | 10 | | 30 | | 10 | 0.5 | 0.3 | 0.3 | | | | 25 | | 25 | 0.2 |
| Example 228 | 0.5 | | | | | | | | | | 2 | | | 28 | | | | | | | 5 | | | 0.5 | 0.5 | 0.5 | | | | 30 | | 10 | 0.5 |
| Example 229 | | | | 1 | | | | | | | 3 | | | 10 | | | | | | | | 8 | 12 | 0.01 | 0.5 | 0.5 | | | | 5 | | 40 | 0.5 |
| Example 230 | | | | 1 | | | | | | | 3 | | | 12 | | | | | | | 5 | | | 0.5 | | | | | 0.01 | 50 | | 25 | 0.5 |
| Example 231 | | | 0.3 | | | | | | | | 2 | | 12 | | | | | | 10 | | 20 | | | | 0.3 | 0.3 | | | | | 40 | 25 | 0.5 |
| Comparative Example C201 | 0.05 | | | | | | | | | | 3 | | | 10 | | | | | 20 | | 40 | | | 0.5 | 0.3 | 0.3 | | | | 25 | | 25 | 0.2 |
| Comparative Example C202 | | | 0.05 | | | | | | | | 3 | | | 10 | | | | | 20 | | 40 | | | 0.5 | 0.3 | 0.3 | | | | 35 | | 25 | 0.2 |
| Comparative Example C203 | | 0.1 | | | | | | | | | 3 | | | 10 | | | | | 20 | | 40 | | | 0.5 | 0.3 | 0.3 | | | | 25 | | 25 | 0.2 |
| Comparative Example C204 | | | | | 0.08 | | | | | | 3 | | | 10 | | | | | 20 | | 40 | | | 0.5 | 0.3 | 0.3 | | | | 35 | | 25 | 0.2 |
| Comparative Example C205 | 2 | | | | | | | | | | 3 | | | 10 | | | | | 20 | | 40 | | | 0.5 | 0.3 | 0.3 | | | | 25 | | 25 | 0.2 |
| Comparative Example C206 | | | | | 2 | | | | | | 3 | | | 10 | | | | | 20 | | 40 | | | 0.5 | 0.3 | 0.3 | | | | 35 | | 25 | 0.2 |
| Comparative Example C207 | | 5 | | | | | | | | | 3 | | | 10 | | | | | 20 | | 40 | | | 0.5 | 0.3 | 0.3 | | | | 25 | | 25 | 0.2 |
| Comparative Example C208 | | | | | | | 5 | | | | 3 | | | 10 | | | | | 20 | | 40 | | | 0.5 | 0.3 | 0.3 | | | | 35 | | 25 | 0.2 |
| Comparative Example C209 | 1 | | | | | | | | | | | | | | | | | 15 | 18 | | 20 | | | 0.5 | 0.3 | 0.3 | | | | 25 | | 25 | 0.2 |
| Comparative Example C210 | | | | | | | | 1 | | | 5 | | | | | | | | 15 | | 35 | | | 0.5 | 0.3 | 0.3 | | | | 25 | | 25 | 0.2 |
| Comparative Example C211 | | | | | | | | | 1 | | 5 | | | | | | | | 15 | | 35 | | | 0.5 | 0.3 | 0.3 | | | | 25 | | 25 | 0.2 |
| Comparative Example C212 | | | | | | | | | | 0.7 | 5 | | | | | | | | 15 | | 35 | | | 0.5 | 0.3 | 0.3 | | | | 25 | | 25 | 0.2 |

[Table 14]

| Composition | Total amount of silane coupling material compounding amount index in composition | Compounding amount of polymerizable monomer having no acidic group and one or more hydroxyl groups with respect to total amount of 100 parts by mass of total amount of silane coupling material and all monomers (%) | Compounding amount of polymerizable monomer having methacryloyl group and/or methacrylamide group with respect to total amount of 100 parts by mass of total amount of silane coupling material and all monomers (%) | Durable adhesive strength | | | | | | | | | |
|---|---|---|---|---|---|---|---|---|---|---|---|---|---|
| | | | | Lithium disilicate | | Resin block | | GF reinforced resin (mesh adherence surface) | | GF reinforced resin (laminated adherend surface) | | Bovine dentin | |
| | | | | Initial preparation product | Acceleration test product | Initial preparation product | Acceleration test product | Initial preparation product | Acceleration test product | Initial preparation product | Acceleration test product | Initial preparation product | Acceleration test product |
| Example 201 | 0.0052 | 53 | 100 | 17.6 | 18.4 | 21.8 | 21.4 | 20.9 | 21.1 | 18.1 | 17.6 | 16.8 | 16.2 |
| Example 202 | 0.0057 | 53 | 100 | 18.0 | 17.8 | 20.4 | 20.5 | 21.2 | 21.2 | 17.1 | 18.1 | 15.2 | 16.9 |
| Example 203 | 0.0052 | 62 | 100 | 17.9 | 18.2 | 20.1 | 21.2 | 20.7 | 21.2 | 18.9 | 17.6 | 15.3 | 15.3 |
| Example 204 | 0.0050 | 53 | 92 | 20.9 | 21.6 | 20.9 | 20.7 | 22.0 | 21.7 | 20.8 | 20.7 | 16.5 | 15.0 |
| Example 205 | 0.0011 | 51 | 100 | 12.1 | 13.2 | 18.9 | 17.5 | 18.4 | 18.4 | 18.8 | 18.2 | 15.4 | 16.3 |
| Example 206 | 0.0041 | 53 | 98 | 21.7 | 21.5 | 21.4 | 21.3 | 21.1 | 21.1 | 21.5 | 21.5 | 16.8 | 15.2 |
| Example 207 | 0.0080 | 53 | 91 | 21.8 | 20.9 | 20.5 | 21.8 | 21.1 | 20.8 | 21.7 | 21.1 | 15.4 | 15.1 |
| Example 208 | 0.0074 | 52 | 90 | 20.3 | 20.8 | 20.3 | 20.2 | 20.8 | 21.6 | 21.5 | 21.5 | 15.4 | 16.8 |
| Example 209 | 0.0061 | 52 | 90 | 21.8 | 21.6 | 21.0 | 20.1 | 21.4 | 20.5 | 21.6 | 21.3 | 16.2 | 16.0 |
| Example 210 | 0.0099 | 78 | 99 | 20.8 | 13.6 | 21.2 | 13.1 | 20.8 | 12.5 | 20.7 | 12.0 | 10.4 | 10.1 |
| Example 211 | 0.0016 | 93 | 100 | 13.1 | 10.2 | 13.7 | 10.2 | 12.6 | 10.5 | 12.5 | 10.4 | 16.4 | 16.8 |
| Example 212 | 0.0012 | 55 | 100 | 12.8 | 12.7 | 13.7 | 13.2 | 12.8 | 12.0 | 12.7 | 12.9 | 16.2 | 15.8 |
| Example 213 | 0.0010 | 55 | 100 | 15.7 | 15.3 | 15.9 | 16.3 | 15.6 | 16.3 | 16.3 | 16.0 | 16.3 | 16.3 |
| Example 214 | 0.0144 | 38 | 100 | 17.2 | 15.5 | 17.1 | 14.2 | 17.7 | 14.9 | 17.3 | 15.7 | 10.7 | 10.2 |
| Example 215 | 0.0143 | 54 | 98 | 20.5 | 17.4 | 20.7 | 17.8 | 22.0 | 16.0 | 20.3 | 16.5 | 10.4 | 10.6 |
| Example 216 | 0.0105 | 31 | 100 | 17.1 | 15.7 | 17.6 | 14.2 | 17.1 | 15.2 | 18.3 | 15.4 | 10.2 | 10.4 |
| Example 217 | 0.0049 | 31 | 100 | 19.0 | 18.2 | 18.7 | 18.5 | 18.8 | 17.6 | 18.4 | 18.3 | 15.7 | 16.6 |
| Example 218 | 0.0106 | 43 | 63 | 16.3 | 12.4 | 17.0 | 13.8 | 17.5 | 12.2 | 16.9 | 12.5 | 10.7 | 10.5 |
| Example 219 | 0.0103 | 42 | 62 | 16.8 | 13.1 | 16.5 | 13.8 | 16.3 | 12.5 | 17.1 | 13.5 | 10.4 | 10.7 |
| Example 220 | 0.0104 | 71 | 76 | 17.7 | 15.5 | 16.3 | 14.9 | 17.2 | 15.7 | 17.0 | 14.7 | 10.5 | 10.4 |
| Example 221 | 0.0059 | 43 | 57 | 18.3 | 17.8 | 17.8 | 14.2 | 18.2 | 17.4 | 17.0 | 17.7 | 16.6 | 16.3 |
| Example 222 | 0.0077 | 29 | 100 | 17.6 | 17.5 | 15.4 | 15.4 | 13.8 | 13.8 | 12.2 | 13.6 | 11.5 | 9.2 |
| Example 223 | 0.0072 | 44 | 100 | 17.7 | 18.5 | 18.5 | 17.2 | 18.6 | 17.3 | 18.2 | 17.1 | 16.5 | 16.9 |
| Example 224 | 0.0050 | 43 | 100 | 18.7 | 18.8 | 17.0 | 17.8 | 17.3 | 17.7 | 17.5 | 18.1 | 16.6 | 15.3 |
| Example 225 | 0.0094 | 20 | 100 | 18.0 | 14.5 | 14.7 | 14.8 | 11.3 | 11.9 | 11.6 | 11.4 | 11.0 | 10.0 |
| Example 226 | 0.0030 | 17 | 83 | 18.8 | 17.3 | 14.3 | 14.3 | 11.5 | 10.6 | 11.8 | 12.0 | 15.9 | 16.3 |
| Example 227 | 0.0093 | 67 | 99 | 21.7 | 16.3 | 21.7 | 17.6 | 21.1 | 16.5 | 21.4 | 16.4 | 10.2 | 10.3 |
| Example 228 | 0.0078 | 14 | 100 | 18.5 | 17.0 | 14.3 | 14.3 | 11.6 | 11.9 | 11.4 | 11.5 | 10.5 | 10.7 |
| Example 229 | 0.0070 | 59 | 100 | 17.5 | 17.1 | 17.1 | 18.0 | 18.5 | 17.3 | 18.7 | 17.5 | 15.2 | 15.3 |
| Example 230 | 0.0058 | 23 | 100 | 18.1 | 17.6 | 17.8 | 18.6 | 12.2 | 12.7 | 13.9 | 12.4 | 16.4 | 16.8 |
| Example 231 | 0.0022 | 45 | 100 | 18.6 | 17.2 | 17.5 | 18.4 | 18.5 | 17.4 | 18.6 | 18.1 | 15.9 | 16.1 |
| Comparative Example C201 | 0.0005 | 54 | 100 | 0.7 | 0.8 | 6.7 | 6.0 | 6.4 | 6.5 | 3.5 | 3.4 | 16.5 | 16.2 |
| Comparative Example C202 | 0.0003 | 54 | 100 | 0.9 | 0.3 | 6.3 | 5.1 | 6.2 | 5.6 | 2.4 | 4.6 | 15.1 | 16.4 |
| Comparative Example C203 | 0.0007 | 54 | 100 | 0.7 | 0.6 | 6.4 | 5.2 | 6.5 | 5.5 | 2.4 | 3.6 | 15.7 | 15.7 |
| Comparative Example C204 | 0.0008 | 54 | 100 | 5.1 | 6.9 | 6.4 | 5.4 | 6.7 | 5.5 | 3.5 | 3.3 | 15.6 | 15.9 |
| Comparative Example C205 | 0.0191 | 53 | 100 | 17.0 | 4.9 | 16.4 | 9.4 | 21.6 | 3.9 | 20.6 | 2.7 | 7.8 | 8.9 |
| Comparative Example C206 | 0.0188 | 53 | 97 | 20.4 | 2.4 | 16.3 | 8.5 | 20.8 | 3.8 | 20.4 | 3.6 | 8.9 | 7.8 |
| Comparative Example C207 | 0.0351 | 51 | 100 | 18.6 | 3.5 | 18.7 | 9.5 | 21.9 | 2.7 | 21.9 | 3.8 | 7.9 | 8.4 |
| Comparative Example C208 | 0.0188 | 51 | 94 | 20.4 | 2.5 | 17.7 | 9.3 | 20.6 | 3.1 | 21.5 | 4.0 | 8.6 | 8.0 |
| Comparative Example C209 | 0.0115 | 37 | 100 | 20.6 | 21.9 | 20.7 | 21.5 | 20.9 | 20.1 | 20.7 | 20.3 | 1.0 | 0.6 |
| Comparative Example C210 | 0.0101 | 62 | 100 | 4.9 | 5.0 | 3.4 | 3.9 | 4.8 | 3.8 | 3.7 | 2.3 | 15.0 | 15.9 |
| Comparative Example C211 | 0.0000 | 62 | 100 | 4.9 | 4.5 | 4.2 | 3.7 | 4.0 | 3.9 | 1.3 | 2.9 | 16.1 | 15.6 |
| Comparative Example C212 | 0.0084 | 62 | 100 | 6.6 | 5.7 | 5.3 | 6.6 | 5.5 | 5.1 | 2.2 | 2.2 | 19.8 | 18.9 |

The adhesive compositions described in Examples 201 to 231 exhibited good adhesive strength of 10 MPa or more with respect to the lithium disilicate, the resin block, the GF reinforced resin and the tooth substance.

Examples 204, 206, 207, 208, 209, 210, 215 and 227 contained a silane coupling material having an acryloyl group, and therefore there was a tendency that good adhesive strength of 20 MPa or more with respect to lithium disilicate was exhibited.

In the compositions of Examples 205, 211, 212 and 213 in which the silane coupling material compounding amount index in composition was in the vicinity of the lower limit of 0.001, there was a tendency that adhesive strength to lithium disilicate was slightly low. In the compositions of Examples 210, 214, 215, 216, 218, 219, 220, 225 and 227 in which the silane coupling material compounding amount index in composition was in the vicinity of the upper limit of 0.015, there was a tendency that the durable adhesive strength after the acceleration test decrease as compared with the initial preparation product.

In the compositions of Examples 222, 225, 226 and 228 in which the compounding amount of the polymerizable monomer having no acidic group and having one or more hydroxyl groups was less than 30 parts by mass with respect to 100 parts by mass of the polymerizable monomer contained in the composition, there was a tendency that the adhesive strength with respect to the resin block and the GF reinforced resin was low, and the adhesive strength with respect to the GF reinforced resin was particularly low. In particular, in Examples 225, 226 and 228 in which the compounding amount was less than 20 parts by mass, the decrease in adhesive strength was particularly large. On the other hand, in Examples 210, 211, 220, 227 in which the compounding amount of the polymerizable monomer having no acidic group and having one or more hydroxyl groups exceeded 60 parts by mass with respect to 100 parts by mass of the polymerizable monomer, there was a tendency that that the adhesive strength of the acceleration test product decrease

as compared with the initial preparation product. In particular, in Examples 210, 211 and 220 in which the compounding amount exceeded 70 parts by mass, the decrease in adhesive strength was remarkable.

In Examples 218, 219, 220 and 221 in which the compounding amount of the a compound having a methacryloyl group and/or a methacrylamide group contained in the composition was less than 80 parts by mass, although a silane coupling material having an acryloyl group was contained, an adhesive strength of 20 MPa or more with respect to lithium silicate was not exhibited.

In Comparative Examples C201 to C204, since the compounding amount of the silane coupling material was less than the lower limit of the silane coupling material compounding amount index in composition represented by the formula (2), the adhesive strength to the lithium disilicate, the resin block and the GF reinforced resin was 10 MPa or less, and there was a tendency that the adhesive strength is low.

In Comparative Examples C205 to C208, since the compounding amount of the silane coupling material exceeded the upper limit of the silane coupling material compounding amount index in composition represented by the formula (2), the adhesive strength to the lithium disilicate, the resin block and the GF reinforced resin was 10 MPa or less, and there was a tendency that the storage stability was poor.

Comparative Example C209 had a low adhesive strength to the tooth substance of 5 MPa or less because it did not contain a polymerizable monomer having an acidic group.

Comparative Examples C210, C211 and C212 not containing the silane coupling material represented by structural formula of [Chemical formula 1] had remarkably low adhesive strength to the lithium disilicate, the resin block and the GF reinforced resin.

With respect to the use of substantially any plural and/or singular terms herein, those having skill in the art can translate from the plural to the singular and/or from the singular to the plural as is appropriate to the context.

Although the description herein has been given with reference to the drawings and embodiments, it should be noted that those skilled in the art may make various changes and modifications on the basis of this invention without difficulty. Accordingly, any such changes and modifications are intended to be included in the scope of the embodiments.

[Industrial applicability]

The present invention is widely used as a dental adhesive composition has been widely used as a dental adhesive material, a dental resin cement, a dental core build-up material, a dental adhesive material, a tooth substance primer, a metal primer, a ceramic primer, a composite resin, a dental pretreatment material and the like in the dental field and therefore can be used industrially.

## Claims

**1.** A dental adhesive composition comprising a matrix wherein

the matrix contains

(A) silane coupling material,
(B) polymerizable monomer having an acidic group,
(C) polymerizable monomer having no acidic group, and
at least one of (D) polymerization initiator and (E) polymerization accelerator,

(A) silane coupling material contains (A1) silane coupling material represented by structural formula of [Chemical formula 1],

[Chemical formula 1]

$$R^3-\underset{\underset{R^1_{(3-n)}}{|}}{Si}-(OR^2)_n$$

(In the formula, $R^3$ represents (meth) acryloyl group having an alkyl group of $C_2$ to $C_{15}$ which may have -O-, -S-, -NH-, -C(O)-O-, -OC(O)-, -OC(O)-NH- and/or -NH-C(O)-O- group, $R_1$ and $R_2$ represent alkyl groups of C1 to C4 and may be the same or different from each other, and n is 1 to 3.)
(C) polymerizable monomer having no acidic group contains (C1) polymerizable monomer having no acidic group and having one or more hydroxyl groups,

the (A1) silane coupling material represented by structural formula of [Chemical formula 1] and the (C1) polymerizable monomer having no acidic group and having one or more hydroxyl groups coexist in at least one matrix, and
the dental adhesive composition satisfies at least one of following formulas (1) and (2).

[Formula (1)]

$$0.005 \leq \text{Total amount of silane coupling material compounding amount index in matrix } ((S1 \times W1) / M1) \leq 0.070$$

(In the formula (1), M1 is a molecular weight of each silane coupling material contained in the matrix, S1 is the number of alkoxysilyl groups in molecule of each silane coupling material contained in the matrix, and W1 is a compounding amount of each silane coupling material in 100 parts by mass of the matrix. The silane coupling material compounding amount index in matrix is calculated by the formula (1) for each type of silane coupling material. )

[Formula (2)]

$$0.001 \leq \text{Total amount of silane coupling material compounding amount index in composition } ((S2 \times W2) / M2) \leq 0.015$$

(In the formula (2), M2 is a molecular weight of each silane coupling material contained in the composition, S2 is the number of alkoxysilyl groups in the molecule of each silane coupling material contained in the composition, and W2 is a compounding amount of each silane coupling material in 100 parts by mass of the composition. The silane coupling material compounding amount index in the composition is calculated by the formula (2) for each type of silane coupling material.)

**2.** The dental adhesive composition according to claim 1, wherein
the total amount of silane coupling material compounding amount index in matrix is 0.010 or more and 0.055 or less.

**3.** The dental adhesive composition according to claim 1 or 2, wherein

the dental adhesive composition consists of a first paste and a second paste,
the first paste contains a first matrix and (F) filler,
the first matrix contains the (A) silane coupling material containing the (A1) silane coupling material represented by structural formula of [Chemical formula 1] and the (C) polymerizable monomer having no acidic group,
the second paste contains a second matrix and (F) filler,
the second matrix contains the (B) polymerizable monomer having an acidic group and the (C) polymerizable monomer having no acidic group,
the first matrix contains at least one of the (D1) chemical polymerization initiator and the (E) polymerization accelerator,
the second matrix contains at least one of the (D1) chemical polymerization initiator and the (E) polymerization accelerator, and
when the first matrix contains one or more (D1) chemical polymerization initiator, the second matrix contains the (E) polymerization accelerator, and
when the first matrix contains one or more (E) polymerization accelerator, the second matrix contains the (D1) chemical polymerization initiator.

**4.** The dental adhesive composition according to claim 3, wherein
the dental adhesive composition substantially does not contain (G) water.

**5.** The dental adhesive composition according to any one of claims 1 to 4, wherein a proportion of the compound having a (meth) acryloyl group and/or a (meth) acrylamide group is 50 to 99.9 parts by mass in 100 parts by mass of a total amount of the (A) silane coupling material containing the (A1) silane coupling material represented by structural formula of [Chemical formula 1], the (B) polymerizable monomer having an acidic group and the (C) polymerizable monomer having no acidic group.

**6.** The dental adhesive composition according to claim 1 or 2, wherein the dental adhesive composition contains a matrix containing the (A) silane coupling material containing the (A1) silane coupling material represented by structural formula of [Chemical formula 1], the (B) polymerizable monomer having an acidic group, the (C) polymerizable monomer having no acidic group and the (D) polymerization initiator, and (F) filler.

**7.** The dental adhesive composition according to claim 6, wherein the (F) filler is surface-treated with one or more surface treatment agents selected from a silane coupling material, a surfactant, an organopolysiloxane, an inorganic oxide and a polymer compound.

**8.** The dental adhesive composition according to claim 1 or 2, wherein

the dental adhesive composition contains a matrix and (F) filler,
a compounding amount of the matrix contained in the dental adhesive composition is 25 to 75 parts by mass with respect to 100 parts by mass of the dental adhesive composition,
a compounding amount the (F) filler contained in the dental adhesive composition is 25 to 75 parts by mass with respect to 100 parts by mass of the dental adhesive composition,
a total amount of silane coupling material compounding amount index in matrix calculated in the formula (3) for each type of the (A1) silane coupling material represented by structural formula of [Chemical formula 3] satisfies following formula (3),

[Formula (3)]

$$0.005 \leq \text{Total amount of (A1) silane coupling material compounding amount index in matrix } ((S3 \times W3) / M3) \leq 0.070$$

(In the formula (3), M3 is a molecular weight of each (A1) silane coupling material represented by structural formula of [Chemical formula 1] contained in the matrix, S3 is the number of alkoxysilyl groups in molecule of each (A1) silane coupling material represented by structural formula of [Chemical formula 1] contained in the matrix, and W3 is a compounding amount of each (A1) silane coupling material represented by structural formula of [Chemical formula 1] in 100 parts by mass of the matrix. The silane coupling material compounding amount index in matrix is calculated by the formula (1) for each type of silane coupling material.)
a compounding amount of the (B) polymerizable monomer having an acidic group contained in the dental adhesive composition is 1 to 20 parts by mass with respect to 100 parts by mass of the matrix,
a compounding amount of the (C) polymerizable monomer having no acidic group contained in the dental adhesive composition is 65 to 95 parts by mass with respect to 100 parts by mass of the matrix,
a compounding amount of the (D) polymerization initiator contained in the dental adhesive composition is 0.3 to 6 parts by mass with respect to 100 parts by mass of the matrix,
a compounding amount of the (C1) polymerizable monomer having no acidic group and having one or more hydroxyl groups contained in the dental adhesive composition is 0.1 to 70 parts by mass with respect to 100 parts by mass of the matrix,
the polymerizable monomer contained in the matrix a polymerizable monomer having a (meth) acryloyl group and/or a (meth) acrylamide group,
a compounding amount of the compound having a (meth) acryloyl group and/or a (meth) acrylamide group is 50 to 99 parts by mass with respect to 100 parts by mass of the matrix.

**9.** The dental adhesive composition according to any one of claims 1 or 8, wherein the (A1) silane coupling material represented by structural formula of [Chemical formula 1] is a silane coupling material having an acryloyl group, and satisfies at least the formula (1).

**10.** The dental adhesive composition according to claim 3, wherein

the dental adhesive composition contains 15 to 80 parts by mass of the (C1) polymerizable monomer having no acidic group and having one or more hydroxyl groups with respect to 100 parts by mass of a total amount of the first matrix and the second matrix, and is used for a dental restoration material for cutting and machining.

**11.** The dental adhesive composition according to claim 10, wherein

a compounding amount of the (C1) polymerizable monomer having no acidic group and having one or more hydroxyl groups contained in the first matrix is 15 to 80 parts by mass with respect to 100 parts by mass of the first matrix,
a compounding amount of the (C1) polymerizable monomer having no acidic group and having one or more hydroxyl groups contained in the second matrix is 15 to 80 parts by mass with respect to 100 parts by mass of the second matrix, and
the dental adhesive composition satisfies at least the formula (1) and is used for a dental restoration material for cutting and machining.

**12.** The dental adhesive composition according to claim 11 or 12, wherein

the first matrix contains (C11) polymerizable monomer having no acidic group, having one or more hydroxyl groups, and having a viscosity of 200 mPa ·s or less at 25 °C, and
a compounding amount of the (C11) polymerizable monomer having no acidic group, having one or more hydroxyl groups, and having a viscosity of 200 mPa ·s or less at 25 °C is 0.1 to 40 parts by mass with respect to 100 parts by mass of the first matrix.

**13.** The dental adhesive composition according to any one of claims 10 to 12, wherein
the (F) filler compounded in the first paste is surface-treated with one or more surface treatment agents selected from an organopolysiloxane, a silane coupling material, an inorganic oxide, a surfactant and a polymer compound.

**14.** The dental adhesive composition according to claim 12, wherein

a volume ratio of the first paste and the second paste is 1: 0.8 to 1.2,
a compounding amount of the first matrix in the first paste is 25 to 75 parts by mass with respect to 100 parts by mass of the first paste,
a compounding amount of the (F) filler in the first paste is 25 to 75 parts by mass with respect to 100 parts by mass of the first paste,
a total amount of silane coupling material compounding amount index in matrix calculated in the formula (3) for each type of the (A1) silane coupling material represented by structural formula of [Chemical formula 1] satisfies following formula (3),

[Formula (3)]

$$0.005 \leq \text{Total amount of (A1) silane coupling material compounding amount index in matrix } ((S3 \times W3) / M3) \leq 0.070$$

(In the formula (3), M3 is molecular weight of each (A1) silane coupling material represented by structural formula of [Chemical formula 1] contained in the matrix, S3 is the number of alkoxysilyl groups in molecule of each (A1) silane coupling material represented by structural formula of [Chemical formula 1] contained in the matrix, and W3 is a compounding amount of each (A1) silane coupling material represented by structural formula of [Chemical formula 1] in 100 parts by mass of the matrix.)
a compounding amount of the (C) polymerizable monomer having no acidic group in the first matrix is 65 to 98 parts by mass with respect to 100 parts by mass of the first matrix,
a compounding amount of the second matrix in the second paste is 25 to 75 parts by mass with respect to 100 parts by mass of the second paste,
a compounding amount of the (F) filler in the second paste is 25 to 75 parts by mass with respect to 100 parts by mass of the second paste,
a compounding amount of the (B) polymerizable monomer having an acidic group in the second matrix is 1 to 30 parts by mass with respect to 100 parts by mass of the second matrix,
a compounding amount of the (C) polymerizable monomer having no acidic group in the second matrix is 65

to 95 parts by mass with respect to 100 parts by mass of the second matrix,
a compounding amount of the (D) polymerization initiator is 0.1 to 5 parts by mass with respect to 100 parts by mass of the total amount of the first matrix and the second matrix,
a compounding amount of the (E) polymerization accelerator is 0.01 to 5 parts by mass with respect to 100 parts by mass of the total amount of the first matrix and the second matrix,
a compounding amount of the (C1) polymerizable monomer having no acidic group and having one or more hydroxyl groups is 15 to 80 parts by mass with respect to 100 parts by mass of the total amount of the first matrix and the second matrix,
a compounding amount of the (C11) polymerizable monomer having no acidic group, having one or more hydroxyl groups, and having a viscosity of 200 mPa ·s or less at 25 °C contained in 100 parts by mass of the first matrix is 0.1 to 50 parts by mass.

**15.** The dental adhesive composition according to claim 1, wherein
the dental adhesive composition further contains (G) water and (H) volatile organic solvent, and satisfies at least the formula (2).

**16.** The dental adhesive composition according to claim 15, wherein
a compounding amount of the (C1) polymerizable monomer having no acidic group and having one or more hydroxyl groups is 20 to 70 parts by mass with respect to 100 parts by mass of a total amount of the (A) silane coupling material containing the (A1) silane coupling material represented by structural formula of [Chemical formula 1], (B) polymerizable monomer having an acidic group and (C) polymerizable monomer having no acidic group.

**17.** The dental adhesive composition according to claim 15 or 16, wherein
the (A1) silane coupling material represented by structural formula of [Chemical formula 1] is a silane coupling material having an acryloyl group.

**18.** The dental adhesive composition according to any one of claims 15 to 17, wherein
the total amount of silane coupling material compounding amount index in composition is 0.002 or more and 0.008 or less.

**19.** The dental adhesive composition according to any one of claims 15 to 18, wherein
the dental adhesive composition further contains a polymerizable monomer having one or more sulfur atoms.

**20.** The dental adhesive composition according to any one of claims 15 to 19, wherein

a total amount of silane coupling material compounding amount index in composition calculated in the formula (4) for each type of the (A1) silane coupling material represented by structural formula of [Chemical formula 4] satisfies following formula (4),

[Formula (4)]

$$0.001 \leq \text{Total amount of (A1) silane coupling material compounding amount index in composition } ((S4 \times W4) / M4) \leq 0.015$$

(In the formula (4), M4 is a molecular weight of each (A1) silane coupling material represented by structural formula of [Chemical formula 1] contained in the composition, S4 is the number of alkoxysilyl groups in molecule of each (A1) silane coupling material represented by structural formula of [Chemical formula 1] contained in the composition, and W4 is a compounding amount of each (A1) silane coupling material represented by structural formula of [Chemical formula 1] in 100 parts by mass of the composition.)
a compounding amount of the (B) polymerizable monomer having an acidic group contained in 100 parts by mass of the dental adhesive composition is 1 to 40 parts by mass,
a compounding amount of the (C) polymerizable monomer having no acidic group contained in 100 parts by mass of the dental adhesive composition is 5 to 60 parts by mass,
a compounding amount of the (D) polymerization initiator contained in 100 parts by mass of the dental adhesive composition is 0.01 to 5 parts by mass, and/or a compounding amount of the (E) polymerization accelerator contained in 100 parts by mass of the dental adhesive composition is 0.01 to 5 parts by mass,
a compounding amount of the (H) volatile organic solvent contained in 100 parts by mass of the dental adhesive

composition is 5 to 90 parts by mass,
a compounding amount of the (G) water contained in 100 parts by mass of the dental adhesive composition is 1 to 50 parts by mass,
a compounding amount of a polymerizable monomer having no acidic group and having two or more polymerizable groups is 40 to 100 parts by mass with respect to 100 parts by mass of the (C) polymerizable monomer having no acidic group contained in the dental adhesive composition,
a compounding amount of the (C1) polymerizable monomer having no acidic group and having one or more hydroxyl groups contained in (C) polymerizable monomer having no acidic group is 20 to 70 parts by mass with respect to 100 parts by mass of a total amount of the (A) silane coupling material containing the (A1) silane coupling material represented by structural formula of [Chemical formula 1], (B) polymerizable monomer having an acidic group and (C) polymerizable monomer having no acidic group,
a compounding amount of a compound having a methacryloyl group and/or a metaacrylamide group is 60 to 100 parts by mass in 100 parts by mass of a total amount of the (A) silane coupling material containing the (A1) silane coupling material represented by structural formula of [Chemical formula 1], (B) polymerizable monomer having an acidic group and (C) polymerizable monomer having no acidic group.

**21.** A dental self-adhesive composite resin containing the dental adhesive composition according to any one of claims 1 to 20.

**22.** Use of the dental adhesive composition according to any one of claims 15 to 20 for adhesion to a dental resin for cutting and machining.

**23.** Use of the dental adhesive composition according to any one of claims 15 to 20 for adhesion to a dental resin for cutting and machining consisting of a glass fiber reinforced material containing a glass fiber and an epoxy resin.

**24.** Use of the dental adhesive composition according to any one of claims 10 to 15 for a dental restoration material for cutting and machining wherein,

the dental restoration material for cutting and machining is a glass fiber reinforced material containing a glass fiber and an epoxy resin, and
the dental restoration material for cutting and machining is a material in which the orientation direction of the glass fibers is not uniform and the glass fibers are randomly compounded, or is a material which is a laminated body in which the glass fibers are woven in a cross shape, and the woven surface in a cross shape and a surface in which the woven surface in a cross shape is rotated 90° in the vertical direction are laminated surfaces of the glass fiber.

**25.** Use of the dental adhesive composition according to any one of claims 10 to 15 for a dental restoration material for cutting and machining wherein,
the dental adhesive composition is used for adhering to two or more adherend surfaces having different structures of the dental restoration material for cutting and machining.

Europäisches Patentamt
European Patent Office
Office européen des brevets

# EUROPEAN SEARCH REPORT

Application Number
EP 21 16 5514

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br><br>Y | WO 2019/082855 A1 (KURARAY NORITAKE DENTAL INC) 2 May 2019 (2019-05-02)<br>* abstract * | 1,2,5,6, 8,9, 15-25<br>3,4,7, 10-14 | INV.<br>A61K6/30<br>A61K6/887 |
| X,P<br><br>Y,P | -& EP 3 701 930 A1 (KURARAY NORITAKE DENTAL INC [JP])<br>2 September 2020 (2020-09-02)<br>* abstract *<br>* claims 1-13 *<br>* table 1 *<br>* paragraphs [0030], [0129], [0132] - [0141] *<br>----- | 1,2,5,6, 8,9, 15-25<br>3,4,7, 10-14 | |
| Y,D | JP 2016 124811 A (KURARAY NORITAKE DENTAL INC) 11 July 2016 (2016-07-11)<br>* abstract *<br>* claim 1 *<br>* table 1 *<br>* paragraphs [0114], [0115] *<br>----- | 3,4,7, 10-14 | |

TECHNICAL FIELDS SEARCHED (IPC)

A61K

The present search report has been drawn up for all claims

| Place of search | Date of completion of the search | Examiner |
|---|---|---|
| The Hague | 24 September 2021 | Gomes Pinto F., R |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# ANNEX TO THE EUROPEAN SEARCH REPORT ON EUROPEAN PATENT APPLICATION NO.

EP 21 16 5514

This annex lists the patent family members relating to the patent documents cited in the above-mentioned European search report. The members are as contained in the European Patent Office EDP file on
The European Patent Office is in no way liable for these particulars which are merely given for the purpose of information.

24-09-2021

| Patent document cited in search report | Publication date | Patent family member(s) | Publication date |
|---|---|---|---|
| WO 2019082855 A1 | 02-05-2019 | AU 2018356814 A1 | 14-05-2020 |
| | | CA 3079805 A1 | 02-05-2019 |
| | | CN 111225647 A | 02-06-2020 |
| | | EP 3701930 A1 | 02-09-2020 |
| | | JP WO2019082855 A1 | 12-11-2020 |
| | | US 2021186823 A1 | 24-06-2021 |
| | | WO 2019082855 A1 | 02-05-2019 |
| EP 3701930 A1 | 02-09-2020 | AU 2018356814 A1 | 14-05-2020 |
| | | CA 3079805 A1 | 02-05-2019 |
| | | CN 111225647 A | 02-06-2020 |
| | | EP 3701930 A1 | 02-09-2020 |
| | | JP WO2019082855 A1 | 12-11-2020 |
| | | US 2021186823 A1 | 24-06-2021 |
| | | WO 2019082855 A1 | 02-05-2019 |
| JP 2016124811 A | 11-07-2016 | JP 6346086 B2 | 20-06-2018 |
| | | JP 2016124811 A | 11-07-2016 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2016124811 A
- JP 2018177677 A
- WO 201900439 A
- JP 2019094276 A